# EUROPEAN PATENT APPLICATION

(11) **EP 4 803 529 A1**
(43) Date of publication of application: **09.09.2026**
(21) Application number: 24893394.7
(22) Date of filing: 18.11.2024
(51) Int. Cl.: C07H 21/00, C12N 15/113, A61K 31/713, A61K 47/54, A61P 25/00

(54) **LIPID COMPOUND, NUCLEIC ACID CONJUGATE AND USE THEREOF**

(30) Priority: 22.11.2023 CN 202311566371
(71) Applicant: SynerK Inc., Grand Cayman KY1-9006 (KY)
(72) Inventor: YU, Dong, Hong Kong 999077 (CN); LAN, Tao, Brookline, Massachusetts 02446 (US); JIANG, Weiwen, Winchester, Massachusetts 01890 (US)
(74) Representative: Lorenz Seidler Gossel Part. mbB
(86) International application number: PCT/CN2024/132723
(87) International publication number: WO 2025/108237

(57) **Abstract**

The present invention relates to the field of biomedicines, and in particular to a lipid compound, a nucleic acid conjugate and a use thereof. The lipid compound and the nucleic acid conjugate significantly improve the intracellular delivery of nucleic acids, improve the delivery of targeting genes, and further achieve a major breakthrough in simultaneous delivery of more than two nucleic acids. The present invention also relates to a use of the nucleic acid conjugate in preparation of a drug for treating gene-related diseases.

## Description

### TECHNICAL FIELD

The present disclosure belongs to the field of biomedicine, and in particular, relates to a lipid compound and a nucleic acid conjugate, which significantly improve intracellular delivery of nucleic acids, enhance delivery of target genes, and further achieve a major breakthrough in simultaneous delivery of various nucleic acids.

### BACKGROUND

Nucleic acids include ribonucleic acid (RNA) and deoxyribonucleic acid (DNA). As fundamental genetic materials, nucleic acids not only control biosynthesis of proteins, but also participate in life processes such as growth, heredity, and variation. Nucleic acids can maintain normal immune functions of the body and the growth and metabolism of the immune system, delay aging, improve hematopoietic functions of the bone marrow and metabolic activity of blood components, eliminate carcinogenic factors, alleviate dementia, inhibit formation of lipid peroxides and cholesterol synthesis, dilate blood vessels, enhance blood flow, correct cardiac decompensation, and promote regeneration of vascular walls. In addition, nucleic acids can promote metabolism of cells (including insulin-secreting cells in pancreas). The metabolic product of nucleic acids, adenosine, also inhibits sugar breakdown, thereby slowing absorption of sugar in the small intestine. Therefore, nucleic acids may be used for the maintenance of the immune system, anti-aging, and treatment of anemia, cerebral thrombosis, myocardial infarction, hypertension, atherosclerosis, and diabetes.

However, due to their large molecular weight, hydrophilicity, and/or charge, nucleic acids can only enter cells through endocytosis. However, lipid bilayers of the cells can capture and retain approximately 99% of the nucleic acid molecules, resulting in degradation of the nucleic acid molecules. It is reported that only 0.3%-1% of nucleic acids can enter cells.

Therefore, how to enhance the intracellular delivery of nucleic acids remains one of the major technical challenges in the field. In particular, how to effectively deliver more than two types of nucleic acids into cells simultaneously constitutes a new technical problem posed by the inventors.

In addition, how to improve the targeted delivery of nucleic acids to specific genes is also one of the technical problems urgently needing to be resolved in the field.

### SUMMARY OF THE INVENTION

The technical problems to be resolved in the present disclosure include: how to improve the intracellular delivery of nucleic acids; how to effectively deliver multiple nucleic acids into cells simultaneously; and how to enhance the targeted delivery of nucleic acids to specific genes.

To resolve the above technical problems, the present disclosure provides a lipid compound with a specific structure and a conjugate using the lipid compound as a conjugating moiety.

The lipid compound according to the present disclosure comprises a saturated lipid compound having a structure represented by the following formula (A) or (B): 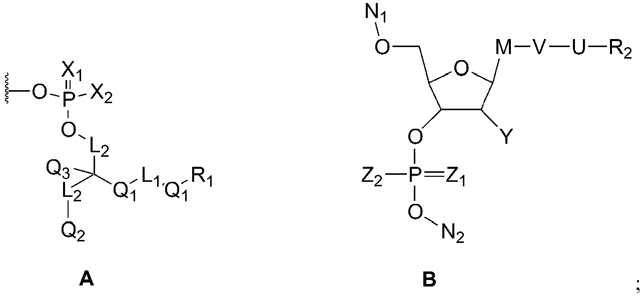
where in the compound represented by formula (A):
Q₁ is selected from -NH₂ (amino group), -COOH (carboxyl group), -NHCO (amide group), -O-, -S-, -S-S-, a phosphate group, or a phosphorothioate group;
Q₂ is selected from -OH (hydroxyl group), -NH₂ (amino group), -H or -CH₃ (methyl group);
Q₃ is selected from -H or a C₁-C₁₀ alkyl group;
L₁ is selected from a C₁-C₁₀ saturated alkyl chain;
L₂ is selected from a C₁-C₁₀ saturated alkyl chain;
X₁ is selected from an O or S atom;
X₂ is selected from -O-, -S-, -OH (hydroxyl group), -NH₂ (amino group), -CH₃ (methyl group), -CH₂CH₃ (ethyl group), -OCH₃ (methoxy group), or -OCH₂CH₃ (ethoxy group);
R₁ is selected from a C₁₀-C₃₀ saturated fatty acid chain or saturated alkyl chain; and
where in the compound represented by formula (B): the five-membered ring is a five-membered sugar ring structure of ribose or deoxyribose, where 1-position of the five-membered ring is selected from CH₂, O, or S:
   M is selected from H, O, C, or a modified or unmodified nucleobase; preferably, M is independently selected from adenine, uracil, thymine, guanine, or cytosine;
   N₁ is selected from H or a C₁-C₃ alkyl group;
   Y is selected from H, NH₂, OH, halogen, a C₁-C₆ alkyl group, a C₁-C₆ haloalkyl group, a C₁-C₆ alkoxy group, MOE (2'-O-methoxyethyl);
   V is selected from a C₁-C₄ saturated alkyl chain;
   U is selected from -NH₂ (amino group), -COOH (carboxyl group), or -NHCO (amide group);
   Z₁ is selected from an O or S atom;
   Z₂ is selected from a C₁₀-C₃₀ alkoxy group or a C₁₀-C₃₀ amide saturated lipid chain; and
   R₂ is selected from a C₁₀-C₃₀ alkoxy group, a C₁₀-C₃₀ saturated fatty acid chain, or a C₁₀-C₃₀ amide saturated lipid chain.

The saturated lipid compound according to the present disclosure comprises at least one selected from the following structures (L1) to (L18): 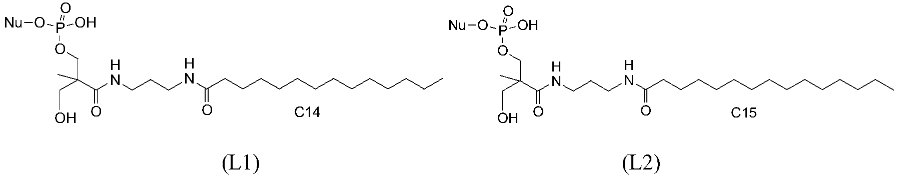 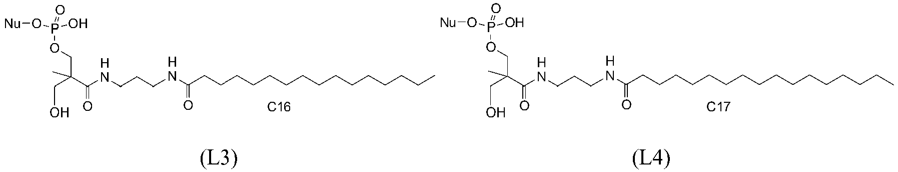 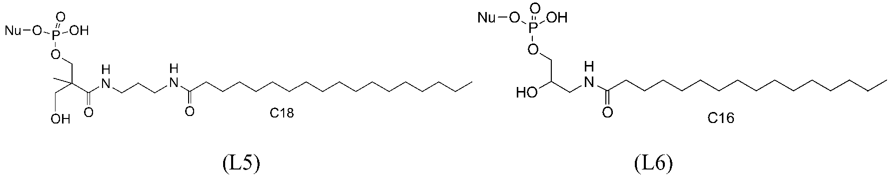 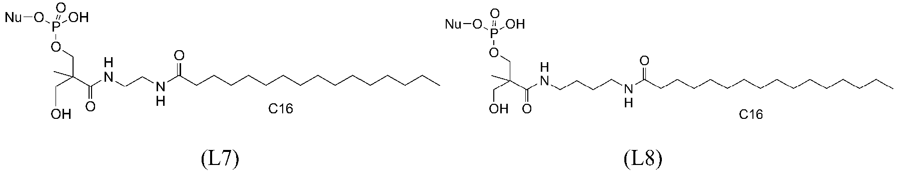 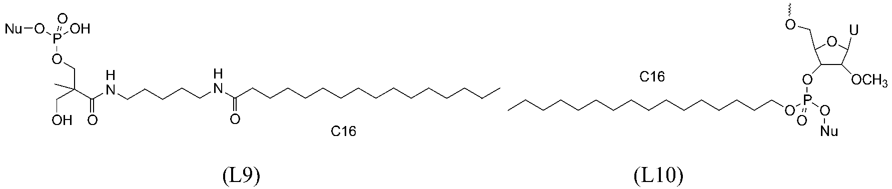 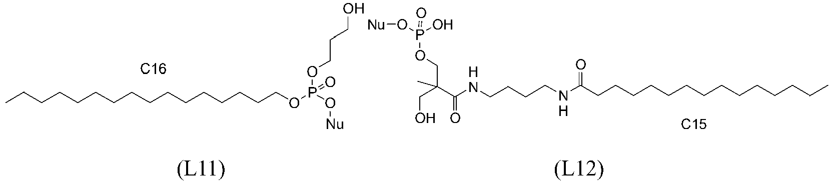 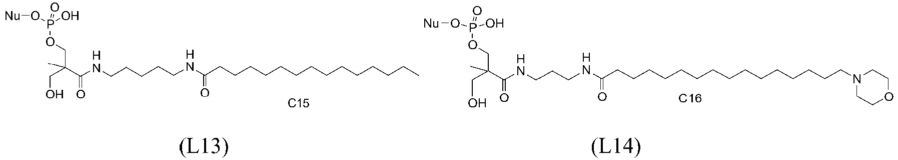 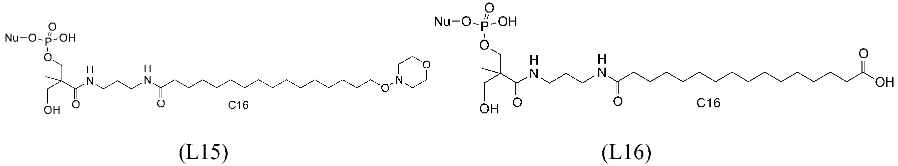 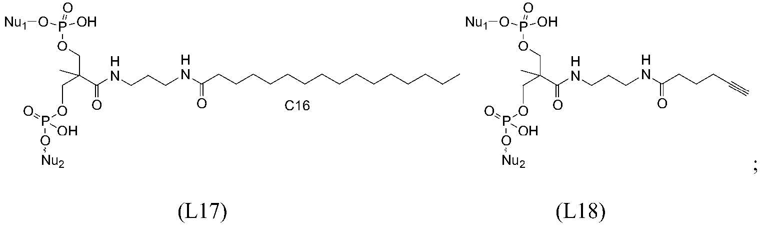 where Nu, Nu₁ and Nu₂ each independently represents a specific, independent nucleotide sequence.

The nucleic acid conjugate according to the present disclosure comprises an oligonucleotide conjugate, which comprises an oligonucleotide and a conjugate moiety conjugated to the oligonucleotide.

The conjugate moiety is selected from the saturated lipid compounds described above.

Preferably, the oligonucleotide conjugate comprises a single-stranded or double-stranded oligonucleotide, preferably with a length of 2-30 mer.

The oligonucleotide conjugate according to the present disclosure comprises siRNA, which contains a sense strand and an antisense strand. Each nucleotide in the siRNA is independently a modified or unmodified nucleotide.

The oligonucleotide conjugate according to the present disclosure comprises an oligonucleotide conjugate in which at least one nucleotide in the sense strand or the antisense strand is a modified nucleotide.

The oligonucleotide conjugate according to the present disclosure comprises an oligonucleotide conjugate in which all nucleotides in the sense strand and/or the antisense strand are modified nucleotides.

The oligonucleotide conjugate according to the present disclosure comprises an oligonucleotide conjugate in which each nucleotide in the sense strand and/or the antisense strand is independently a nucleotide with fluorine-substitution modification or a nucleotide with non-fluorine-substitution modification.

Preferably, the fluorine-substitution modification is that the hydroxy group at the 2'-position of the nucleotide is substituted with F.

Preferably, the non-fluorine-substitution modification is that the hydroxy group at the 2'-position of the nucleotide is substituted with an alkoxy group.

The oligonucleotide conjugate according to the present disclosure comprises at least one selected from the following structures:

| | |
|---|---|
| SNK-981-A | 5'-mCsmAsmUmUmUmUAfmAUfCfCfmUmCmAmCmUmCmUmAsmAsmA-3'-L1; |
| | 5'-mUsUfsmUmAmGAfmGUfGfmAmGmGmAUfmUAfmAmAmAmUmGsmAsmG-3'; |
| SNK-981-B | 5'-mCsmAsmUmUmUmUAfmAUfCfCfmUmCmAmCmUmCmUmAsmAsmA-3'-L2; |
| | 5'-mUsUfsmUmAmGAfmGUfGfmAmGmGmAUfmUAfmAmAmAmUmGsmAsmG-3'; |
| SNK-981-C | 5'-mCsmAsmUmUmUmUAfmAUfCfCfmUmCmAmCmUmCmUmAsmAsmA-3'-L3; |
| | 5'-mUsUfsmUmAmGAfmGUfGfmAmGmGmAUfmUAfmAmAmAmUmGsmAsmG-3'; |
| SNK-981-D | 5'-mCsmAsmUmUmUmUAfmAUfCfCfmUmCmAmCmUmCmUmAsmAsmA-3'-L4; |
| | 5'-mUsUfsmUmAmGAfmGUfGfmAmGmGmAUfmUAfmAmAmAmUmGsmAsmG-3'; |
| SNK-981-E | 5'-mCsmAsmUmUmUmUAfmAUfCfCfmUmCmAmCmUmCmUmAsmAsmA-3'-L6; |
| | 5'-mUsUfsmUmAmGAfmGUfGfmAmGmGmAUfmUAfmAmAmAmUmGsmAsmG-3'; |
| SNK-981-F | 5'-mCsmAsmUmUmUmUAmAUfCfCfmUmCmAmCmUmCmUmAsmAsmA-3'-L7; |
| | 5'-mUsUfsmUmAmGAfmGUfGfmAmGmGmAUfmUAfmAmAmAmUmGsmAsmG-3'; |
| SNK-981-G | 5'-mCsmAsmUmUmUmUAfmAUfCfCfmUmCmAmCmUmCmUmAsmAsmA-3'-L8; |
| | 5'-mUsUfsmUmAmGAfmGUfGfmAmGmGmAUfmUAfmAmAmAmUmGsmAsmG-3'; |
| SNK-981-H | 5'-mCsmAsmUmUmUmUAfmAUfCfCfmUmCmAmCmUmCmUmAsmAsmA-3'-L9; |
| | 5'-mUsUfsmUmAmGAfmGUfGfmAmGmGmAUfmUAfmAmAmAmUmGsmAsmG-3'; |
| SNK-981-J | 5'-mCsmAsmUmUmUmUAfmAUfCfCfmUmCmAmCmUmCmUmAsmAsmA-3'-L12; |
| | 5'-mUsUfsmUmAmGAfmGUfGfmAmGmGmAUfmUAfmAmAmAmUmGsmAsmG-3'; |
| SNK-981-K | 5'-mCsmAsmUmUmUmUAfmAUfCfCfmUmCmAmCmUmCmUmAsmAsmA-3'-L13; |
| | 5'-mUsUfsmUmAmGAfmGUfGfmAmGmGmAUfmUAfmAmAmAmUmGsmAsmG-3'; |
| SNK-981-L | 5'-mCsmAsmUmU-(L10)-mUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |
| SNK-981-M | 5'-L11-mCsmAsmUmUmUmUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |
| SNK-981-N | 5'-mCsmAsmUmUmUmUAfmAUfCfCfmUmCmAmCmUmCmUmAsmAsmA-3'-L3; |
| | 5'-VP-mUsUfsmUmAmGAfmGUfGfmAmGmGmAUfmUAfmAmAmAmUmGsmAsmG- 3'; |
| SNK-981-P | 5'-mCsmAsmUmUmUmUAfmAUfCfCfmUmCmAmCmUmCmUmAsmAsmA-3'-L3; |
| | 5'-Alexa647- mUsUfsmUmAmGAfmGUfGfmAmGmGmAUfmUAfmAmAmAmUmGsmAsmG-3'; |
| SNK-981-Q | 5'-TsAGsGAstsastsastsaststs*C*stsasCAsGCsT-L17- mCsmAsmUmUmUmUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |
| SNK-981-R | 5'-mCsmAsmUmUmUmUAfmAUfCfCfmUmCmAmCmUmCmUmAsmAsmA-L17- TsAGsGAstsastsastststsCstsasCAsGCsT 3'; |
| | 5'-mUsUfsmUmAmGAfmGUfGfmAmGmGmAUfmUAfmAmAmAmUmGsmAsmG-3'; |
| SNK-981-S | 5'-CsCsGsTsCsgsCsCsCststsCsasgsCsAsCsGsCsA-L17- mCsmAsmUmUmUmUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |
| SNK-981-T | 5'-mCsmAsmUmUmUmUAfmAUfCfCfmUmCmAmCmUmCmUmAsmAsmA-L17- CsCsGsTsCsgsCsCsCststsCsasgsCsAsCsGsCsA-3'; |
| | 5'-mUsUfsmUmAmGAfmGUfGfmAmGmGmAUfmUAfmAmAmAmUmGsmAsmG-3'; |
| SNK-981-U | 5'-mAsmAsmAmUmCmUmCmAmCmUCfCfUfmAAfmUmUmUmUsmAsmC-L17- CsCsGsTsCsgsCsCsCststsCsasgsCsAsCsGsCsA-3'; |
| | 5'-mUsUfsmUmAmGAfmGUfGfmAmGmGmAUfmUAfmAmAmAmUmGsmAsmG-3'; |
| SNK-981-V | 5'-AsCsGsCsAsCsgsgsasCststsCsCsCsgsgsCsTsGsCsCs-L17- mCsmAsmUmUmUmUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |
| SNK-981-W | 5'-CsCsGsTsCsgsCsCsCststsCsasgsCsAsCsGsCsA-L18- mCsmAsmUmUmUmUAfmAUfCfCfmUmCmAmCmUmCmUmAsmAsmA-3'; |
| | 5'-mUsUfsmUmAmGAfmGUfGfmAmGmGmAUfmUAfmAmAmAmUmGsmAsmG-3'; |

.

The present disclosure also provides use of the oligonucleotide conjugate for manufacture of a medicament for treating a central nervous system disease.

Preferably, the central nervous system disease comprises Alzheimer's disease, amyotrophic lateral sclerosis (ALS), Huntington's disease, Parkinson's disease, Dravet syndrome, Charcot triad, Alexander disease, spinocerebellar ataxia, or Angelman syndrome.

The present disclosure also provides a pharmaceutical composition comprising the oligonucleotide conjugate according to the present disclosure and a pharmaceutically acceptable carrier.

Preferably, the pharmaceutical composition comprises the medicament for treating the central nervous system disease.

It should be noted that the five-membered ring in the structure of formula (B) described above may be a five-membered sugar ring structure of ribose or deoxyribose, that is, ribo-pentofuranose, which is a ribose structure capable of carrying a saturated lipid chain. This structure may be inserted into an appropriate position in the nucleic acid sequence, so that the sequence fragment carries a saturated lipid chain for delivery.

The lipid compound according to the present disclosure can bind to nucleic acid(s) at one or two sites, thereby improving the intracellular delivery of the nucleic acid. When the delivered nucleic acid is siRNA, the gene silencing effect may be significantly improved and the target gene may be inhibited. The lipid compound according to the present disclosure can bind to the nucleic acid by forming a bond with the hydroxyl group (e.g., hydroxyl group at the 2-, 3- or 5-position) or the phosphate group (including phosphorothioate group) in the ribose moiety of the nucleic acid. Therefore, as long as there is a hydroxyl group and/or phosphate group in the ribose moiety of the nucleic acid, the nucleic acid can bind to the lipid compound according to the present disclosure, thereby significantly improving the cellular delivery of the nucleic acid.

When the lipid compound according to the present disclosure binds to nucleic acids at two sites, simultaneous delivery of two or more nucleic acids may be achieved. The aforementioned nucleic acids comprise double-stranded and single-stranded nucleic acids. When the lipid compound of the present invention binds to one double-stranded nucleic acid and one single-stranded nucleic acid, the targeted delivery efficiency for a specific gene is significantly increased compared with binding to a single double-stranded nucleic acid or a single single-stranded nucleic acid.

In the technical solution of the present disclosure, the C₁₀-C₃₀ saturated fatty acid chain comprises -(CH₂)ₘ-COOH or -(CH₂)ₘ-COOR'₁₆, where m and R'₁₆ are defined as below. In the structural formulae of the present disclosure, the wavy line refers, for example, to the wavy line in formula (I), the wavy line in 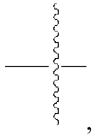 the wavy line in the structural formula of L1, or the wavy line in 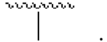 The wavy line indicates the position for binding to another chemical structure.

The present disclosure also discloses a nucleic acid conjugate, comprising an oligonucleotide and a conjugate moiety conjugated to the oligonucleotide.

The conjugate moiety is selected from said lipid compounds.

Preferably, the nucleic acid conjugate comprises a single-stranded or double-stranded oligonucleotide, preferably with a length of 2-30 mer.

In the nucleic acid conjugate according to the present disclosure, the active functional oligonucleotide may be selected from the following nucleic acid substances: small interfering RNA, microRNA, single-stranded RNA, antisense nucleic acid, guide oligonucleotide, or stem-loop RNA. The functional oligonucleotide consists of a single-stranded oligonucleotide or a double-stranded oligonucleotide. The small interfering RNA in the present disclosure is selected from a double-stranded oligonucleotide, comprising a sense strand and an antisense strand, where the sense strand and the antisense strand are partially or fully complementary, that is, in the double-stranded nucleic acid molecule, bases of one strand pair with bases of the other strand through hydrogen bonding in a partially or fully complementary manner. In a double-stranded helical oligonucleotide, the purine base adenine (A) pairs with the pyrimidine base thymine (T) or uracil (U); and the purine base guanine (G) always pairs with the pyrimidine base cytosine (C). The sequences of the two complementary strands are oriented from 5'-end to 3'-end and from 3'-end to 5'-end, respectively. Each nucleotide in the small interfering RNA is independently a modified or unmodified nucleotide, where modification refers to the substitution of any part of the nucleotide by another group. The modification at the 2'-position of the nucleotide may be selected from groups such as 2'-methoxy, 2'-fluoro, 2'-methoxyethyl, 2'-2,4-dinitrophenol, 2'-amino, and 2'-4'-constrained ethyl. Adjacent nucleosides in the sequence are linked by a phosphodiester bond, where a phosphorothioate diester bond may be formed by replacing one oxygen atom in the phosphodiester bond with a sulfur atom. The sense strand and antisense strand sequences typically have a length of 19 to 23 nucleotides, and form a duplex by complementary pairing. The nucleotide sequence of the sense strand is a segment that shares at least 9 consecutive nucleotides identical to the target mRNA. The antisense strand typically comprises two or more consecutive deoxythymidine nucleotides or two or more consecutive uracil nucleotides. In addition, the target mRNA generally refers to mRNA of a gene associated with abnormal protein expression in cells.

Specifically, in the nucleic acid conjugate, the oligonucleotide comprises a sense strand and an antisense strand, and each nucleotide in the oligonucleotide is independently a modified or unmodified nucleotide.

Optionally, the sense strand comprises the following nucleotide sequence: 5'-CAUUUUAAUCCUCACUCUAAA-3'.

Optionally, the antisense strand comprises the following nucleotide sequence: 5'-UUUAGAGUGAGGAUUAAAAUGAG-3'.

Specifically, in the nucleic acid conjugate, at least one nucleotide in the sense strand or the antisense strand is a modified nucleotide.

Specifically, in the nucleic acid conjugate, all nucleotides in the sense strand and/or the antisense strand are modified nucleotides.

Specifically, in the nucleic acid conjugate, each nucleotide in the sense strand and/or the antisense strand is independently a nucleotide with fluorine-substitution modification or a nucleotide with non-fluorine-substitution modification.

Preferably, the fluorine-substitution modification is that the hydroxy group at the 2'-position of the nucleotide is substituted with F.

Preferably, the non-fluorine-substitution modification is that the hydroxy group at the 2'-position of the nucleotide is substituted with an alkoxy group.

The present disclosure further discloses use of the nucleic acid conjugate for manufacture of a medicament for treatment of a central nervous system disease.

The present disclosure further discloses a pharmaceutical composition, comprising the nucleic acid conjugate and a pharmaceutically acceptable carrier.

Preferably, the pharmaceutical composition comprises the medicament for the treatment of the central nervous system disease.

The lipid compounds and nucleic acid conjugates according to the present disclosure relate to nucleic acids, such as small nucleic acid drugs. Through the design of a series of lipid compounds and nucleic acid conjugates and nucleotide conjugates formed by the lipid compounds, as well as the optimization and modification of nucleic acid and nucleotide structures, the ability of delivering nucleic acids is greatly improved, thereby allowing the nucleic acids to function better in cells and enhancing the delivery of nucleic acids targeting genes.

The lipid compound provided in the present disclosure has the advantages of low raw material cost, simple synthetic method, fewer synthetic steps, and high synthetic yield. For example, the price of a starting material for preparing the key lipid compound according to the present disclosure is as follows: 2,2-bis(hydroxymethyl)propionic acid; 4767-03-7; analytical grade: 99%; 23 CNY/kg. In the present disclosure, a conventional esterification reaction in the chemical field is used, and complex processes such as purification, separation, and chiral stereospecificity are not involved. A preparation process of a fatty ester in the present disclosure involves a four-step reaction. In the art, even if the yield of each step is 80%, the overall yield may be at most 40%. However, the average overall yield in the present disclosure is 45%-50%, which is of milestone-like significance for massive production.

The lipid compound has multiple linking sites, providing more options for the linkage design of active compounds and exerting a certain promoting effect on the development of active drugs. The lipid compound comprises an unsaturated fatty acid carrier, and preferably, straight-chain or branched alkane with multiple functional groups is used as the carrier linker. The lipid compound may be directly or indirectly linked to any active drug. For example, the lipid compound may be linked to the active drug via the linker. The lipid compound can also be directly or indirectly linked to oligonucleotides, cholesterol, polypeptides, nanoparticles, aptamers, antibodies, nanobodies, small molecules, or any other agents with clinical application value. For example, the lipid compound may be inserted into the sense strand or antisense strand of the nucleic acid, which effectively improves targeted delivery performance of the nucleic acid; preferably, the lipid compound may be inserted into the sense strand or antisense strand of the siRNA; preferably, the lipid compound may be inserted into a single-stranded antisense oligoribonucleotide.

The targeted genes of the lipid nucleic acid conjugates provided in the present disclosure comprise, but are not limited to, APP, SOD1, HTT, MeCP2, DUX4, HDAC2, GPR75, Ataxin 1, Ataxin 2, Ataxin 3, Ataxin 6, Ataxin 7, C9ORF72, UBE3A, Prion, PMP22, Tau, LRRK2, LINGO2, GYS1, KCNT1, IRF5, Progranulin, GFAP, TARDBP, SNCA, FUS, SCA1, SCA7, SCA8, MeCP2, PRNP, SOD1, DMPK, MAPT, and TTR. By targeting the foregoing genes, the conjugates according to the present disclosure may be used for the treatment of diseases associated with such genes, for example, Alzheimer's disease, presenile dementia (genes: APP, HDAC2, and MAPT/tau), amyotrophic lateral sclerosis (ALS) (genes: SOD1, C9orf72, TARDBP, and FUS), Huntington's disease (genes: HTT and ATXN6), Rett syndrome (gene: MeCP2), Facioscapulohumeral muscular dystrophy (gene: DUX4), obesity (gene: GPR75), spinocerebellar ataxia (genes: ATXN1, ATXN2, ATXN3, ATXN6, and ATXN7), Angelman syndrome (gene: UBE3A), Creutzfeldt-Jakob disease/variant Creutzfeldt-Jakob disease/Gerstmann-Sträussler-Scheinker syndrome/fatal familial insomnia (gene: Prion), Charcot-Marie-Tooth disease (gene: PMP22), Parkinson's disease (genes: LRRK2, LINGO2, and SNCA), glycogen synthase deficiency (gene: GYS1), epilepsy (gene: KCNT1), inflammation (gene: IRF5), frontotemporal dementia (genes: Progranulin and FUS), Alexander disease (gene: GFAP), multiple system atrophy (gene: SNCA), Lewy body dementia (gene: SNCA), myotonic dystrophy type 1 (gene: DMPK), and polyneuropathy (gene: TTR). Accordingly, upon understanding the disclosure of this application, for a specific gene-related disease, a person skilled in the art can reasonably select the corresponding nucleic acid targeting the specific gene, and conjugate it with the lipid compound of the present disclosure, thereby forming the corresponding conjugate. The conjugates thus produced are also encompassed within the scope of the present disclosure.

When the nucleic acid conjugate containing one or more nucleic acids is formed through the specific lipid compound according to the present disclosure, the intracellular delivery of nucleic acids is significantly improved, and the inhibition of the nucleic acid on mRNA is remarkably enhanced.

In the field of nucleic acid delivery, intracellular delivery of a single nucleic acid, such as a double-stranded nucleic acid, has already proven difficult. Consequently, related technical improvements in this field have generally been limited to methods for improving the delivery of a single nucleic acid. A person skilled in the art has had no motivation to attempt simultaneous delivery of two nucleic acids, such as both double-stranded and single-stranded nucleic acids. However, the inventors of the present disclosure have made pioneering and bold attempts to address both types of nucleic acids in combination with the relevant lipid compounds. Unexpectedly, it was discovered that the lipid compound having the specific structure disclosed herein can form a conjugate simultaneously conjugated with both double-stranded and single-stranded nucleic acids. Such conjugates achieve remarkable improvements in intracellular delivery and significantly enhance mRNA inhibition. Even more surprisingly, the inventors further discovered that the conjugates of the present disclosure exhibit a synergistic effect in nucleic acid intracellular delivery and mRNA inhibition. That is, the conjugates achieve results superior to the use of either double-stranded or single-stranded nucleic acids alone, thereby realizing a "1+1 > 2" synergistic effect.

Accordingly, through the use of specific lipid compounds, the present disclosure makes a groundbreaking and epoch-making contribution to the field of nucleic acid delivery.

### BRIEF DESCRIPTION OF THE DRAWINGS

For ease of understanding the content of the present disclosure clearly, the present disclosure is described in further detail below with reference to specific embodiments of the present disclosure and the accompanying drawings.
FIG. 1 shows residual SOD1 mRNA levels in rat B35 cells in Example 10 (1);
FIG. 2 shows residual SOD1 mRNA levels in the brains of SD rats in Example 10 (2);
FIG. 3 shows residual SOD1 mRNA levels in the brains of SD rats in Example 10 (3);
FIG. 4 shows residual AT×N3 mRNA levels in the brains of mice in Example 10 (4).
FIG. 5 shows residual SOD1 mRNA levels in the brains of SD rats in Example 10 (5);
FIG. 6 shows residual SOD1 mRNA levels in the brains of rats in Example 10 (6);
FIG. 7 shows residual SOD1 mRNA levels in the brains of rats in Example 10 (7);
FIG. 8 shows residual human MAPT mRNA levels in the brains of mice in Example 10 (8).
FIG. 9 shows residual SOD1 mRNA levels in the brains of rats in Example 10 (9);
FIG. 10 shows residual SOD1 mRNA levels in the brains of rats in Example 10 (10);
FIG. 11 shows residual SODl mRNA levels in the brains of rats in Example 10 (11);
FIG. 12 shows residual SOD1 mRNA levels in various regions of the brains of rats in Example 10 (12); and
FIG. 13 shows residual SOD1 mRNA levels in the brains of rats in Example 10 (13).

### DETAILED DESCRIPTION

The present disclosure provides the following embodiments.

Embodiment 1. A lipid compound having a structure represented by formula (I), (II), or (V) below: 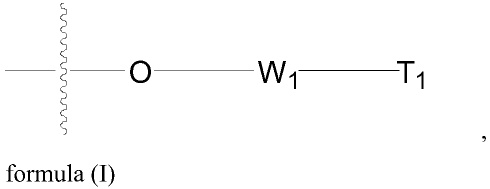
where W₁ is selected from a direct bond or 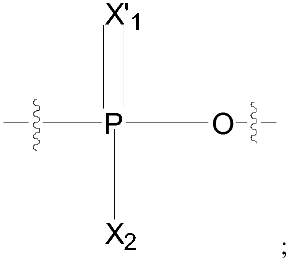
X'₁ is selected from an O or S atom, or is absent,
when X'₁ is selected from an O or S atom, X₂ is selected from -O-, -S-, -SH, -OH (hydroxyl group), -NH₂ (amino group), a C₁-C₆ alkyl group, a C₁-C₆ alkoxy group, or -O-(CH₂)_{n'}-OR'₅, where R'₅ is selected from H, a direct bond, or 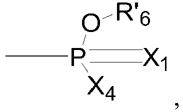 R'₆ is H or a direct bond, X₁ is selected from an O or S atom, X₄ is -OH or -SH, and n' is an integer from 1 to 10; or when X'₁ is absent, X₂ is a direct bond;
T₁ is selected from -(CH₂)ₘCH₃ or 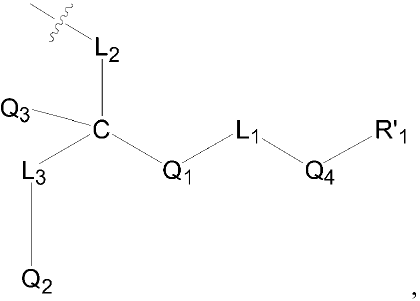 where m is an integer from 10 to 30;
where Q₁ and Q₄ are each independently selected from a direct bond, -NH₂ (amino group), -COOH (carboxyl group), an amide group (-NHCO- or -CONH-), -O-, -S-, -S-S-, a phosphate group, or a phosphorothioate group;
Q₂ is selected from -SH, -OH (hydroxyl group), -NH₂ (amino group), -H, or a C₁-C₆ alkyl group, preferably, -CH₃ (methyl group), -COOH (carboxyl group), an amide group (-NHCO- or -CONH-), -O-, -S-, -S-S-, a phosphate group, a phosphorothioate group, or 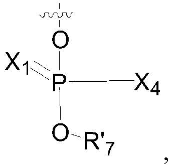 where R'₇ is H or a direct bond; and definitions of X₁ and X₄ are the same as defined above;
Q₃ is selected from -H or a C₁-C₁₀ alkyl group;
L₁ is -(CH₂)₁-(NR'₄)ₜ-(CH₂)_{q}-, where 1 and q are each an integer from 0 to 10, and 1+q=1 to 10; t is 0 or 1; R'₄ is -CO(CH₂)ᵣCOOH; and r is an integer from 10 to 30;
L₂ and L₃ are each independently selected from a C₁-C₁₀ saturated alkyl chain or a direct bond;
R'₁ is selected from a C₁₀-C₃₀ saturated fatty acid chain, a C₁₀-C₃₀ saturated alkyl chain, a C₁₀-C₃₀ unsaturated hydrocarbon group, or -(CH₂)ₘ-X₃-R'₃, where m is an integer from 10 to 30; X₃ is selected from a direct bond, an oxygen atom or a sulfur atom; R'₃ is selected from a saturated six-membered heterocyclic group containing nitrogen and oxygen, H, a direct bond, or 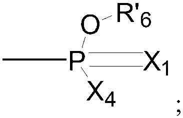 and definitions of R'₆, X₁ and X₄ are the same as defined above; and when X₃ is a direct bond, R'₃ is not H or a direct bond;
when W₁ is a direct bond, T₁ is not -(CH₂)ₘCH₃; 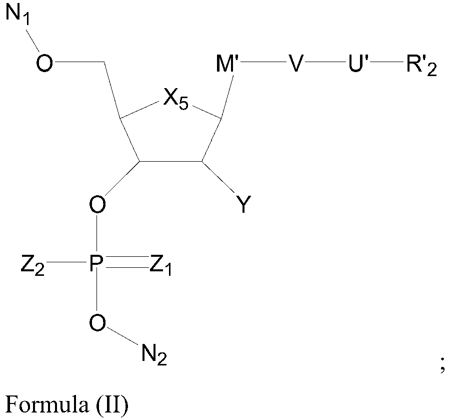 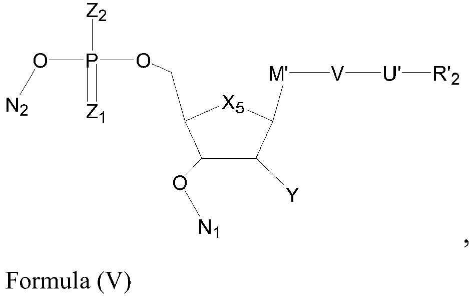
in formula (II) and formula (V), the five-membered ring is a five-membered sugar ring structure of ribose or deoxyribose, where X₅ is selected from -CH₂-, -CH(CH₃)-, -C(CH₃)₂-, -O-, -NH-, -N(CH₃)-, or -S-;
M' is selected from H, -O-, -C-, or a modified or unmodified nucleobase;
N₁ is selected from a direct bond, H, a C₁-C₃ alkyl group, or 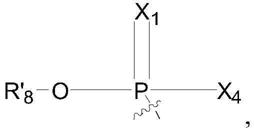 where R'₈ is H or a direct bond; and definitions of X₁ and X₄ are the same as defined above;
N₂ is selected from a direct bond, H, or a C₁-C₃ alkyl group;
Y is selected from H, -NH₂, -OH, halogen, a C₁-C₆ alkyl group, a C₁-C₆ haloalkyl group, -O-R'₉, or -O-(CH₂)ₙ-O-R'₁₀, wherein R'₉ is a C₁-C₆ alkyl group, preferably -O-CH₃; n is an integer from 1 to 6; R'₁₀ a C₁-C₆ alkyl group, preferably, n is 2 and R'₁₀ is a C₁ alkyl group, namely 2'-methoxyethoxy group;
V is selected from a C₁-C₄ saturated alkyl chain or is absent;
U' is selected from -NH₂ (amino group), -COOH (carboxyl group), or an amide group (-NHCO- or -CONH-), or is absent;
Z₁ is selected from an O or S atom;
Z₂ is selected from a C₁₀-C₃₀ alkoxy group or a fatty acid chain, preferably, a fatty acid with a terminal carboxyl group, an amide lipid chain, an alkenyl chain, or an alkyl chain; and
R'₂ is selected from a C₁₀-C₃₀ alkoxy group or a fatty acid chain, preferably, is selected from a fatty acid with a terminal carboxyl group, an amide lipid chain, an alkenyl chain, or an alkyl chain; or is absent.

Embodiment 2. The lipid compound according to Embodiment 1, which has a structure represented by formula (I),
when W₁ is 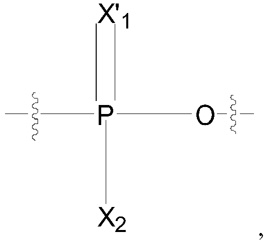 X'₁ is selected from an O or S atom;
T₁ is 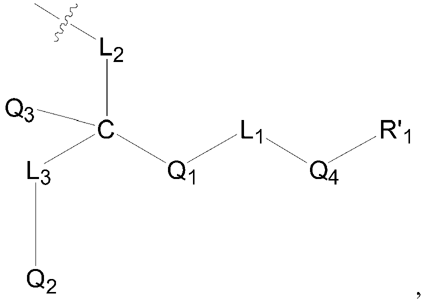
where:
Q₁ and Q₄ are selected from a direct bond, -NH₂ (amino group), -COOH (carboxyl group), an amide group (-NHCO- or -CONH-), -O-, -S-, -S-S-, a phosphate group, or a phosphorothioate group;
Q₂ is selected from -SH, -OH (hydroxyl group), -NH₂ (amino group), -H, or a C₁-C₆ alkyl group, preferably, -CH₃ (methyl group), -COOH (carboxyl group), an amide group (-NHCO- or -CONH-), -O-, -S-, -S-S-, a phosphate group, a phosphorothioate group, or 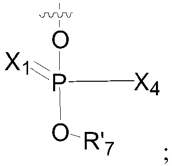
Q₃ is selected from -H or a C₁-C₁₀ alkyl group;
L₂ and L₃ are selected from a C₁-C₁₀ saturated alkyl chain or a direct bond;
X₁ is selected from an O or S atom;
X₂ is selected from -O-, -S-, -SH, -OH (hydroxyl group), -NH₂ (amino group), or a C₁-C₆ alkyl group, preferably, -CH₃ (methyl group), -CH₂CH₃ (ethyl group), or a C₁-C₆ alkoxy group, more preferably, -OCH₃ (methoxy group), -OCH₂CH₃ (ethoxy group), or -O-(CH₂)ₙ-OR'₅, where R'₅ is selected from H, a direct bond, or 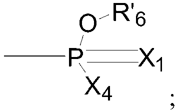 and
R'₁ is selected from a C₁₀-C₃₀ saturated fatty acid chain, a C₁₀-C₃₀ saturated alkyl chain, a C₁₀-C₃₀ unsaturated hydrocarbon group, or -(CH₂)ₘ-X₃-R'₃, where m is an integer from 10 to 30; X₃ is selected from a direct bond, an oxygen atom or a sulfur atom; and R'₃ is a saturated six-membered heterocyclic group containing nitrogen and oxygen, H, a direct bond, or

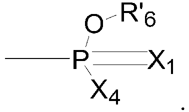

Embodiment 3. The lipid compound according to Embodiment 2, both Q₁ and Q₄ are amide groups (-NHCO- or -CONH-); and Q₂ is selected from -SH, -OH (hydroxyl group), -S-, -O-, or 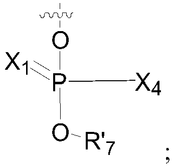 and
R'₁ is selected from a C₁₀-C₃₀ saturated fatty acid chain or a C₁₀-C₃₀ saturated alkyl chain, preferably, a C₁₃-C₁₆ saturated alkyl chain, more preferably, a C₁₅ saturated alkyl chain, a C₁₀-C₃₀ unsaturated hydrocarbon group, or -(CH₂)ₘ-X₃-R'₃, where m is an integer from 10 to 30, preferably, an integer from 2 to 5, more preferably, 3; X₃ is selected from a direct bond, an oxygen atom or a sulfur atom; and R'₃ is a saturated six-membered heterocyclic group containing nitrogen and oxygen, H, a direct bond, or

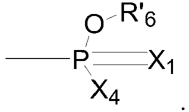

Embodiment 4. The lipid compound according to Embodiment 3, where L₁ is - (CH₂)₁-(NR'₄)ₜ-(CH₂)_{q}-, where t is 0.

Embodiment 5. The lipid compound according to Embodiment 3, where L₁ is - (CH₂)₁-(NR'₄)ₜ-(CH₂)_{q}-, where 1+q=1 to 10 and t is 1;
L₂ is selected from a C₁-C₁₀ saturated alkyl chain or a direct bond;
L₃ is a direct bond; and
Q₂ is H;
Q₃ is selected from -H or a C₁-C₁₀ alkyl group; and
R'₁ is -(CH₂)ₘ-X₃-R'₃, where m is an integer from 10 to 30; X₃ is selected from an oxygen atom or a sulfur atom; and R'₃ is H, a direct bond, or

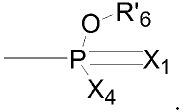

Embodiment 6. The lipid compound according to Embodiment 2, where Q₁ is a direct bond and Q₄ is an amide group (-NHCO- or -CONH-);
L₁ is -(CH₂)₁-(NR'₄)ₜ-(CH₂)_{q}-, where l+q=1 to 10 and t is 0;
L₂ is a C₁-C₁₀ saturated alkyl chain;
L₃ is a direct bond; and
Q₂ is selected from -SH, -OH (hydroxyl group), -S-, -O-, or 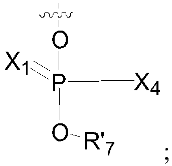 and definitions of R'₇ and X₄ are the same as defined above.

Embodiment 7. The lipid compound according to any one of Embodiments 1 to 6, where W₁ is a direct bond; Q₂ is selected from -SH, -OH (hydroxyl group), or 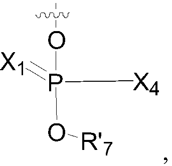 where R'₇ is H; and R'₆ in R'₁ is not a direct bond;
W₁ is a direct bond; Q₂ is selected from -S-, -O-, or 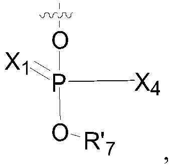 where R'₇ is a direct bond; and R'₆ in R'₁ is not a direct bond;
W₁ is a direct bond; Q₂ is not -S-, -O-, or -S-S-, and when Q₂ is 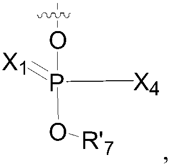 R'₇ is H; and R'₁ is -(CH₂)ₘ-X₃-R'₃, where R'₃ is a direct bond or 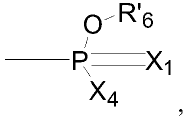 where R'₆ is a direct bond;
W₁ is 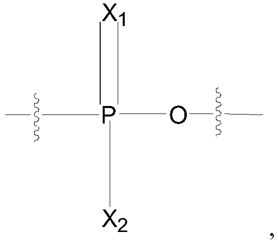 where X₂ is selected from -OH or -SH; Q₂ is selected from -SH or -OH (hydroxyl group); and R'₃ and R'₆ in R'₁ are not direct bonds;
W₁ is 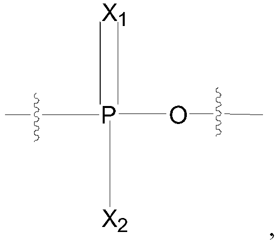 where X₂ is selected from -OH or -SH; Q₂ is selected from -S-, -O-, or 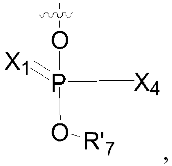 where R'₇ is a direct bond; and R'₃ and R'₆ in R'₁ are not direct bonds; or
W₁ is 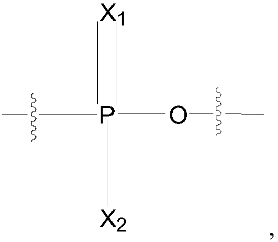 where X₂ is selected from -OH or -SH; Q₂ is not -S-, -O-, or -S-S-; and when Q₂ is 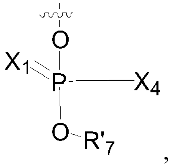 R'₇ is H; and R'₃ in R'₁ is a direct bond; or when R'₃ is 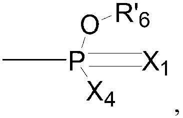 R'₆ is a direct bond;
preferably, W₁ is a direct bond; the wavy line in formula (I) is linked to X₆, and X₆ has the following structure 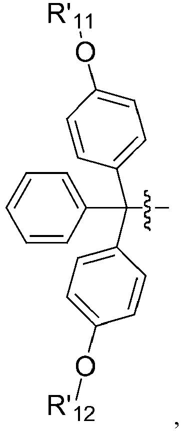 where R'₁₁ and R'₁₂ are each independently selected from a C₁-C₆ alkyl group; Q₁ is a direct bond or an amide group (-NHCO- or -CONH-), and Q₄ is an amide group (-NHCO- or -CONH-); Q₂ is selected from -S-, -O- or H; L₁ is - (CH₂)₁-(NR'₄)ₜ-(CH₂)_{q}-, where 1 and q are integers from 0 to 10, l+q=1 to 10, and t is 0 or 1, and when Q₂ is H, L₃ is a direct bond and t is 1; when Q₂ is -S- or -O-, Q₂ is linked to X₇, and X₇ is selected from (B'1) or (B'2), 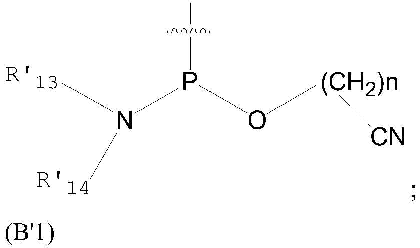
where R'₁₃ and R'₁₄ are each independently selected from a C₁-C₆ alkyl group, and n is an integer from 1 to 6; 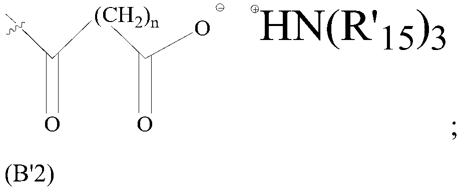
where R'₁₅ is a C₁-C₆ alkyl group; R'₁ is selected from a C₁₀-C₃₀ saturated alkyl chain, a C₁₀-C₃₀ unsaturated hydrocarbon group, or -(CH₂)ₘ-X₃-R'₃, where m is an integer from 10 to 30, X₃ is selected from a direct bond, an oxygen atom, or a sulfur atom, and R'₃ is selected from a saturated six-membered heterocyclic group containing nitrogen and oxygen, or a direct bond, and when Q₂ is H, R'₃ is a direct bond, and R'₃ is linked to 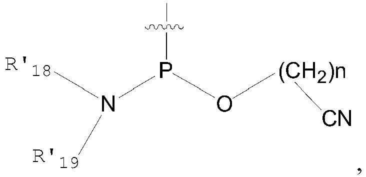 where R'₁₈ and R'₁₉ are each independently selected from a C₁-C₆ alkyl group, and n is an integer from 1 to 6; or
preferably, W₁ is 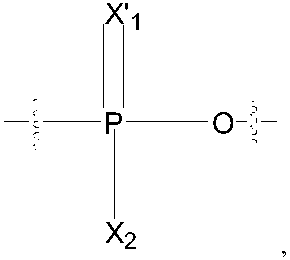 wherein X'₁ is absent, and X₂ is a direct bond; the wavy line in formula (I) is linked to -(CH₂)ₙ-O-X₆, where n is an integer from 1 to 6 and the definition of X₆ is the same as defined above; T₁ is -(CH₂)ₘCH₃; and X₂ is linked to -N(R'₂₀)₂, where R'₂₀ is a C₁-C₆ alkyl group, preferably, a C₃ alkyl group, more preferably, an isopropyl group.

Embodiment 8. The lipid compound according to any one of Embodiments 1 to 7, in formula (I), R'₁ is a C₁₀-C₃₀ saturated fatty acid chain, and the C₁₀-C₃₀ saturated fatty acid chain is -(CH₂)ₘ-COOH or -(CH₂)ₘ-COOR'₁₆, where m is an integer from 10 to 30; R'₁₆ is a C₁-C₆ alkyl group or 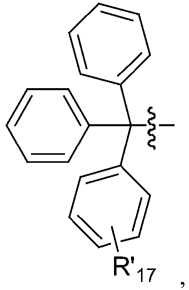 preferably 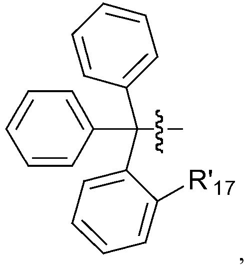 and the C₁-C₆ alkyl group is preferably a methyl group, an ethyl group, an isopropyl group or a tert-butyl group, and R'₁₇ is a halogen, preferably Cl;
R'₁ is a C₁₀-C₃₀ unsaturated hydrocarbon group, and the C₁₀-C₃₀ unsaturated hydrocarbon group is -(CH₂)ₘ-R'₄, where m is an integer from 10 to 30, and R'₄ is an unsaturated bond, preferably a triple bond; or
R'₁ is -(CH₂)ₙ-X₃-R'₃, where R'₃ is a saturated six-membered heterocyclic group containing nitrogen and oxygen, and X₃ is bonded to the nitrogen atom of R'₃, preferably, in R'₃, the six-membered heterocyclic ring contains one nitrogen atom and one oxygen atom, and the nitrogen atom and the oxygen atom are located at para positions of the six-membered heterocyclic ring; and/or
X₂ is selected from -OH, -SH, -CH₃ (methyl group), -CH₂CH₃ (ethyl group), -OCH₃ (methoxy group) or -OCHH₂CH₃ (ethoxy group), preferably, -OH or -SH.

Embodiment 9. The lipid compound according to Embodiment 1, where W₁ is ; 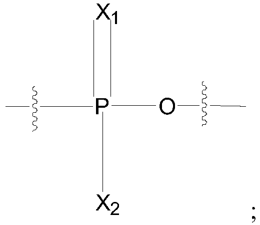 X'₁ is O or S; X₂ is -(CH₂)_{n'}-OR'₅, where R'₅ is selected from H, a direct bond, or 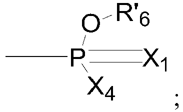 the definitions of X₁, R'₆, and X₄ are the same as defined above; n' is an integer from 1 to 10; and T₁ is -(CH₂)ₘCH₃, where m is an integer from 10 to 30.

Embodiment 10. The lipid compound according to Embodiment 9, where R'₅ is selected from a direct bond or 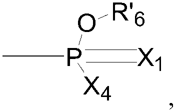 where R'₆ is a direct bond.

Embodiment 11. The lipid compound according to any one of Embodiments 1 to 10, where the wavy line in formula (I) is linked to H or X₆.

Embodiment 12. The lipid compound according to Embodiment 1, which has a structure represented by the formula (II) or formula (V), where N₁ is a direct bond, H, or 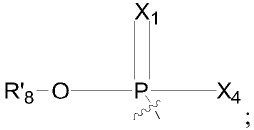
N₂ is selected from a direct bond or H;
Y is a C₁-C₆ alkoxy group;
M' is selected from -O-, -C-, or a modified or unmodified nucleobase; and
when M' is a modified or unmodified nucleobase, U', V, and R'₂ are all absent; preferably, M' is independently selected from adenine, uracil, thymine, guanine, or cytosine; more preferably, M' is 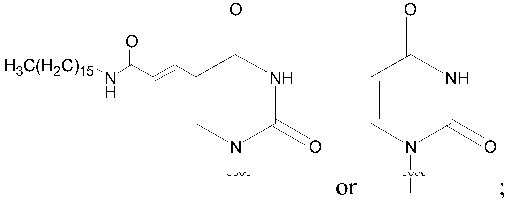 or
when M' is -O- or -C-, V is a C₁-C₄ saturated alkyl chain, U' is an amide group (-NHCO- or -CONH-), and R'₂ is an alkyl chain.

Embodiment 13. The lipid compound according to Embodiment 12, where M' is independently selected from adenine, uracil, thymine, guanine, or cytosine; preferably,

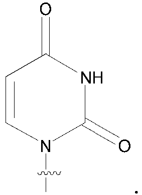

Embodiment 14. The lipid compound according to Embodiment 12 or 13, which has a structure represented by the formula (II) or formula (V), where X₅ is O or S.

Embodiment 15. The lipid compound according to any one of Embodiments 12 to 14, where N₁ is not a direct bond, R'₈ is not a direct bond, and N₂ is a direct bond; preferably, N₂ is linked to X₇;
N₁ is a direct bond or 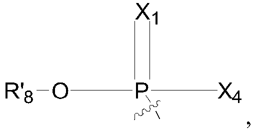 where R'₈ is a direct bond, and N₂ is not a direct bond; preferably, N₁ is linked to X₆; or
N₁ is a direct bond or 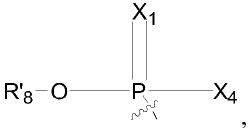 R'₈ is a direct bond, and N₂ is a direct bond; preferably, N₁ is linked to X₆ and N₂ is linked to X₇.

Embodiment 16. The lipid compound according to any one of Embodiments 12 to 15, when N₁, N₂ or R'₈ is a direct bond, N₁, N₂ or R'₈ is connected to H.

Embodiment 17. The lipid compound according to Embodiment 1, which is selected from at least one of the following structures (L1) to (L36), or (L'10):

**Table 1**

| | | | |
|---|---|---|---|
| L1 | 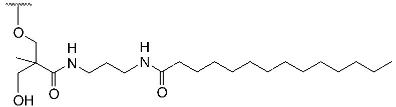 | L21 | 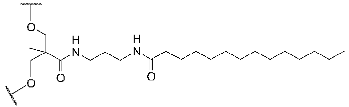 |
| L2 | 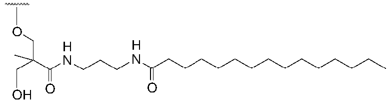 | L22 | 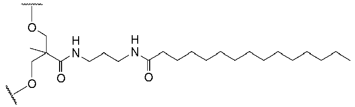 |
| L3 | 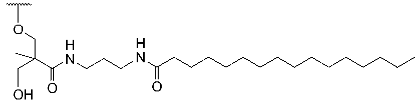 | L17 | 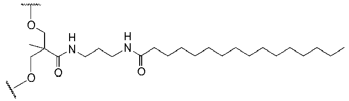 |
| L4 | 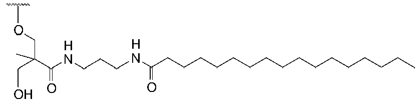 | L23 | 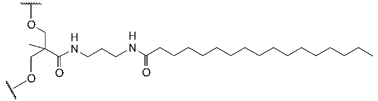 |
| L5 | 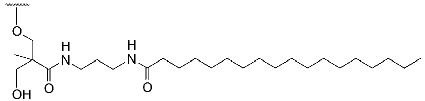 | L24 | 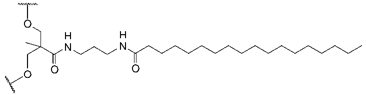 |
| L6 | 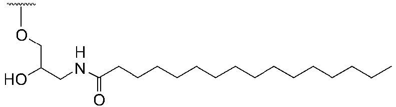 | L25 | 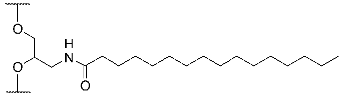 |
| L7 | 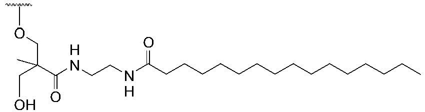 | L26 | 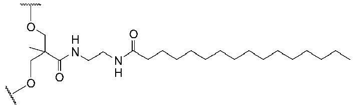 |
| L8 | 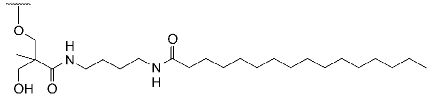 | L27 | 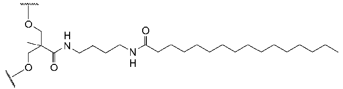 |
| L9 | 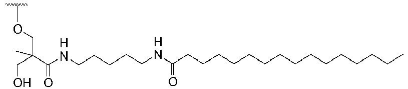 | L28 | 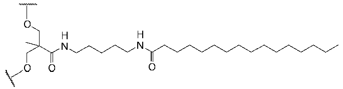 |
| L10 | 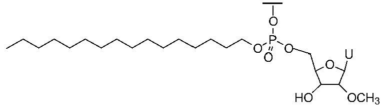 | L'10 | 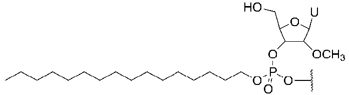 |
| L11 | 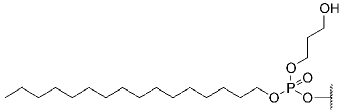 | L29 | 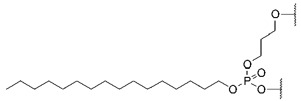 |
| L12 | 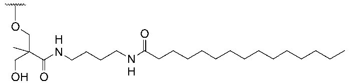 | L30 | 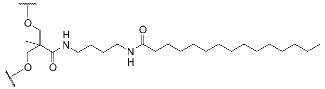 |
| L13 | 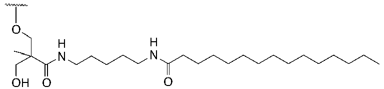 | L31 | 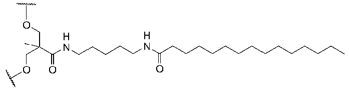 |
| L14 | 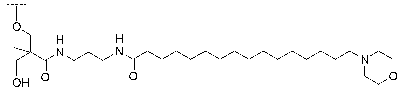 | L32 | 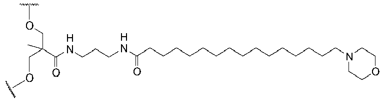 |
| L15 | 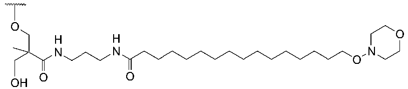 | L33 | 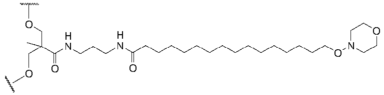 |
| L16 | 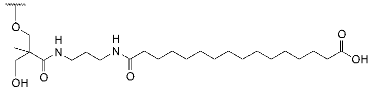 | L19 | 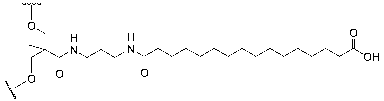 |
| L34 | 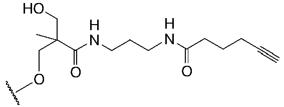 | L18 | 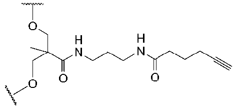 |
| L35 | 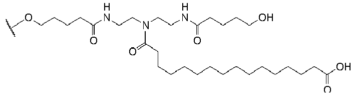 | L20 | 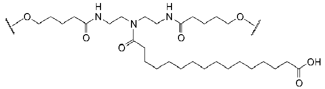 |
| L36 | 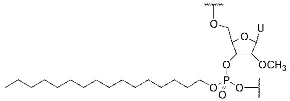 | | |

where U is 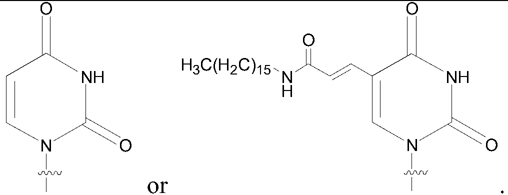 Upon understanding the disclosure of the present application, a person skilled in the art can reasonably infer that L'10 and L'10' shown below may be used to synthesize the nucleic acid conjugate, and L'10' may be used to synthesize conjugate L'10": 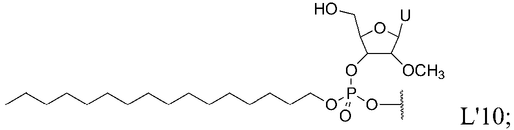 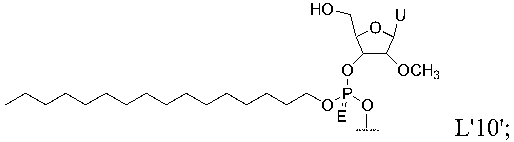 and 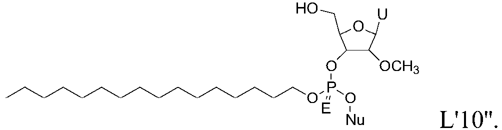 Based on the disclosure of the present application, a person skilled in the art can also reasonably infer that conjugates in which U is replaced by other bases (for example, A, G, and C) may be prepared using a method similar to that for preparing L10", and the conjugates can also achieve the technical effects of the present disclosure and resolve the technical problems of the present disclosure.

Embodiment 18. The lipid compound according to Embodiment 1, which is selected from at least one of the following structures (L1') to (L36') or (L'10'):

**Table 2**

| | | | |
|---|---|---|---|
| L1' | 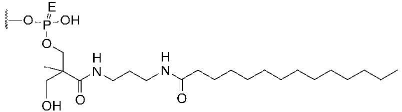 | L21' | 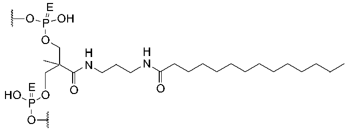 |
| L2' | 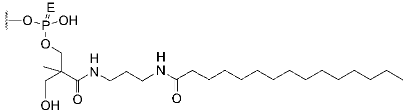 | L22' | 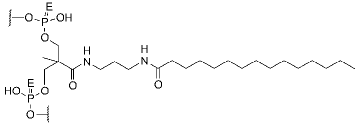 |
| L3' | 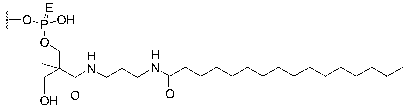 | L17' | 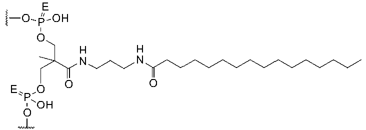 |
| L4' | 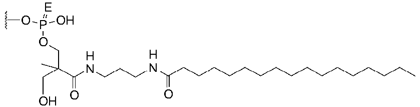 | L23' | 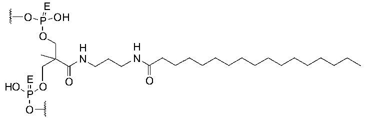 |
| L5' | 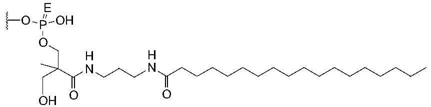 | L24' | 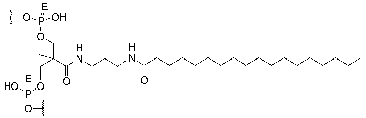 |
| L6' | 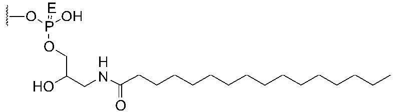 | L25' | 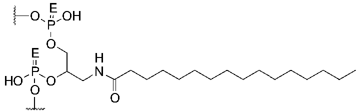 |
| L7' | 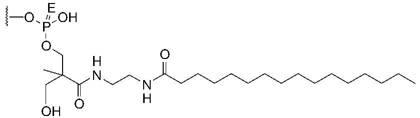 | L26' | 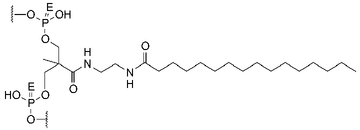 |
| L8' | 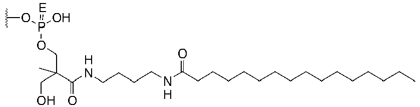 | L27' | 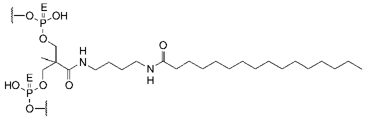 |
| L9' | 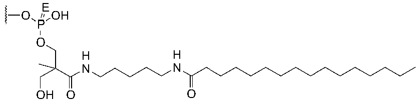 | L28' | 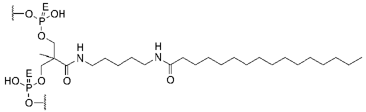 |
| L10' | 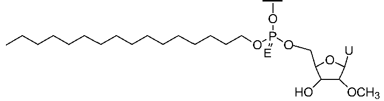 | L'10' | 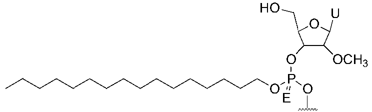 |
| L11' | 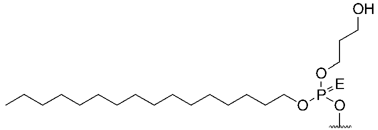 | L29' | 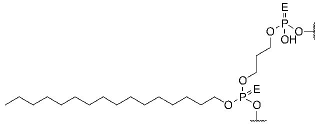 |
| L12' | 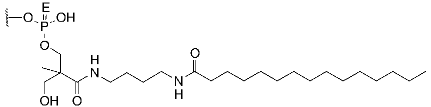 | L30' | 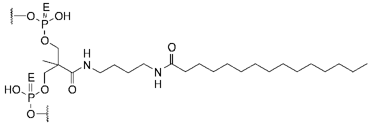 |
| L13' | 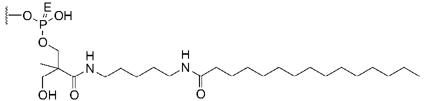 | L31' | 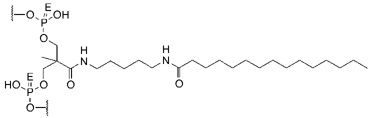 |
| L14' | 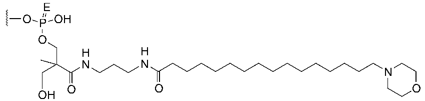 | L32' | 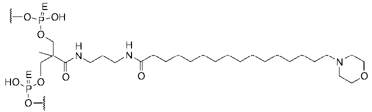 |
| L15' | 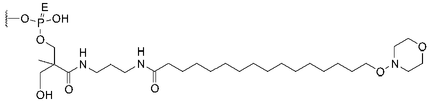 | L33' | 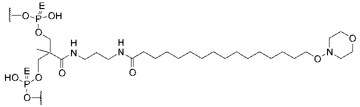 |
| L16' | 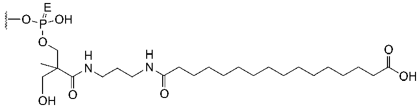 | L19' | 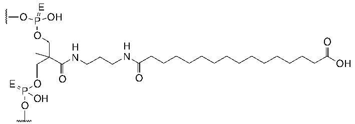 |
| L34' | 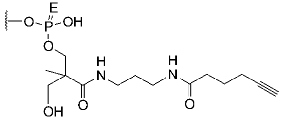 | L18' | 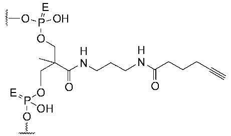 |
| L35' | 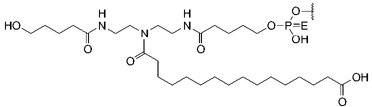 | L20' | 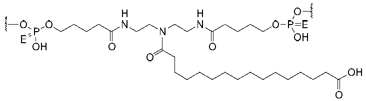 |
| L36' | 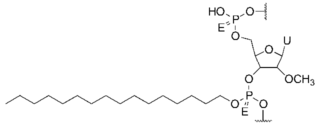 | | |

where E is selected from O and S, and U is 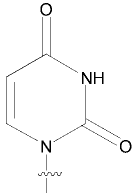 or

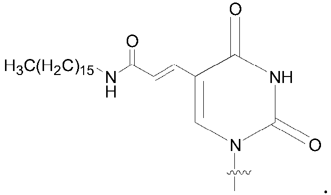

Embodiment 19. A nucleic acid conjugate comprising a nucleic acid and a conjugate moiety conjugated to the nucleic acid,
the conjugate moiety is selected from the lipid compound according to any one of Embodiments 1 to 18.

Embodiment 20. The nucleic acid conjugate according to Embodiment 19, where the conjugate moiety is conjugated to a phosphate group of the nucleic acid or a hydroxyl group of the ribose.

Embodiment 21. The nucleic acid conjugate according to Embodiment 20, which has the following structure:

Nu-O-W₁-T₁; Formula (III)

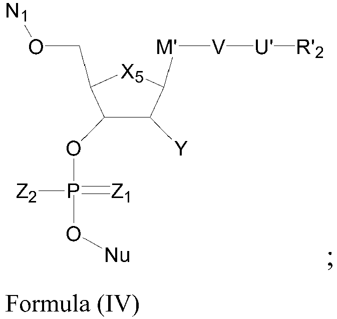

or 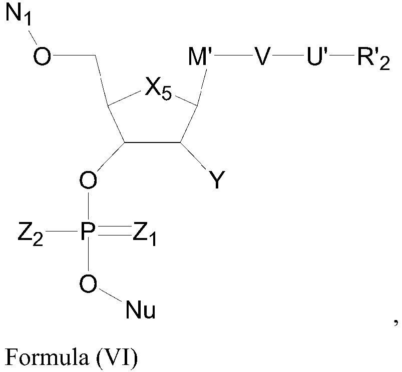 where Nu is a nucleic acid or a nucleic acid fragment, and definitions of other variables are the same as defined in Embodiments 1 to 18. A person skilled in the art can reasonably exclude the relevant technical solutions in the foregoing Embodiments 1 to 18 that cannot be used to conjugate the nucleic acid or the nucleic acid fragment.

Embodiment 22. The nucleic acid conjugate according to Embodiment 21, which has a structure of the formula (III) or (VI), where X₂ is -O-(CH₂)_{n'}-OR'₅, where R'₅ is selected from a direct bond or 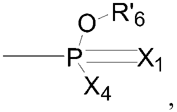 where R'₆ is a direct bond, and n' is an integer from 1 to 10; or
Q₂ is selected from -O-, -S-, or 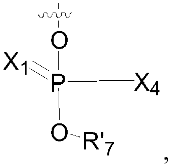 where R'₇ is a direct bond; or
R'₁ is -(CH₂)ₘ-X₃-R'₃, where m is an integer from 10 to 30; X₃ is selected from an oxygen atom, a sulfur atom, a direct bond or 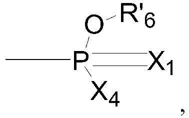 where R'₆ is a direct bond;
   or
the nucleic acid conjugate has a structure of formula (IV) or (VI), where N₁ is a direct bond or 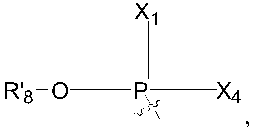 where R'₈ is a direct bond.

Embodiment 23. The nucleic acid conjugate according to Embodiment 21, where Nu is a nucleic acid or a nucleic acid fragment, and definitions of other variables are the same as defined in any one of Embodiments 7 to 10, Embodiments 12 to 15, and Embodiments 17 to 28.

Embodiment 24. The nucleic acid conjugate according to any one of Embodiments 21 to 23, where the direct bond is conjugated to the nucleic acid or nucleic acid fragment. The direct bond comprises those in "-O-" and "-S-" that are capable of conjugating to the nucleic acid and fragment thereof. A person skilled in the art can reasonably understand that the direct bond does not comprise those incapable of conjugating to the nucleic acid or nucleic acid fragment.

Embodiment 25. The nucleic acid conjugate according to any one of Embodiments 21 to 24, where the nucleic acid is selected from a single-stranded nucleic acid or a fragment thereof, or a double-stranded nucleic acid or a fragment thereof; preferably, a length of the double-stranded nucleic acid or the fragment thereof is 12-30 mer, and the double-stranded nucleic acid or the fragment thereof is preferably siRNA or a fragment thereof; preferably, a molecular weight of the double-stranded nucleic acid or the fragment thereof ranges from 6000 to 20000 Daltons; a length of the single-stranded nucleic acid or the fragment thereof is 12-30 mer, and the single-stranded nucleic acid or the fragment thereof is preferably a single-stranded phosphorothioate oligonucleotide or a fragment thereof; preferably, a molecular weight of the single-stranded nucleic acid or the fragment thereof ranges from 3000 to 10000 Daltons.

Embodiment 26. The nucleic acid conjugate according to Embodiment 25, where each nucleotide in the nucleic acid is independently a modified or unmodified nucleotide; or two adjacent nucleotides in the nucleic acid are linked via a phosphodiester bond, and one or more of the phosphodiester bonds are phosphorothioate diester bonds.

Embodiment 27. The nucleic acid conjugate according to Embodiment 25 or 26, where each nucleotide in the nucleic acid is independently a nucleotide with fluorine-substitution modification or a nucleotide with non-fluorine-substitution modification;
preferably, the fluorine-substitution modification is that the hydroxy group at the 2'-position of the pentose of the nucleotide is substituted with F; and
preferably, the non-fluorine-substitution modification is that the hydroxy group at the 2'-position of the pentose of the nucleotide is substituted with an alkoxy group, and the hydroxy group at the 2'-position is preferably substituted with a methoxy group or a 2'-methoxyethoxy group.

Embodiment 28. The nucleic acid conjugate according to any one of Embodiments 25 to 27, where the conjugate moiety is conjugated to the double-stranded nucleic acid; and the double-stranded nucleic acid comprises a sense strand and an antisense strand, preferably, the conjugate moiety is conjugated to the 3' or 5' end of the sense strand or the antisense strand; more preferably, the conjugate moiety is conjugated to the 3' end of the sense strand.

Embodiment 29. The nucleic acid conjugate according to any one of Embodiments 25 to 27, where the conjugate moiety is conjugated to the double-stranded nucleic acid on one end, and conjugated to the single-stranded nucleic acid on the other end; preferably, the conjugate moiety is conjugated to the sense strand of the double-stranded nucleic acid on one end, and conjugated to the single-stranded nucleic acid on the other end to form the sense strand of the nucleic acid conjugate (for example, SN-17001 or SN-17024); preferably, the single-stranded nucleic acid is located at the 3' end (for example, SN-17024) or 5' end (for example, SN-17001) of the sense strand of the nucleic acid conjugate; and preferably, the single-stranded nucleic acid is located at the 3' end of the sense strand of the nucleic acid conjugate.

Embodiment 30. The nucleic acid conjugate according to Embodiment 28 or 29, where a sequence of the sense strand is selected from the following sequences:
1) CAUUUUAAUCCUCACUCUAAA (SEQ ID NO: 1 in the sequence listing);
2) GCUCAGCAUUGCCUGAAUAAA (SEQ ID NO: 2 in the sequence listing); or
3) UGCAAAUAGUCUACAAACCAA (SEQ ID NO: 3 in the sequence listing); and
   a sequence of the antisense strand is selected from the following sequences:
4) UUUAGAGUGAGGAUUAAAAUGAG (SEQ ID NO: 4 in the sequence listing);
5) UUUAUUCAGGCAAUGCUGAGCUU (SEQ ID NO: 5 in the sequence listing); or
6) UUGGUUUGUAGACUAUUUGCACA (SEQ ID NO: 6 in the sequence listing).

Embodiment 31. The nucleic acid conjugate according to any one of Embodiments 25 to 27, and 29 to 30, the single-stranded nucleic acid comprises a sequence selected from the following:
CCGTCGCCCTTCAGCACGCA (SEQ ID NO: 7 in the sequence listing);
CGTCGCCCTTCAGCACGC (SEQ ID NO: 8 in the sequence listing);
GTCGCCCTTCAGCACG (SEQ ID NO: 9 in the sequence listing); or
TCGCCCTTCAGCAC (SEQ ID NO: 10 in the sequence listing). Preferably, the single-stranded nucleic acid comprises TCGCCCTTCAGCAC. More preferably, the single-stranded nucleic acid is the above sequence.

Embodiment 32. The conjugate according to Embodiment 19, which is selected from at least one of the following structures (L1") to (L36"), or (L'10"):

**Table 3**

| | | | |
|---|---|---|---|
| L1" | 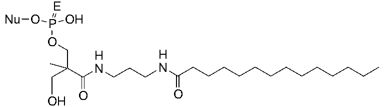 | L21" | 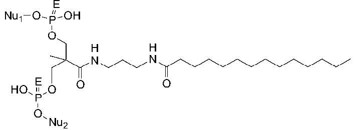 |
| L2" | 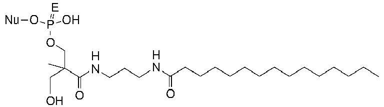 | L22" | 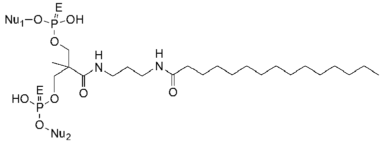 |
| L3" | 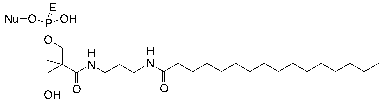 | L17" | 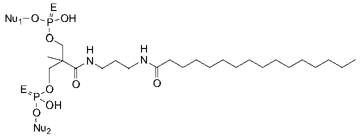 |
| L4" | 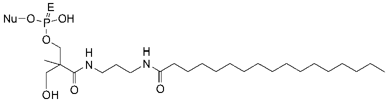 | L23" | 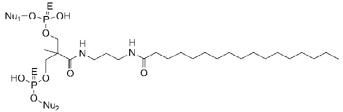 |
| L5" | 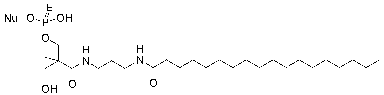 | L24" | 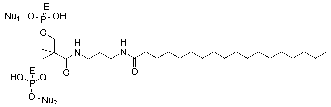 |
| L6" | 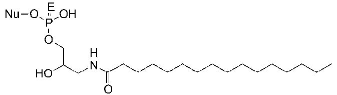 | L25" | 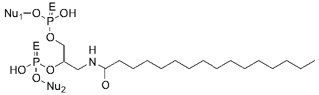 |
| L7" | 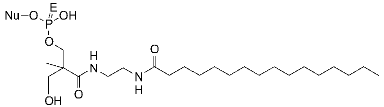 | L26" | 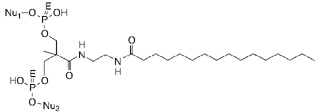 |
| L8" | 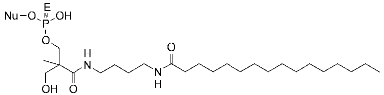 | L27" | 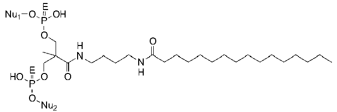 |
| L9" | 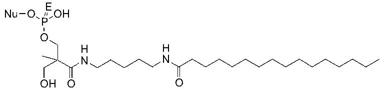 | L28" | 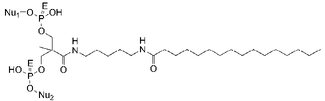 |
| L10" | 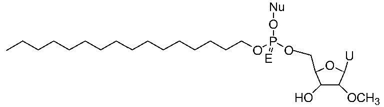 | L'10" | 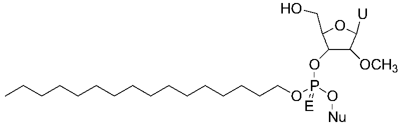 |
| L11" | 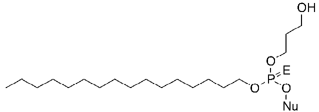 | L29" | 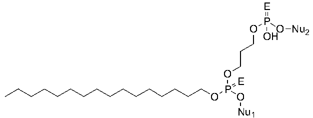 |
| L12" | 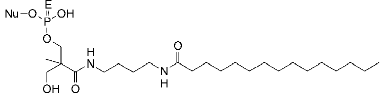 | L30" | 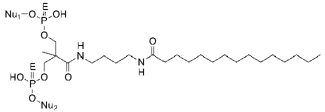 |
| L13" | 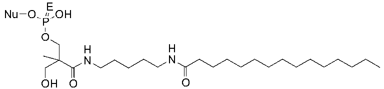 | L31" | 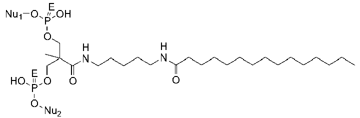 |
| L14" | 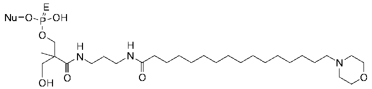 | L32" | 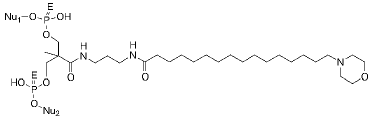 |
| L15" | 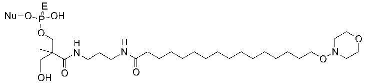 | L33" | 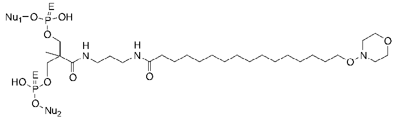 |
| L16" | 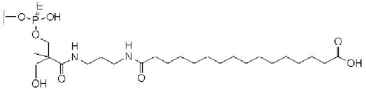 | L19" | 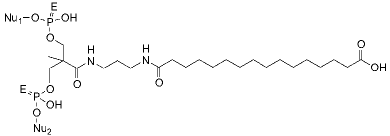 |
| L34" | 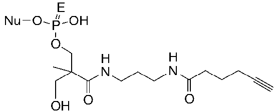 | L18" | 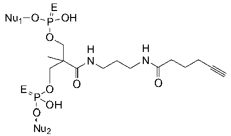 |
| L35" | 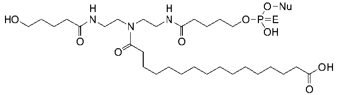 | L20" | 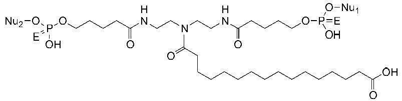 |
| L36" | 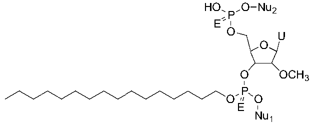 | | |

in the above table, Nu, Nu₁ and Nu₂ each independently represents a nucleic acid or a fragment of a nucleic acid; and Nu, Nu₁ and Nu₂ may be the same or different;
where E is selected from O or S.

Embodiment 33. The nucleic acid conjugate according to Embodiment 19, which has a structure selected from a group consisting of:

**Table 4**

| | |
|---|---|
| SN-16981 | ss: 5'-mCsmAsmUmUmUmUAfmAUfCfCfmUmCmAmCmUmCmUmAsmAsmA-3'-L1'; |
| | as: 5'-mUsUfsmUmAmGAfmGUfGfmAmGmGmAUfmUAfmAmAmAmUmGsmAsmG-3'; |
| SN-16982 | ss: 5'-mCsmAsmUmUmUmUAfmAUfCfCfmUmCmAmCmUmCmUmAsmAsmA-3'-L2'; |
| | as: 5'-mUsUfsmUmAmGAfmGUfGfmAmGmGmAUfmUAfmAmAmAmUmGsmAsmG-3'; |
| SN-16983 | ss: 5'-mCsmAsmUmUmUmUAfmAUfCfCfmUmCmAmCmUmCmUmAsmAsmA-3'-L3'; |
| | as: 5'-mUsUfsmUmAmGAfmGUfGfmAmGmGmAUfmUAfmAmAmAmUmGsmAsmG-3'; |
| SN-17011 | ss: 5'-mGsmCsmUmCmAmGmCfmAmUfmUfmGfmCmCmUmGmAmAmUmAmAmA-3'- L3'; as: 5'- mUsmUfsmUmAfmUmUfmCmAmGmGmCmAmAmUfmGmCfmUmGmAmGmCsmUsmU- 3'; |
| SN-16984 | ss: 5'-mCsmAsmUmUmUmUAfmAUfCfCfmUmCmAmCmUmCmUmAsmAsmA-3'-L4'; |
| | as: 5'-mUsUfsmUmAmGAfmGUfGfmAmGmGmAUfmUAfmAmAmAmUmGsmAsmG-3'; |
| SN-17012 | ss: 5'-mCsmAsmUmUmUmUAfmAUfCfCfmUmCmAmCmUmCmUmAsmAsmA-3'-L5'; |
| | as: 5'-mUsUfsmUmAmGAfmGUfGfmAmGmGmAUfmUAfmAmAmAmUmGsmAsmG-3'; |
| SN-16985 | ss: 5'-mCsmAsmUmUmUmUAfmAUfCfCfmUmCmAmCmUmCmUmAsmAsmA-3'-L6'; |
| | as: 5'-mUsUfsmUmAmGAfmGUfGfmAmGmGmAUfmUAfmAmAmAmUmGsmAsmG-3'; |
| SN-16986 | ss: 5'-mCsmAsmUmUmUmUAfmAUfCfCfmUmCmAmCmUmCmUmAsmAsmA-3'-L7'; |
| | as: 5'-mUsUfsmUmAmGAfmGUfGfmAmGmGmAUfmUAfmAmAmAmUmGsmAsmG-3'; |
| SN-16987 | ss: 5'-mCsmAsmUmUmUmUAfmAUfCfCfmUmCmAmCmUmCmUmAsmAsmA-3'-L8'; |
| | as: 5'-mUsUfsmUmAmGAfmGUfGfmAmGmGmAUfmUAfmAmAmAmUmGsmAsmG-3'; |
| SN-16988 | ss: 5'-mCsmAsmUmUmUmUAfmAUfCfCfmUmCmAmCmUmCmUmAsmAsmA-3'-L9'; |
| | as: 5'-mUsUfsmUmAmGAfmGUfGfmAmGmGmAUfmUAfmAmAmAmUmGsmAsmG-3'; |
| SN-17028 | ss: 5'-mCsmAsmUmUmUmUAfmAUfCfCfmUmCmAmCmUmCmUmAsmA-(L10')-3'; |
| | as: 5'-mUsUfsmUmAmGAfmGUfGfmAmGmGmAUfmUAfmAmAmAmUmGsmAsmG-3'; |
| SN-16991 | ss: 5'-mCsmAsmUmU-(L36')-mUAfmAUfCfCfmUmCmAmCmUmCmUmAsmAsmA-3 '; |
| | as: 5'-mUsUfsmUmAmGAfmGUfGfmAmGmGmAUfmUAfmAmAmAmUmGsmAsmG-3'; |
| SN-16992 | ss: 5'-mCsmAsmUmUmUmUAfmAUfCfCfmUmCmAmCmUmCmUmAsmAsmA-3'-L11'; |
| | as: 5'-mUsUfsmUmAmGAfmGUfGfmAmGmGmAUfmUAfmAmAmAmUmGsmAsmG-3'; |
| SN-16989 | ss: 5'-mCsmAsmUmUmUmUAfmAUfCfCfmUmCmAmCmUmCmUmAsmAsmA-3'-L12'; |
| | as: 5'-mUsUfsmUmAmGAfmGUfGfmAmGmGmAUfmUAfmAmAmAmUmGsmAsmG-3'; |
| SN-16990 | ss: 5'-mCsmAsmUmUmUmUAfmAUfCfCfmUmCmAmCmUmCmUmAsmAsmA-3'-L13'; |
| | as: 5'-mUsUfsmUmAmGAfmGUfGfmAmGmGmAUfmUAfmAmAmAmUmGsmAsmG-3'; |
| SN-17013 | ss: 5'-mCsmAsmUmUmUmUAfmAUfCfCfmUmCmAmCmUmCmUmAsmAsmA-3'-L14'; |
| | as: 5'-mUsUfsmUmAmGAfmGUfGfmAmGmGmAUfmUAfmAmAmAmUmGsmAsmG-3'; |
| SN-17014 | ss: 5'-mCsmAsmUmUmUmUAfmAUfCfCfmUmCmAmCmUmCmUmAsmAsmA-3'-L15'; |
| | as: 5'-mUsUfsmUmAmGAfmGUfGfmAmGmGmAUfmUAfmAmAmAmUmGsmAsmG-3'; |
| SN-17002 | ss: 5'-mCsmAsmUmUmUmUAfmAUfCfCfmUmCmAmCmUmCmUmAsmAsmA-3'-L16'; |
| | as: 5'-mUsUfsmUmAmGAfmGUfGfmAmGmGmAUfmUAfmAmAmAmUmGsmAsmG-3'; |
| SN-683081 | ss: 5'-mUsmGsmCmAmAmAmUfmAmGfmUfmCfmUmAmCmAmAmAmCmCsmAsmA-3'-L16'; |
| | as: 5'-mUsmUfsmGmGmUmUmUmGmUmAmGmAmCmUfmAmUfmUmUmGmCmAsmCsmA- 3'; |
| SN-17034 | ss: 5'-mCsmAsmUmUmUmUAfmAUfCfCfmUmCmAmCmUmCmUmAsmAsmA-3'-L34'; |
| | as: 5'-mUsUfsmUmAmGAfmGUfGfmAmGmGmAUfmUAfmAmAmAmUmGsmAsmG-3'; |
| SN-17009 | ss:5'-mCsmAsmUmUmUmUAfmAUfCfCfmUmCmAmCmUmCmUmAsmAsmA-3'-L35'; |
| | as: 5'-mUsUfsmUmAmGAfmGUfGfmAmGmGmAUfmUAfmAmAmAmUmGsmAsmG-3'; |

**Table 5**

| | |
|---|---|
| SN-17015 | ss: 5'-mCsmAsmUmUmUmUAfmAUfCfCfmUmCmAmCmUmCmUmAsmAsmA-L21'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17016 | ss: 5'-mCsmAsmUmUmUmUAfmAUfCfCfmUmCmAmCmUmCmUmAsmAsmA-L22'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17029 | ss: 5'-mCsmAsmUmUmUmUAfmAUfCfCfmUmCmAmCmUmCmUmAsmAsmA-L17'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17017 | ss: 5'-mCsmAsmUmUmUmUAfmAUfCfCfmUmCmAmCmUmCmUmAsmAsmA-L23'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17025 | ss: 5'-mCsmAsmUmUmUmUAfmAUfCfCfmUmCmAmCmUmCmUmAsmAsmA-L24'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17018 | ss: 5'-mCsmAsmUmUmUmUAfmAUfCfCfmUmCmAmCmUmCmUmAsmAsmA-L25'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17019 | ss: 5'-mCsmAsmUmUmUmUAfmAUfCfCfmUmCmAmCmUmCmUmAsmAsmA-L26'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17020 | ss: 5'-mCsmAsmUmUmUmUAfmAUfCfCfmUmCmAmCmUmCmUmAsmAsmA-L27'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17021 | ss: 5'-mCsmAsmUmUmUmUAfmAUfCfCfmUmCmAmCmUmCmUmAsmAsmA-L28'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17030 | ss: 5'-mCsmAsmUmUmUmUAfmAUfCfCfmUmCmAmCmUmCmUmAsmAsmA-L29'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17022 | ss: 5'-mCsmAsmUmUmUmUAfmAUfCfCfmUmCmAmCmUmCmUmAsmAsmA-L30'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17023 | ss: 5'-mCsmAsmUmUmUmUAfmAUfCfCfmUmCmAmCmUmCmUmAsmAsmA-L31'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17026 | ss: 5'-mCsmAsmUmUmUmUAfmAUfCfCfmUmCmAmCmUmCmUmAsmAsmA-L32'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17027 | ss: 5'-mCsmAsmUmUmUmUAfmAUfCfCfmUmCmAmCmUmCmUmAsmAsmA-L33'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17031 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-16995 | ss: 5'-TsAGsGAstsastsastststsCstsasCAsGCsT-L17'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-16996 | ss: 5'-mCsmAsmUmUmUmUAfmAUfCfCfmUmCmAmCmUmCmUmAsmAsmA-L17'- TsAGsGAstsastsastststsCstsasCAsGCsT 3'; |
| | as: 5'-mUsUfsmUmAmGAfmGUfGfmAmGmGmAUfmUAfmAmAmAmUmGsmAsmG-3'; |
| SN-16997 | ss: 5'-CsCsGsTsCsgsCsCsCststsCsasgsCsAsCsGsCsA-L17'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-16998 | ss: 5'-mCsmAsmUmUmUmUAfmAUfCfCfmUmCmAmCmUmCmUmAsmAsmA-L17'- CsCsGsTsCsgsCsCsCststsCsasgsCsAsCsGsCsA-3'; |
| | as: 5'-mUsUfsmUmAmGAfmGUfGfmAmGmGmAUfmUAfmAmAmAmUmGsmAsmG-3'; |
| SN-17003 | ss: 5'-mCsmAsmUmUmUmUAfmAUfCfCfmUmCmAmCmUmCmUmAsmAsmA-L17'- CsGsTsCsgsCsCsCststsCsasgsCsAsCsGsC-3'; |
| | as: 5'-mUsUfsmUmAmGAfmGUfGfmAmGmGmAUfmUAfmAmAmAmUmGsmAsmG-3'; |
| SN-17004 | ss: 5'-mCsmAsmUmUmUmUAfmAUfCfCfmUmCmAmCmUmCmUmAsmAsmA-L17'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17005 | ss: 5'-mCsmAsmUmUmUmUAfmAUfCfCfmUmCmAmCmUmCmUmAsmAsmA-L17'- TsCsgsCsCsCststsCsasgsCsAsC-3'; |
| | as: 5'-mUsUfsmUmAmGAfmGUfGfmAmGmGmAUfmUAfmAmAmAmUmGsmAsmG-3'; |
| SN-17001 | ss: 5'-CsCsGsTsCsgsCsCsCststsCsasgsCsAsCsGsCsA-L18'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-17024 | ss: 5'-mCsmAsmUmUmUmUAfmAUfCfCfmUmCmAmCmUmCmUmAsmAsmA-L18'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17032 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17006 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17010 | ss: 5'-mCsmAsmUmUmUmUAfmAUfCfCfmUmCmAmCmUmCmUmAsmAsmA-L20'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |

preferably, E is O.

Embodiment 34. Use of the nucleic acid conjugate according to any one of Embodiments 19 to 33 in preparation of a medicament for treatment of a gene-related disease.

Embodiment 35. The use according to Embodiment 34, where the gene is selected from APP, SOD1, HTT, MeCP2, DUX4, HDAC2, GPR75, Ataxin 1, Ataxin 2, Ataxin 3, Ataxin 6, Ataxin 7, C9ORF72, UBE3A, Prion, PMP22, Tau, LRRK2, LINGO2, GYS1, KCNT1, IRF5, Progranulin, GFAP, TARDBP, SNCA, FUS, SCA1, SCA7, SCA8, MeCP2, PRNP, SOD1, DMPK, MAPT, or TTR.

Embodiment 36. The use according to Embodiment 34 or 35, where the disease is a central nervous system disease; preferably, the central nervous system disease is Alzheimer's disease, preferably, presenile dementia, amyotrophic lateral sclerosis (ALS), Huntington's disease, Parkinson's disease, Dravet syndrome, Charcot triad, Alexander disease, spinocerebellar ataxia, or Angelman syndrome; more preferably, the disease is selected from presenile dementia, amyotrophic lateral sclerosis (ALS), or spinocerebellar ataxia.

Embodiment 37. The use according to any one of Embodiments 34 to 36, where the medicament is an injection or an oral preparation, preferably, an injection administered intracranially, intrathecally, subcutaneously, intravenously, or intramuscularly.

Embodiment 38. A method for treating a gene-related disease, comprising administering to a subject a therapeutically effective amount of the conjugate according to any one of Embodiments 19 to 33.

Embodiment 39. The method according to Embodimen 38, where the gene is selected from APP, SOD1, HTT, MeCP2, DUX4, HDAC2, GPR75, Ataxin 1, Ataxin 2, Ataxin 3, Ataxin 6, Ataxin 7, C9ORF72, UBE3A, Prion, PMP22, Tau, LRRK2, LINGO2, GYS1, KCNT1, IRF5, Progranulin, GFAP, TARDBP, SNCA, FUS, SCA1, SCA7, SCA8, MeCP2, PRNP, SOD1, DMPK, or TTR.

Embodiment 40. The method according to Embodiment 38 or 39, where the disease is a central nervous system disease; preferably, the central nervous system disease is Alzheimer's disease, preferably, presenile dementia, amyotrophic lateral sclerosis (ALS), Huntington's disease, Parkinson's disease, Dravet syndrome, Charcot triad, Alexander disease, spinocerebellar ataxia, or Angelman syndrome; more preferably, the disease is selected from presenile dementia, amyotrophic lateral sclerosis (ALS), or spinocerebellar ataxia.

Embodiment 41. The method according to any one of Embodiments 38 to 40, where the conjugate is administered in a form of an injection or an oral preparation, preferably, an injection is administered intracranially, intrathecally, subcutaneously, intravenously or intramuscularly.

Embodiment 42. Use of the lipid compound according to any one of Embodiments 1 to 18 in preparation of the nucleic acid conjugate according to any one of Embodiments 19 to 33.

In the following embodiments of the present disclosure, a lipid compound is designed, which has a structure represented by formula (A) or (B) below: 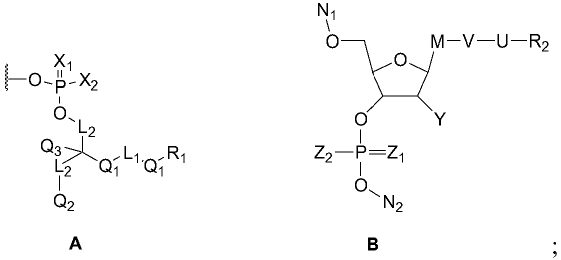 where in the compound represented by formula (A):
Q₁ is selected from -NH₂ (amino group), -COOH (carboxyl group), -NHCO (amide group), -O-, -S-, -S-S-, a phosphate group, or a phosphorothioate group;
Q₂ is selected from -OH (hydroxyl group), -NH₂ (amino group), -H or -CH₃ (methyl group);
Q₃ is selected from -H or a C₁-C₁₀ alkyl group;
L₁ is selected from a C₁-C₁₀ saturated alkyl chain;
L₂ is selected from a C₁-C₁₀ saturated alkyl chain;
X₁ is selected from an O or S atom;
X₂ is selected from -O-, -S-, -SH, -OH (hydroxyl group), -NH₂ (amino group), -CH₃ (methyl group), -CH₂CH₃ (ethyl group), -OCH₃ (methoxy group), or -OCH₂CH₃ (ethoxy group);
R₁ is selected from a C₁₀-C₃₀ saturated fatty acid chain or saturated alkyl chain; and
where in the compound represented by formula (B):
   M is selected from H, O, C, or a nucleobase independently selected from modified or unmodified bases, such as adenine, uracil, thymine, guanine or cytosine;
   N₁ is selected from H or a C₁-C₃ alkyl group;
   Y is selected from H, NH₂, OH, halogen, a C₁-C₆ alkyl group, a C₁-C₆ haloalkyl group, a C₁-C₆ alkoxy group, MOE (2'-O-methoxyethyl);
   V is selected from a C₁-C₄ saturated alkyl chain;
   U is selected from -NH₂ (amino group), -COOH (carboxyl group), or -NHCO (amide group);
   Z₁ is selected from an O or S atom;
   Z₂ is selected from a C₁₀-C₃₀ alkoxy group or a C₁₀-C₃₀ amide saturated lipid chain; and
   R₂ is selected from a C₁₀-C₃₀ alkoxy group, a C₁₀-C₃₀ saturated fatty acid chain, or a C₁₀-C₃₀ amide saturated lipid chain.

In some embodiments, the saturated lipid chain moiety is a C₆-C₃₀ acid, for example, linear saturated hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, oleic acid, and linoleic acid.

In some embodiments, optionally, arachidonic acid, cis-4,7,10,13,16,19-docosahexaenoic acid, vitamin A, vitamin E, cholesterol, or the like; or C₆-C₃₀ alcohol (for example, hexanol, heptanol, octanol, nonanol, decanol, undecanol, dodecanol, tridecanol, tetradecanol, pentadecanol, hexadecanol, heptadecanol, octadecanol, oleyl alcohol, linolenyl alcohol, arachidonyl alcohol, cis-4,7,10,13,16,19-docosahexaenol, retinol, vitamin E, and cholesterol).

In some embodiments, a lipophilic moiety may comprise a saturated or unsaturated lipid chain (C₄-C₃₀ hydrocarbon chain, for example, a C₄-C₃₀ alkyl or alkenyl group) and an optional functional group selected from the group consisting of: hydroxyl, amine, carboxylic acid, sulfonate, phosphate, thiol, azide, and alkyne. These functional groups may be used to attach the lipophilic moiety to double-stranded or single-stranded oligonucleotides.

In some embodiments, the lipophilic moiety comprises a saturated or unsaturated C₆-C₁₈ hydrocarbon chain, for example, a linear C₆-C₁₈ alkyl or alkenyl group.

In an embodiment, the lipophilic moiety comprises a saturated or unsaturated C₁₆ hydrocarbon chain, for example, a linear C₁₆ alkyl or alkenyl group.

In some embodiments, the lipid chain moiety is lipid, cholesterol, retinoic acid, cholic acid, adamantaneacetic acid, 1-pyrenebutyric acid, dihydrotestosterone, 1,3-bis-O(hexadecyl)glycerol, geranyloxyhexanol, hexadecylglycerol, borneol, menthol, 1,3-propanediol, heptadecyl, palmitic acid, or myristic acid.

In some embodiments, the lipid chain moiety may form a conjugate with the double-stranded ribose via direct linkage. The lipid chain moiety may form a conjugate with the double-stranded ribose via a linker or carrier.

In some embodiments, the lipid chain moiety is conjugated to double-stranded RNA via a linker which comprises ether, thioether, urea, carbonate, amine, amide, maleimide-thioether, disulfide, phosphodiester, sulfonamide bond, or a triazole formed by post-ligation azide-alkyne cycloaddition.

In some embodiments, at least one lipid chain moiety is conjugated to the starting position of the 3'-end of the sense strand of the double-helical RNA.

In some embodiments, at least one lipid chain moiety is conjugated to the starting position of the 3'-end of the antisense strand of the double-helical RNA.

In some embodiments, at least one lipid chain moiety is conjugated to the starting position of the 5'-end of the sense strand of the double-helical RNA.

In some embodiments, at least one lipid chain moiety is conjugated to the starting position of the 5'-end of the antisense strand of the double-helical RNA.

In some embodiments, at least one lipid chain moiety is conjugated at any position within the sense strand sequence of the double-helical RNA.

In some embodiments, at least one lipid chain moiety is conjugated at any position within the antisense strand sequence of the double-helical RNA.

In some embodiments, at least one lipid chain moiety is conjugated to the 5'-end and 3'-end of the sense strand sequences of two double-helical RNAs with different sequences.

In some embodiments, at least one lipid chain moiety is conjugated to the 5'-end and 3'-end of the antisense strand sequences of two double-helical RNAs with different sequences.

In some embodiments, at least one lipid chain moiety is conjugated to the 3'-end and 3'-end of the sense strand sequences of two double-helical RNAs with different sequences.

In some embodiments, at least one lipid chain moiety is conjugated to the 5'-end and 5'-end of the sense strand sequences of two double-helical RNAs with different sequences.

In some embodiments, at least one lipid chain moiety is conjugated to the 3'-end and 3'-end of the antisense strand sequences of two double-helical RNAs with different sequences.

In some embodiments, at least one lipid chain moiety is conjugated to the 5'-end and 5'-end of the antisense strand sequences of two double-helical RNAs with different sequences.

In some embodiments, a phosphate or phosphate analog is incorporated at the 5'-end of the antisense strand of the double-stranded RNA.

In some embodiments, the phosphate analog is 5'-vinylphosphonate (VP).

In some embodiments, the phosphate analog is 5'-phosphorothioate.

In some embodiments, the phosphate analog is 5'-O-methylphosphorothioate.

In some embodiments, the double-stranded RNA contains at least one chiral phosphorus atom at an end of the sense or antisense strand. In an embodiment of the present disclosure, the chiral modification may occur on the sense strand or the antisense strand in the double-helical siRNA. Each chirally pure phosphorus atom may have an Rp configuration, an Sp configuration, or a combination thereof (Iyer, R. P.; Gou, M; Yu, D. and Agrawal, S. "Stereoselective Synthesis of Oligonucleoside Phosphorothioates: The Nucleoside Bicyclic Oxazaphospholidines as a Novel Synthons" Tetrahedron Letters, 1998, 39, 2491; Yu, D.; Kandimalla, E. R.; Roskey, A.; Zhao, Q.; Chen, L.; Chen, J. and Agrawal, S. "Stereo-Enriched Phosphorothioate Oligonucleotides: Synthesis, Biophysical and Biological Properties" Bioorganic & Medicinal Chemistry, 2000, 8, 275).

In some embodiments, siRNA further comprises at least one ASGPR ligand. The ASGPR (asialoglycoprotein receptor) is a lectin highly expressed on hepatocytes, and can efficiently clear glycoproteins from the blood circulation. Because the ASGPR can effectively bind asialoglycoproteins, the ASGPR ligand is used for liver-specific delivery. For example, the ASGPR ligand is a derivative of one or more galactosamines linked via a multifunctional linker (CN 114763367A), such as:

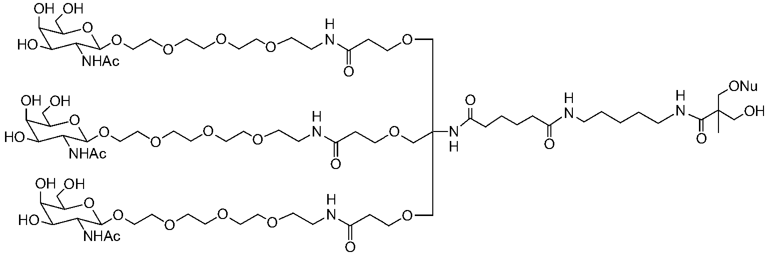

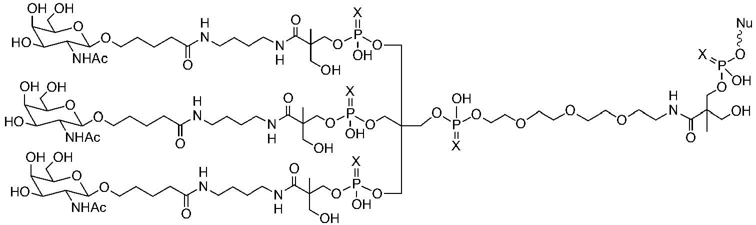

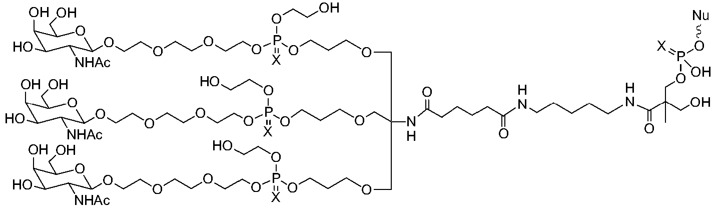

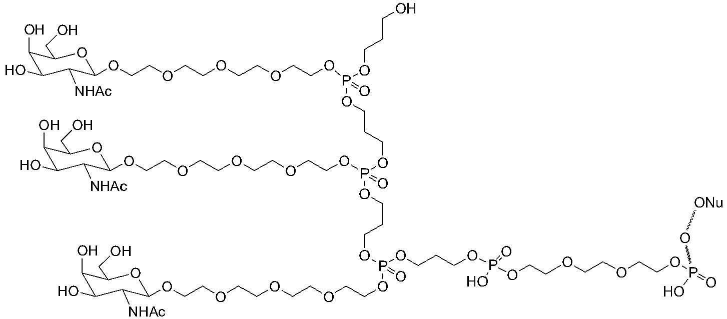

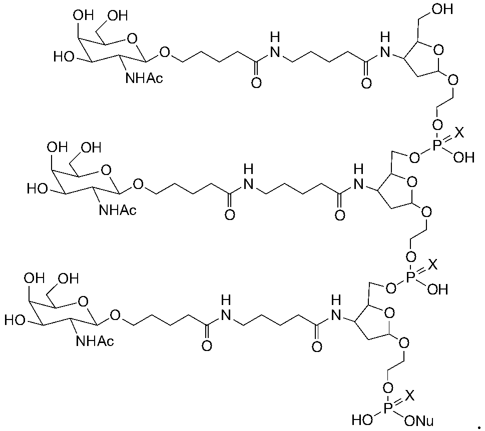

In the following examples of the present disclosure, the lipid compounds may be synthesized in methods known and feasible in the art. For example, the following compounds can synthesized via a variety of approaches and routes known in the art.

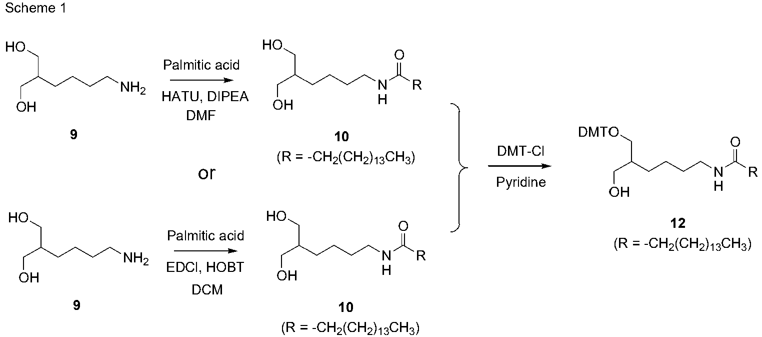

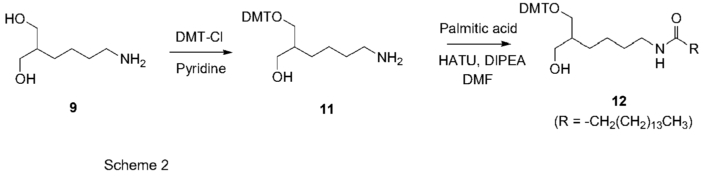

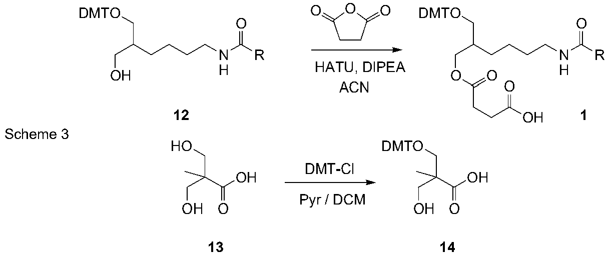

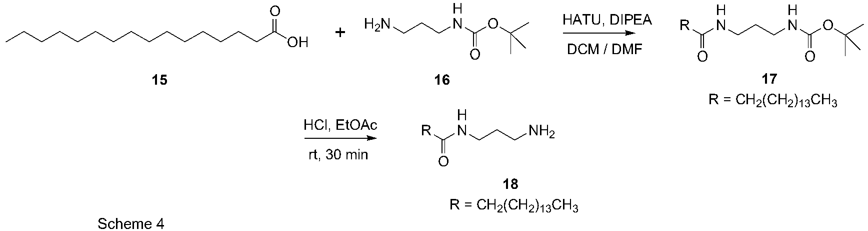

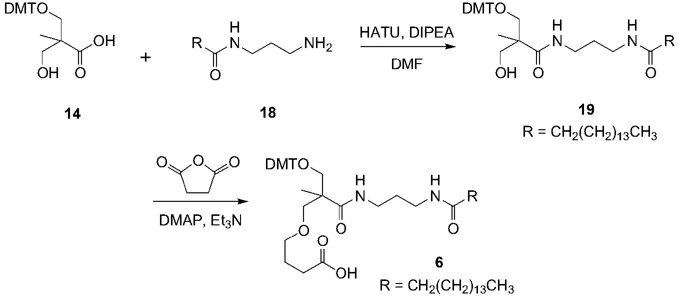

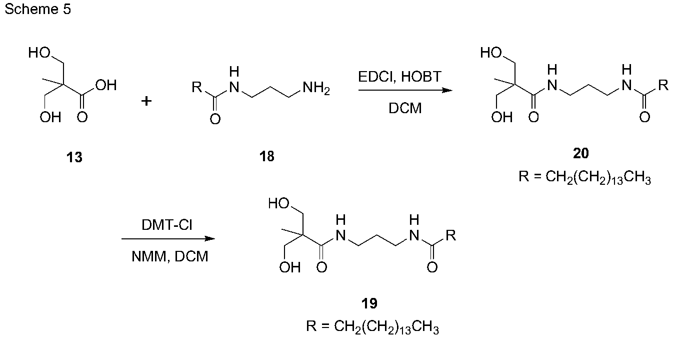

In the foregoing synthetic route, the relevant English abbreviations/terms have the following meanings:
HATU is the abbreviation for 2-(7-azabenzotriazol-1-yl)-N,N,N',N'-tetramethyluronium hexafluorophosphate;
DIPEA is the abbreviation for N,N-diisopropylethylamine;
DMF is the abbreviation for dimethylformamide;
EDCl is the abbreviation for 1-ethyl-3-(3-dimethylaminopropyl)carbodiimide;
HOBT is the abbreviation for 1-hydroxybenzotriazole;
DCM is the abbreviation for dichloromethane;
DMT-Cl is the abbreviation for 4,4'-dimethoxytrityl chloride;
ACN is the abbreviation for acetonitrile;
Pyr is the abbreviation for pyridine;
HCl is the abbreviation for hydrochloric acid;
EtOAc is the abbreviation for ethyl acetate;
DMAP is the abbreviation for 4-dimethylaminopyridine;
Et₃N is the abbreviation for triethylamine; and
NMM is the abbreviation for 4-methylmorpholine.

The synthetic routes of the foregoing compounds are intended to illustrate that the lipid compounds with different structures in the present disclosure may be synthesized in various routes in the art. A person skilled in the art can select appropriate synthetic routes and conditions based on their own starting materials and conditions.

### Example 1

In this example, a multifunctional linker with the following structure is designed and synthesized:

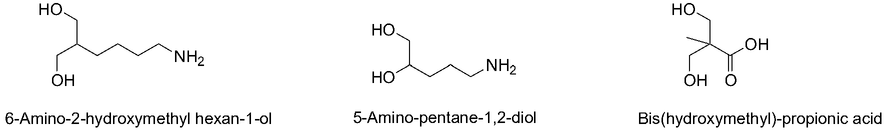

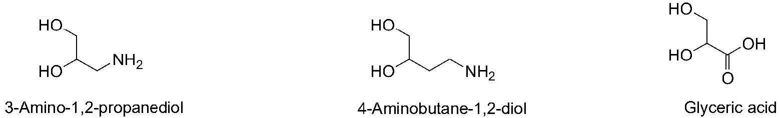

In this example, the exemplified multifunctional linkers exhibit the characteristics of polyhydroxyamino and polyhydroxycarboxyl groups. These functional groups may be used to independently conjugate different functional compounds, to achieve the overall performance of the designed molecule. For example, hydroxyl groups are intended for linking small nucleic acid fragments, and can independently conjugate two or more distinct sequence fragments. Amino or carboxyl groups are conjugated to functional groups that are relatively stable under various conditions, for example, lipid alkyl chains (saturated or unsaturated), polyethylene glycol, or cholesterol-derived functional molecules.

### Example 2

Based on the structures of the foregoing lipid compounds, in this example, phosphoramidite structures of lipid compound monomers with the following structures are designed, as shown in Table 6.

**Table 6: Lipid Phosphoramidite Structures**

| | |
|---|---|
| A1 | 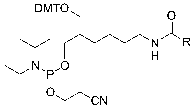 R = C₁₀-C₃₀ alkyl substituted group |
| A2 | 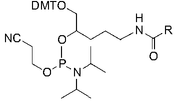 R = C₁₀-C₃₀ alkyl substituted group |
| A3 | 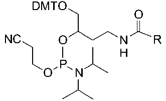 R = C₁₀-C₃₀ alkyl substituted group |
| A4 | 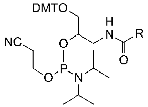 R = C₁₀-C₃₀ alkyl substituted group |
| A5 | 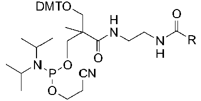 R = C₁₀-C₃₀ alkyl substituted group |
| A6 | 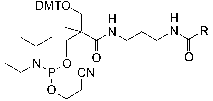 R = C₁₀-C₃₀ alkyl substituted group |
| A7 | 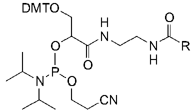 R = C₁₀-C₃₀ alkyl substituted group |
| A8 | 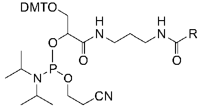 R = C₁₀-C₃₀ alkyl substituted group |
| A9 | 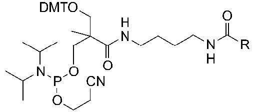 R = C₁₀-C₃₀ alkyl substituted group |
| A10 | 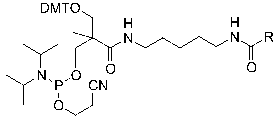 R = C₁₀-C₃₀ alkyl substituted group |
| A11 | 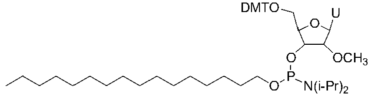 |
| A12 | 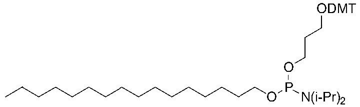 |
| A13 | 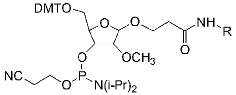 R = C₁₀-C₃₀ alkyl substituted group |
| A14 | 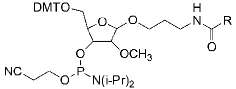 R = C₁₀-C₃₀ alkyl substituted group |
| A15 | 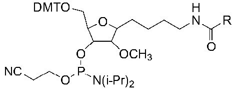 R = C₁₀-C₃₀ alkyl substituted group |
| A16 | 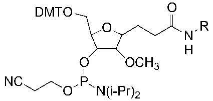 R = C₁₀-C₃₀ alkyl substituted group |
| A17 | 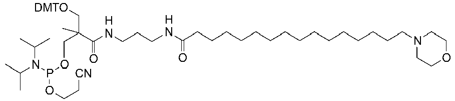 |
| A18 | 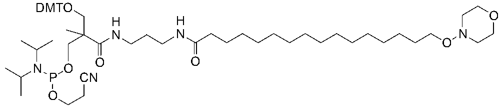 |
| A19 | 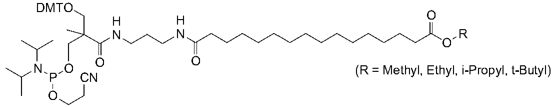 |
| A20 | 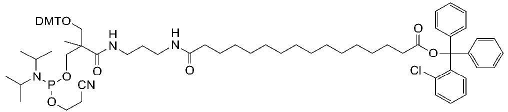 |
| A21 | 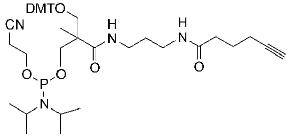 |
| A22 | 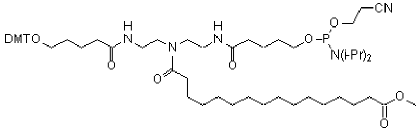 |

In the foregoing table, English abbreviations/terms have the following meanings: alkyl substituted group refers to an alkyl substituent;
Methyl refers to methyl group;
Ethyl refers to ethyl group;
i-Propyl refers to isopropyl group;
t-Butyl refers to tert-butyl group; and
i-Pr is the abbreviation for isopropyl group.

In this example, the phosphoramidites with such structures are critical chemical reagents for subsequent solid-phase synthesis. By virtue of a trivalent phosphorus chemical structure, phosphoramidites exhibit extremely high chemical reactivity, which is critical for improving the reaction yield. The solid-phase synthesis is employed in the subsequent preparation, which greatly improves the purification and separation effects.

### Example 3

Based on the structures of the foregoing lipid compounds, in this example, saturated lipid linker conjugates with the following structure are designed, as shown in Table 7.

**Table 7 Structures of Saturated Lipid Linker Conjugates**

| | |
|---|---|
| B1 | 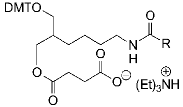 R = C₁₀-C₃₀ alkyl substituted group |
| B2 | 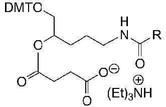 R = C₁₀-C₃₀ alkyl substituted group |
| B3 | 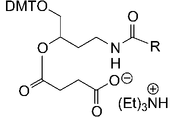 R = C₁₀-C₃₀ alkyl substituted group |
| B4 | 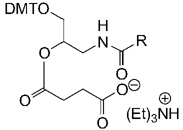 R = C₁₀-C₃₀ alkyl substituted group |
| B5 | 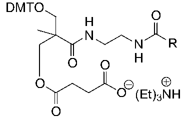 R = C₁₀-C₃₀ alkyl substituted group |
| B6 | 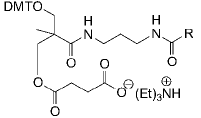 R = C₁₀-C₃₀ alkyl substituted group |
| B7 | 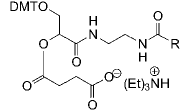 R = C₁₀-C₃₀ alkyl substituted group |
| B8 | 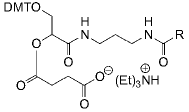 R = C₁₀-C₃₀ alkyl substituted group |
| B9 | 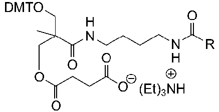 R = C₁₀-C₃₀ alkyl substituted group |
| B10 | 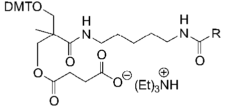 R = C₁₀-C₃₀ alkyl substituted group |
| B11 | 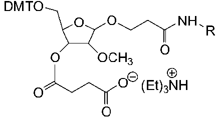 R = C₁₀-C₃₀ alkyl substituted group |
| B12 | 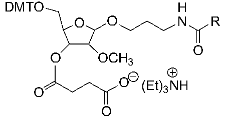 R = C₁₀-C₃₀ alkyl substituted group |
| B13 | 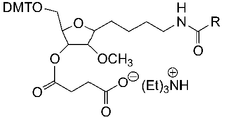 R = C₁₀-C₃₀ alkyl substituted group |
| B14 | 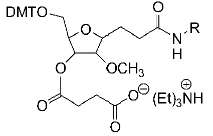 R = C₁₀-C₃₀ alkyl substituted group |
| B21 | 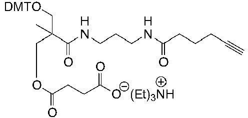 |

In the foregoing table, English abbreviations/terms have the following meanings: Et is the abbreviation for ethyl group.

In this example, utilization of solid-phase synthesis in subsequent procedures allows lipid chains to be conjugated to either the phosphoramidite moiety or directly to the solid-phase support. In consideration that the lipid chain is eventually conjugated to the nucleic acid sequence, an intermediate compound is provided to link the solid-phase support and the lipid chain. Succinic acid is the optimal choice, which not only ensures the conjugation between the two moieties but also enables cleavage from the solid support in subsequent treatment and separation from the nucleic acid-lipid chain. Therefore, the saturated lipid linker conjugate serves as an important intermediate in the complementary method for solid-phase synthesis of nucleic acid-lipid chains.

### Example 4

Based on the structures of the foregoing lipid compounds, in this example, saturated lipid linker conjugates having the following structures and capable of being conjugated to the solid-phase support are designed, as shown in Table 8.

**Table 8 Solid-Phase Support Structures of Saturated Lipid Linker Conjugates**

| | |
|---|---|
| C1 | 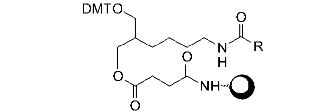 R = C₁₀-C₃₀ alkyl substituted group |
| C2 | 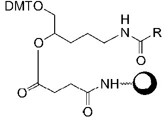 R = C₁₀-C₃₀ alkyl substituted group |
| C3 | 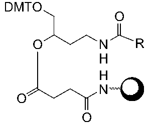 R = C₁₀-C₃₀ alkyl substituted group |
| C4 | 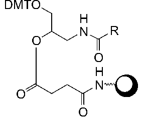 R = C₁₀-C₃₀ alkyl substituted group |
| C5 | 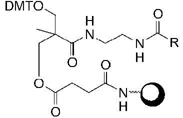 R = C₁₀-C₃₀ alkyl substituted group |
| C6 | 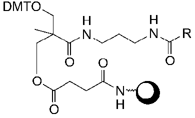 R = C₁₀-C₃₀ alkyl substituted group |
| C7 | 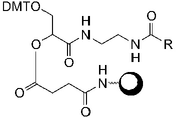 R = C₁₀-C₃₀ alkyl substituted group |
| C8 | 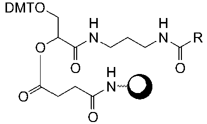 R = C₁₀-C₃₀ alkyl substituted group |
| C9 | 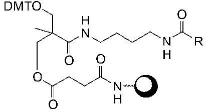 R = C₁₀-C₃₀ alkyl substituted group |
| C10 | 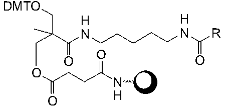 R = C₁₀-C₃₀ alkyl substituted group |
| C11 | 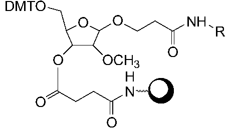 R = C₁₀-C₃₀ alkyl substituted group |
| C12 | 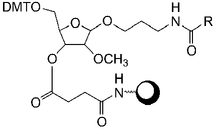 R = C₁₀-C₃₀ alkyl substituted group |
| C13 | 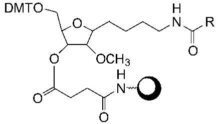 R = C₁₀-C₃₀ alkyl substituted group |
| C14 | 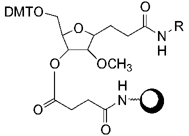 R = C₁₀-C₃₀ alkyl substituted group |
| C21 | 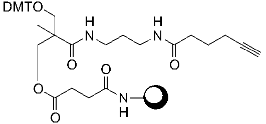 |

Consistent with the solution described in the foregoing Example 3, this example further describes a lipid compound monomer. The lipid compound monomer has a carboxyl functional group at an end, and the solid phase is linked to the lipid chain of the lipid compound monomer through a condensation reaction by using the amino or hydroxyl group on the solid-phase support. In this way, the lipid compound monomer may be directly applied to the subsequent synthesis of the nucleic acid sequence.

### Example 5

Based on the structures of the foregoing lipid compounds, in this example, lipid compound monomers having the structures shown in the foregoing Tables 1 and 2, as well as nucleic acid conjugates formed from the foregoing lipid compound monomers, are designed (for specific structures, refer to the foregoing Tables 3 to 5).

Consistent with the solution described in the foregoing Examples 2 to 4, in this example, the solid-phase synthesis method is adopted for the conjugate between the lipid compound monomer and the nucleotide.

### Example 6

In this example, the foregoing lipid compound monomer was synthesized.

### (1) Synthesis of Lipid Compound Monomer (B1)

In this example, the synthesis procedure and specific method of the lipid compound monomer (B1) in the present disclosure are described as follows.

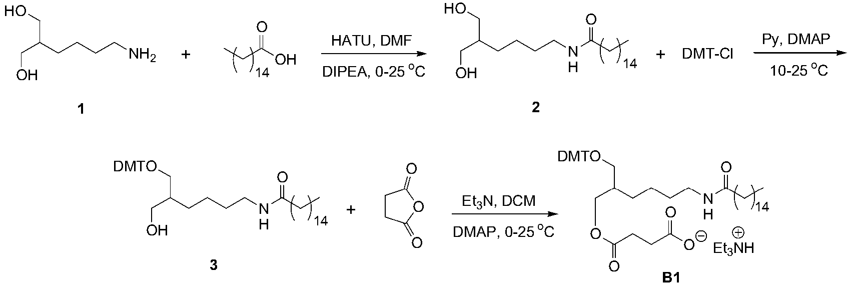

Palmitic acid (0.513g, 2 mmol) was dissolved in 10 mL of dried dimethylformamide (DMF) at room temperature. After complete dissolution, 2-(7-azabenzotriazol-1-yl)-N,N,N',N'-tetramethyluronium hexafluorophosphate (HATU, 0.78g, 2.05 mmol) and N,N-diisopropylethylamine (DIPEA, 1 mL, 6 mmol) were sequentially added under an ice bath, and the reaction solution was stirred thoroughly at 0°C for 10 minutes. After formation of the intermediate product, 6-amino-2-(hydroxymethyl)hexan-1-ol 1 (0.309g, 2.1 mmol) was dissolved in 5 mL of dimethylformamide and the resulting mixture was then added dropwise to a reaction flask. The reaction solution was warmed to room temperature (25°C) and further stirred at room temperature for 12 hours. The reaction solution was added into 30 mL of saturated brine, and precipitation of a solid was observed. After complete precipitation of the solid, the solid was filtered out and washed with water to remove the unreacted reagent and solvent. Finally, product 2 (0.747g, 97%) was dried under vacuum at room temperature and used directly in the next step.

4,4'-Dimethoxytrityl chloride (DMT-Cl, 0.6g, 1.7 mmol) was dissolved in 5 mL of dichloromethane (DCM). The solution was slowly added dropwise to a solution of compound 2 (0.718g, 1.86 mmol) in anhydrous pyridine (Py, 10 mL) at room temperature, and a small amount of 4-dimethylaminopyridine (20 mg) was added to the reaction solution. The reaction solution was stirred continuously at room temperature for 14 hours. 20 mL of saturated brine was added to the reaction mixture, and the resulting mixture was then extracted with ethyl acetate (EtOAc, 2×50 mL). The organic phase was concentrated to semi-dryness by rotary evaporation and further purified by silica gel column chromatography using one gradient elution: the organic phase was first eluted with n-hexane, then with n-hexane and ethyl acetate (n-hexane/ethyl acetate=3:1, v/v, containing 1% triethylamine), and finally with n-hexane and ethyl acetate (n-hexane/ethyl acetate=1:1, v/v, containing 1% triethylamine). The product components were collected and evaporated to dryness under reduced pressure to obtain product 3 as a yellow solid (0.58g, 50%), and the product 3 was used directly in the next step.

Compound 3 (0.58g, 0.844 mmol) was dissolved in 15 mL of dry dichloromethane, and 0.6 mL of triethylamine was then added. 4-Dimethylaminopyridine (0.195g, 1.6 mmol) was dissolved in the reaction solution during stirring, succinic anhydride (0.127g, 1.27 mmol) was then dissolved in the reaction solution at room temperature with during, and the resulting mixture was stirred for reaction for 8 hours. Succinic anhydride (32 mg, 0.32 mmol) was further added, and the resulting mixture was further stirred at room temperature for 14 hours. The reaction solution was added into saturated brine, the mixture was extracted with dichloromethane (2 × 50 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:1:0.01, v/v/v) to obtain the final product B1. The solvent was evaporated under reduced pressure to obtain compound B1 as a white solid (0.62g, 93%). The structure information for the tested product is as follows:¹H NMR (CDCl₃): d, 7.41-7.40 (m, 3H, trityl), 7.39-7.27 (m, 6H, trityl), 7.25-7.19 (m, 1H), 6.83-6.80 (m, 4H, trityl), 4.24-4.12 (m, 2H), 3.78 (s, 6H), 3.19-3.16 (m, 2H), 3.10-3.07 (m, 2H), 3.03-3.01 (m, 1H), 3.00-2.99 (m, 2H), 2.56-2.54 (m, 4H), 2.15-2.12 (m, 2H), 1.60-1.57 (m, 2H), 1.43-1.39 (m, 2H), 1.34-1.20 (m, 26H), 1.19-1.17 (m, 2H), 0.89-86 (m, 3H) ppm. It can be seen that the structure of the product was confirmed to be correct.

### (2) Synthesis of Lipid Compound Monomer (B3)

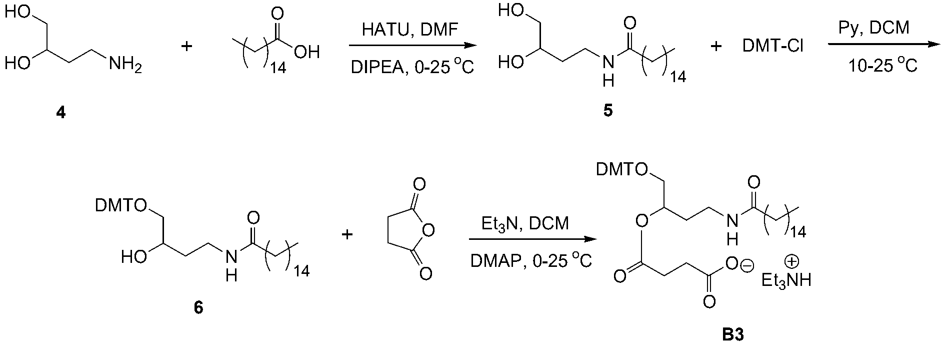

Palmitic acid (0.513g, 2 mmol) was dissolved in 10 mL of dried dimethylformamide at room temperature. After complete dissolution, 2-(7-azabenzotriazol-1-yl)-N,N,N',N'-tetramethyluronium hexafluorophosphate (0.78g, 2.05 mmol) and N,N-diisopropylethylamine (1 mL, 6 mmol) were sequentially added under an ice bath, and the reaction solution was stirred thoroughly at 0°C for 10 minutes. After formation of the intermediate product, 4-amino-2-(hydroxymethyl)butan-1-ol 4 (0.25g, 2.1 mmol) was dissolved in 5 mL of dimethylformamide and the resulting mixture was then added dropwise to a reaction flask. The reaction solution was warmed to room temperature (25°C) and further stirred at room temperature for 12 hours. The reaction solution was added into 30 mL of saturated brine, and precipitation of a solid was observed. After complete precipitation of the solid, the solid was filtered out and washed with water to remove the unreacted reagent and solvent. Finally, product 5 (0.678g, 98%) was dried under vacuum at room temperature and used directly in the next step.

4,4'-Dimethoxytrityl chloride (0.63g, 1.9 mmol) was dissolved in 4 mL of dichloromethane. The solution was slowly added dropwise to a solution of compound 5 (0.67g, 1.86 mmol) in anhydrous pyridine (20 mL) at room temperature, and a small amount of 4-dimethylaminopyridine (20 mg) was added to the reaction solution. The reaction solution was stirred continuously at room temperature for 14 hours. 30 mL of saturated brine was added to the reaction mixture, and the resulting mixture was then extracted with ethyl acetate (2×50 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), and finally with n-hexane and ethyl acetate (n-hexane/ethyl acetate=1:1, v/v, containing 1% triethylamine). The product components were collected and evaporated to dryness under reduced pressure to obtain product 6 as a yellow solid (0.76g, 63%), and product 6 was used directly in the next step.

Compound 6 (0.76g, 1.18 mmol) was dissolved in 15 mL of dry dichloromethane, and 0.8 mL of triethylamine was then added. 4-Dimethylaminopyridine (0.725g, 5.9 mmol) was dissolved in the reaction solution during stirring, succinic anhydride (0.172g, 1.72 mmol) was then dissolved in the reaction solution at room temperature with during, and the resulting mixture was stirred for reaction for 8 hours. Succinic anhydride (32 mg, 0.32 mmol) was further added, and the resulting mixture was further stirred at room temperature for 14 hours. The reaction solution was added into saturated brine, the mixture was extracted with dichloromethane (2 × 50 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:1:0.01, v/v/v) to obtain the final product B3. The solvent was evaporated under reduced pressure to obtain compound B3 as a white solid (0.72g, 82%). The tested data for the product structure is as follows: ¹H NMR (CDCl₃): d, 7.41-7.40 (m, 2H, trityl), 7.39-7.27 (m, 6H, trityl), 7.20-7.17 (m, 1H, trityl), 6.82-6.80 (m, 4H, trityl), 5.12-5.11 (m, 1H), 3.77 (s, 6H), 3.17-3.14 (m, 2H), 3.10-3.07 (m, 2H), 3.03-2.99 (m, 2H), 2.69-2.66 (m, 2H), 2.64-2.57 (m, 2H), 2.13-2.10 (m, 2H), 1.78-1.73 (m, 2H), 1.59-1.56 (m, 2H), 1.31-1.28 (m, 2H), 1.28-1.23 (m, 22H), 0.89-0.86 (m, 3H) ppm. It can be seen that the structure of the product was confirmed to be correct.

### (3) Synthesis of Lipid Compound Monomers (A4 and B4)

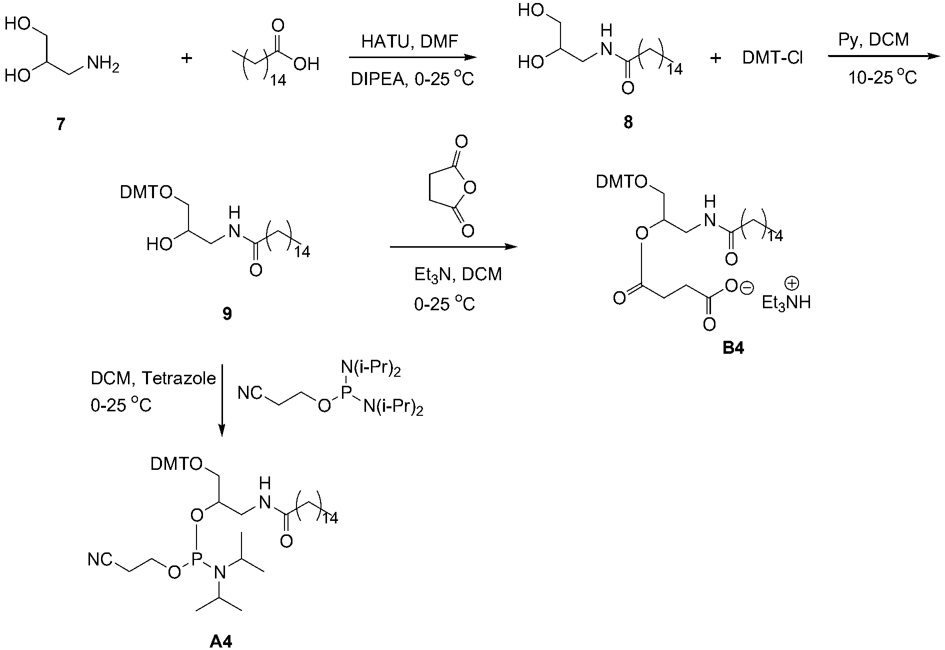

Palmitic acid (5.13g, 20 mmol) was dissolved in 100 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (8.4g, 22 mmol) and N,N-diisopropylethylamine (8.7 mL, 50 mmol) were sequentially added under an ice bath, and the reaction solution was stirred thoroughly at 0°C for 20 minutes. After formation of the intermediate product, 3-amino-2-(hydroxymethyl)propan-1-ol 7 (2g, 21 mmol) was dissolved in 50 mL of dimethylformamide and the resulting mixture was then added dropwise to a reaction flask. The reaction solution was warmed to room temperature (25°C) and further stirred at room temperature for 12 hours. The reaction solution was added into 500 mL of saturated brine, and precipitation of a solid was observed. After complete precipitation of the solid, the solid was filtered out and washed with water to remove the unreacted reagent and solvent. Finally, product 8 (6.25g, 95%) was dried under vacuum at room temperature and used directly in the next step.

4,4'-Dimethoxytrityl chloride (6.2g, 18.8 mmol) was dissolved in 20 mL of dichloromethane. The solution was slowly added dropwise to a solution of compound 8 (6.1g, 18.6 mmol) in anhydrous pyridine (100 mL) at room temperature. The reaction solution was stirred continuously at room temperature for 14 hours. 300 mL of saturated brine was added to the reaction mixture, and the resulting mixture was then extracted with ethyl acetate (2 × 250 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), and finally with n-hexane and ethyl acetate (n-hexane/ethyl acetate=1:1, v/v, containing 1% triethylamine). The product components were collected and evaporated to dryness under reduced pressure to obtain product 9 as a yellow solid (6.93g, 59%), and product 9 was used directly in the next step.

Compound 9 (4.75g, 7.52 mmol) was dissolved in 50 mL of dry dichloromethane, and 3.3 mL of triethylamine was then added. 4-Dimethylaminopyridine (0.72g, 5.9 mmol) was dissolved in the reaction solution during stirring, succinic anhydride (1.13g, 11.3 mmol) was then dissolved in the reaction solution at room temperature with during, and the resulting mixture was stirred for reaction for 8 hours. Succinic anhydride (100 mg, 1 mmol) was further added, and the resulting mixture was further stirred at room temperature for 14 hours. The reaction solution was added into saturated brine, the mixture was extracted with dichloromethane (2 × 250 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:1:0.01, v/v/v) to obtain the final product B4. The solvent was evaporated under reduced pressure to obtain compound B4 as a pale yellow solid (4.5g, 82%). The confirmed information for the product structure is as follows: ¹H NMR (CDCl₃): d, 7.42-7.40 (m, 2H, trityl), 7.31-7.27 (m, 4H, trityl), 7.25-7.18 (m, 2H, trityl), 6.83-6.80 (m, 4H, trityl), 5.14-5.11 (m, 1H), 3.79 (s, 6H), 3.78-3.70 (m, 1H), 3.31-3.30 (m, 1H), 3.20-3.19 (m, 2H), 3.02-2.98 (m, 3H), 2.64-2.56 (m, 4H), 2.09-2.05 (m, 2H), 1.54-1.51 (m, 2H), 1.31-1.23 (m, 22H), 0.89-0.86 (m, 3H) ppm. It can be seen that the structure of the product was confirmed to be correct.

Compound 9 (4.8g, 7.6 mmol) was dissolved in 100 mL of dry dichloromethane, and N,N,N',N'-tetraisopropyl-2-cyanoethoxy phosphite (3.4 mL, 11.4 mmol) was then added rapidly to the solution. The reaction solution was stirred at room temperature for 20 minutes under nitrogen atmosphere. 20 mL of a solution of tetrazole (0.64g, 9.12 mmol) in dry dichloromethane was added to the above reaction solution, and the mixture was further stirred for reaction at room temperature under nitrogen atmosphere for 2 hours. The reaction solution was added into saturated brine, the mixture was extracted with dichloromethane (2 × 150 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=5:1, v/v, containing 1% triethylamine), and finally with n-hexane and ethyl acetate (n-hexane/ethyl acetate=3:1, v/v, containing 1% triethylamine). The product components were collected and evaporated to dryness under reduced pressure to obtain product A4 as a white solid (5.2g, 85%). The confirmed information for the product structure is as follows: ¹H NMR (CDCl₃): d, 7.45-7.34 (m, 3H, trityl), 7.33-7.27 (m, 6H, trityl), 7.25-7.21 (m, 1H, trityl), 6.84-6.79 (m, 4H, trityl), 4.15-4.09 (m, 1H), 4.03-4.02 (m, 1H), 3.91-3.86 (m, 2H), 3.79 (s, 6H), 3.78-3.75 (m, 2H), 3.61-3.55 (m, 4H), 3.21-3.17 (m, 2H), 3.11-3.08 (m, 1H), 2.66-2.62 (m, 2H), 2.12-2.08 (m, 2H), 1.58-1.54 (m, 2H), 1.32-1.25 (m, 22H), 1.20-1.17 (m, 6H), 1.12-1.11 (m, 6H), 0.90-0.86 (m, 3H) ppm. ³¹P NMR (CDCl₃): d, 149.16, 148.96 ppm. It can be seen that the structure of the product was confirmed to be correct.

### (4) Synthesis of Lipid Compound Monomers (A5 and B5)

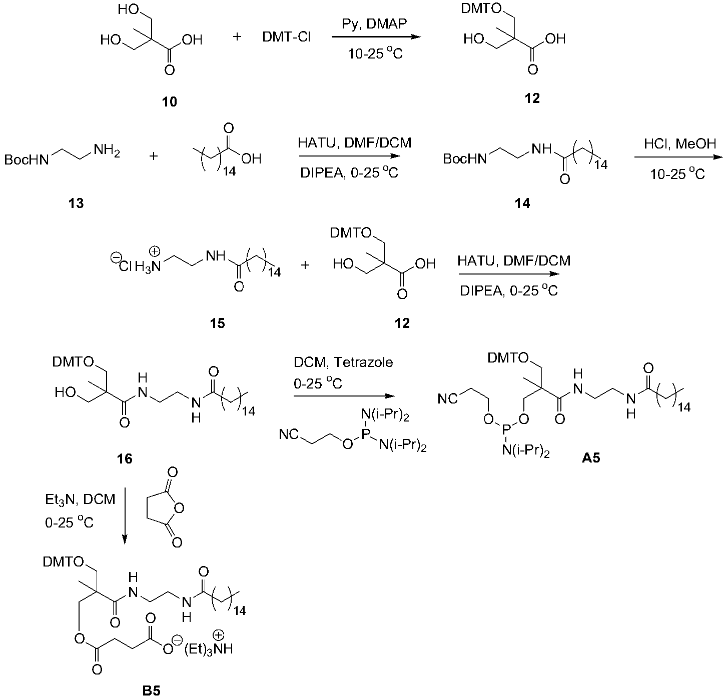

4,4'-Dimethoxytrityl chloride (1.8g, 5.3 mmol) was dissolved in 5 mL of dichloromethane. The solution was slowly added dropwise to a solution of 3-hydroxy-2-(hydroxymethyl)-2-methylpropanoic acid 10 (0.8g, 5.97 mmol) in anhydrous pyridine (10 mL) at room temperature. The solution was stirred continuously at room temperature for 14 hours. 20 mL of water was added to the reaction mixture, and the resulting mixture was then extracted with ethyl acetate (2×50 mL). The organic phase was concentrated to semi-dryness by rotary evaporation and further purified by silica gel column chromatography using one gradient elution: the organic phase was first eluted with n-hexane, then with n-hexane and ethyl acetate (n-hexane/ethyl acetate=1:1, v/v). The product components were collected and evaporated to dryness under reduced pressure to obtain product 12 as a yellow solid (1.5g, 58%), and the product 12 was used directly in the next step.

Palmitic acid (16.20g, 63.28 mmol) was dissolved in 240 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (26.4g, 69.61 mmol) and N,N-diisopropylethylamine (27.5 mL, 158.2 mmol) were sequentially added under an ice bath, and the reaction solution was stirred thoroughly at 0°C for 10 minutes. After the intermediate was formed, tert-butyl N-(2-aminoethyl)carbamate 13 (10.2g, 63.2 mmol) was dissolved in 50 mL of dichloromethane, and the resulting mixture was then added dropwise to a reaction flask. The reaction solution was warmed to room temperature (25°C) and further stirred at room temperature for 6 hours. The dichloromethane was removed under reduced pressure by rotary evaporation, the reaction solution was then added into 500 mL of saturated brine, and precipitation of a solid was observed. After complete precipitation of the solid, the solid was filtered out and washed with water and then with 50 mL of ethyl acetate to remove the unreacted reagent and the solvent. Finally, product 14 (23.5g, 93%) was dried under vacuum at room temperature and used directly in the next step.

Compound 14 (23.5g, 59 mmol) was dissolved in 300 mL of a solvent mixture (methanol (MeOH)/dichloromethane=1:1, v/v), and the reaction solution was mixed with 40 mL of 4M aqueous hydrochloric acid. The mixed reaction solution was stirred continuously at room temperature for 30 minutes, and then was further stirred at room temperature for 24 hours. After concentration by rotary evaporation, methanol and dichloromethane were removed. 100 mL of ethyl acetate was further added, and the reaction solution was mixed with ethyl acetate thoroughly and concentrated by rotary evaporation. The concentrated solution was mixed with 150 mL of a solvent mixture (n-hexane/ethyl acetate=1:1, v/v), and the solid product 15 gradually precipitated. After filtration and vacuum drying, the product 15 (20g, 90%) was used directly in the next reaction.

Compound 12 (18.4g, 31.1 mmol) was dissolved in 200 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (10.4g, 27.4 mmol) and N,N-diisopropylethylamine (22 mL, 74.2 mmol) were sequentially added under an ice bath, and the reaction solution was stirred thoroughly at 0°C for 20 minutes. After formation of the intermediate product, compound 15 (8.6g, 25.7 mmol) was dissolved in 50 mL of dichloromethane and the resulting mixture was then added dropwise to a reaction flask. The reaction solution was warmed to room temperature (25°C) and further stirred at room temperature for 12 hours. The dichloromethane was removed under reduced pressure by rotary evaporation, the reaction solution was then added into 500 mL of saturated brine, and the resulting mixture was extracted with ethyl acetate (2 × 250 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 16 as a yellow solid (13g, 59%).

Compound 16 (2.7g, 3.77 mmol) was dissolved in 50 mL of dry dichloromethane, and 2.6 mL of triethylamine was then added. 4-Dimethylaminopyridine (0.72g, 5.9 mmol) was dissolved in the reaction solution during stirring, succinic anhydride (0.57g, 5.6 mmol) was then dissolved in the reaction solution at room temperature with during, and the resulting mixture was stirred for reaction for 8 hours. Succinic anhydride (100 mg, 1 mmol) was further added, and the resulting mixture was further stirred at room temperature for 14 hours. The reaction solution was added into saturated brine, the mixture was extracted with ethyl acetate (2 × 250 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:2:0.01, v/v/v) to obtain the final product B5. The solvent was evaporated under reduced pressure to obtain compound B5 as a pale yellow solid (2.5g, 78%). The confirmed information for the product structure is as follows: ¹H NMR (CDCl₃): d, 7.39-7.37 (m, 3H, trityl), 7.29-7.26 (m, 6H, trityl), 7.21-7.18 (m, 1H, trityl), 6.84-6.81 (m, 4H, trityl), 4.34-4.32 (m, 1H), 4.16-4.14 (m, 1H), 3.78 (s, 6H), 3.35-3.27 (m, 3H), 3.25-2.97 (m, 3H), 2.54-2.50 (m, 4H), 2.08-2.00 (m, 2H), 1.30-1.19 (m,31H), 0.88-0.86 (m, 3H) ppm. It can be seen that the structure of the product was confirmed to be correct.

Compound 16 (7.16g, 10 mmol) was dissolved in 100 mL of dry dichloromethane, and N,N,N',N'-tetraisopropyl-2-cyanoethoxy phosphite (3.6 mL, 12 mmol) was then added rapidly to the solution. The reaction solution was stirred at room temperature for 20 minutes under nitrogen atmosphere. 22 mL of a solution of tetrazole (0.68g, 10 mmol) in dry dichloromethane was added to the above reaction solution, and the mixture was further stirred for reaction at room temperature under nitrogen atmosphere for 2 hours. The reaction solution was added into saturated brine, the mixture was extracted with dichloromethane (2 × 150 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A5 as a white solid (7g, 82%). The confirmed information for the product structure is as follows: ¹H NMR (CDCl₃): d, 7.42-7.40 (m, 3H, trityl), 7.30-7.27 (m, 6H, trityl), 7.23-7.21 (m, 1H, trityl), 6.84-6.82 (m, 4H, trityl), 4.14-4.09 (m, 1H), 3.79 (s, 6H), 3.77-3.71 (m, 4H), 3.70-3.66 (m, 4H), 3.55-3.49 (m, 2H), 2.56-2.53 (m, 2H), 2.04-2.00 (m, 2H), 1.55-1.51 (m, 2H), 1.31-1.20 (m, 22H), 1.16-1.11 (m, 6H), 1.09-1.08 (m, 6H), 0.88-0.87 (m, 3H) ppm. ³¹P NMR (CDCl₃): d, 148.57, 148.48 ppm. It can be seen that the structure of the product was confirmed to be correct.

### (5) Chemical Synthesis of Lipid Compound Monomers (A6-R1-R5 and B6-R1-R5)

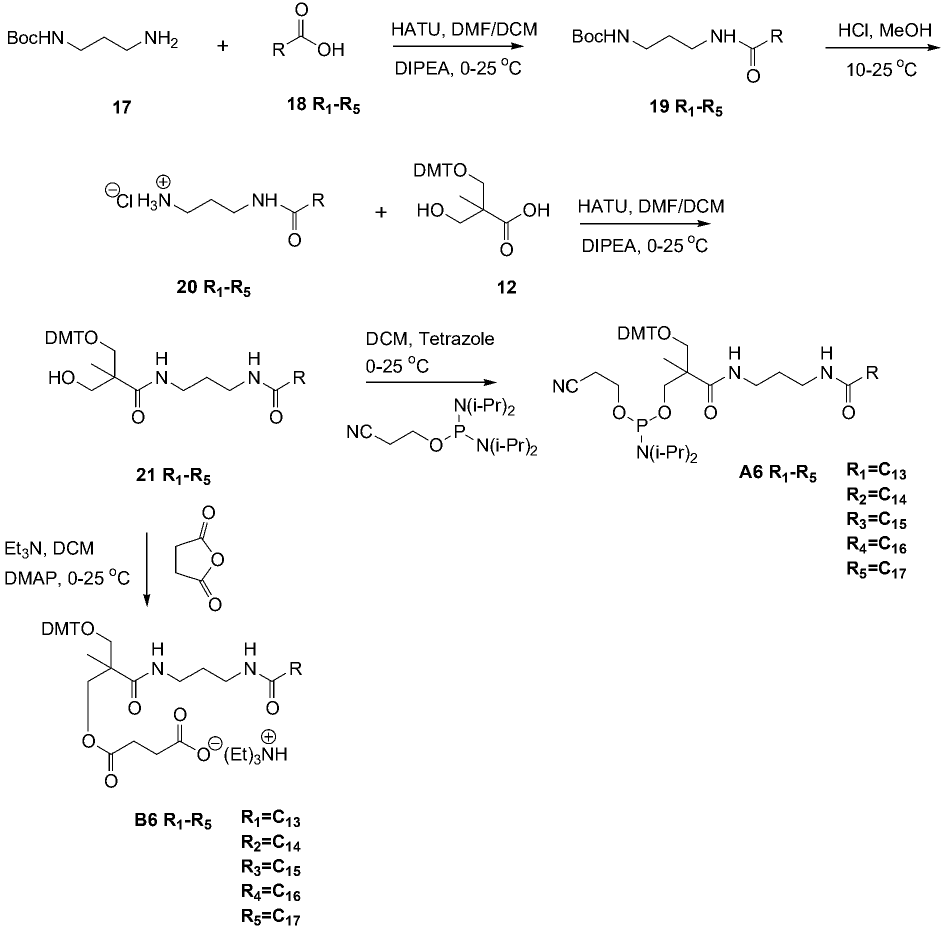

Palmitic acid (16.20g, 63.28 mmol) was dissolved in 250 mL of a solvent mixture (dried dimethylformamide/dried dichloromethane=1.5:1, v/v) at room temperature. After complete dissolution, 2-(7-azabenzotriazol-1-yl)-N,N,N',N'-tetramethyluronium hexafluorophosphate (26.4g, 69.61 mmol) and N,N-diisopropylethylamine (27.5 mL, 158.2 mmol) were sequentially added under an ice bath, and the reaction solution was stirred thoroughly at 0°C for 20 minutes. After the intermediate was formed, tert-butyl N-(3-aminopropyl)carbamate 17 (11g, 63.13 mmol) was dissolved in 50 mL of dichloromethane, and the resulting mixture was then added dropwise to a reaction flask. The reaction solution was warmed to room temperature (25°C) and further stirred at room temperature for 6 hours. The dichloromethane was removed under reduced pressure by rotary evaporation, the reaction solution was then added into 500 mL of saturated brine, and precipitation of a solid was observed. After complete precipitation of the solid, the solid was filtered out and washed with water and then with 50 mL of ethyl acetate to remove the unreacted reagent and the solvent. Finally, product 19-R3 (25g, 95%) was dried under vacuum at room temperature and used directly in the next step.

The same synthetic procedure was used, except that palmitic acid was replaced with myristic acid, pentadecanoic acid, heptadecanoic acid, and stearic acid, respectively. Products 19-R1 (24g, 94%), 19-R2 (26g, 96%), 19-R4 (21g, 91%), and 19-R5 (22g, 93%) were obtained respectively.

Compound 19-R3 (25g, 62 mmol) was dissolved in 200 mL of methanol. The reaction solution was mixed with 35 mL of 4 M aqueous hydrochloric acid, and 35 mL of dioxane was added. The mixed reaction solution was stirred continuously at room temperature for 30 minutes, and then was further stirred at room temperature for 24 hours. After concentration by rotary evaporation, methanol and dioxane were removed. 100 mL of ethyl acetate was further added, and the reaction solution was mixed with ethyl acetate thoroughly and concentrated by rotary evaporation. The concentrated solution was mixed with 150 mL of a solvent mixture (n-hexane/ethyl acetate=1:1, v/v), and the solid product 20-R3 gradually precipitated. After filtration and vacuum drying, the product 20-R3 (19g, 91%) was used directly in the next reaction.

The same synthetic procedure was used, except that compounds 19-R1, 19-R2, 19-R4, and 19-R5 were subjected to aftertreatment and precipitation, to obtain products 20-R1 (17g, 88%), 20-R2 (15g, 89%), 20-R4 (19g, 85%), and 20-R5 (14g, 82%), respectively.

Compound 12 (18.4g, 31.1 mmol) was dissolved in 200 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (9.4g, 24.8 mmol) and N,N-diisopropylethylamine (21.5 mL, 124 mmol) were sequentially added under an ice bath, and the reaction solution was stirred thoroughly at 0°C for 20 minutes. After formation of the intermediate product, compound 20-R3 (8.6g, 25.7 mmol) was dissolved in 50 mL of dichloromethane and the resulting mixture was then added dropwise to a reaction flask. The reaction solution was warmed to room temperature (25°C) and further stirred at room temperature for 4 hours. The dichloromethane was removed under reduced pressure by rotary evaporation, the reaction solution was then added into 500 mL of saturated brine, and the resulting mixture was extracted with ethyl acetate (2 × 250 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 21-R3 as a yellow solid (12g, 57%).

The same synthetic procedure was used, except that compounds 20-R1, 20-R2, 20-R4, and 20-R5 were separated and purified by silica gel column chromatography after reaction to obtain products 21-R1 (9g, 51%), 21-R2 (11g, 55%), 21-R4 (14g, 55%), and 21-R5 (13g, 56%), respectively.

The compound 21-R3 (2.26g, 3.1 mmol) was dissolved in 50 mL of dry dichloromethane, and 2.5 mL of triethylamine was then added. 4-Dimethylaminopyridine (0.72g, 5.9 mmol) was dissolved in the reaction solution during stirring, succinic anhydride (0.47g, 4.65 mmol) was then dissolved in the reaction solution at room temperature with during, and the resulting mixture was stirred for reaction for 8 hours. Succinic anhydride (100 mg, 1 mmol) was further added, and the resulting mixture was further stirred at room temperature for 14 hours. The reaction solution was added into saturated brine, the mixture was extracted with ethyl acetate (2 × 250 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:2:0.01, v/v/v) to obtain the final product B6-R3. The solvent was evaporated under reduced pressure to obtain compound B6-R3 as a pale yellow solid (2.1g, 75%). The confirmed information for the product structure is as follows: ¹H NMR (CDCl₃): d, 7.39-7.37 (m, 2H, trityl), 7.29-7.26 (m, 6H, trityl), 7.25-7.21 (m, 1H, trityl), 6.84-6.81 (m, 4H, trityl), 4.29-4.26 (m, 1H), 4.22-4.20 (m, 1H), 3.78 (s, 6H), 3.28-3.24 (m, 2H), 3.23-3.17 (m, 3H), 3.16-3.10 (m, 3H), 3.05-3.00 (m, 3H), 2.53-2.48 (m, 4H), 2.17-2.14 (m, 2H), 1.61-1.58 (m, 4H), 1.54-1.52 (m, 2H), 1.31-1.22 (m, 22H), 0.88-0.85 (m, 3H) ppm. It can be seen that the structure of the product was confirmed to be correct.

The same synthetic procedure was used, except that compounds 21-R1, 21-R2, 21-R4 and 21-R5 were separated and purified by silica gel column chromatography after reaction to obtain products B6-R1 (1.5g, 70%), B6-R2 (2.4g, 74%), B6-R4 (2.1g, 71%), and B6-R5 (1.8g, 68%), respectively.

Compound 21-R3 (4.5g, 6.16 mmol) was dissolved in 80 mL of dry dichloromethane, and N,N,N',N'-tetraisopropyl-2-cyanoethoxy phosphite (2.3 mL, 7.34 mmol) was then added rapidly to the solution. The reaction solution was stirred at room temperature for 20 minutes under nitrogen atmosphere. 20 mL of a solution of tetrazole (1.37 mL, 0.45M, 6.16 mmol) in dry dichloromethane was added to the above reaction solution, N,N,N',N'-tetraisopropyl-2-cyanoethoxy phosphite (0.5 mL, 0.26 mmol) was further added to the reaction solution, and the mixture was further stirred for reaction at room temperature under nitrogen atmosphere for 2 hours. The reaction solution was added into saturated brine, the mixture was extracted with dichloromethane (2 × 150 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A6-R3 as a white solid (4.9g, 85%). The confirmed information for the product structure is as follows: ¹H NMR (CDCl₃): d, 7.42-7.40 (m, 2H, trityl), 7.30-7.26 (m, 6H, trityl), 7.23-7.21 (m, 1H, trityl), 6.87-6.82 (m, 4H, trityl), 4.14-4.11 (m, 2H), 3.79 (s, 6H), 3.78-3.72 (m, 2H), 3.71-3.67 (m, 2H), 3.55-3.50 (m, 2H), 3.31-3.26 (m, 4H), 3.24-3.22 (m, 2H), 2.55-2.53 (m, 2H), 2.17-2.14 (m, 2H), 1.63-1.60 (m, 2H), 1.53-1.51 (m, 2H), 1.29-1.25 (m, 22H), 1.22-1.15 (m, 6H), 1.11-1.09 (m, 6H), 0.88-0.86 (m, 3H) ppm. ³¹P NMR (CDCl₃): d, 148.95, 148.52 ppm. It can be seen that the structure of the product was confirmed to be correct.

The same synthetic procedure was used, except that compounds 21-R1, 21-R2, 21-R4, and 21-R5 were separated and purified by silica gel column chromatography after reaction to obtain products.

A6-R1: (3.8g, 91%); ¹H NMR (CDCl₃): d, 7.42-7.40 (m, 2H, trityl), 7.30-7.23 (m, 6H, trityl), 7.21-7.19 (m, 1H, trityl), 6.88-6.81 (m, 4H, trityl), 4.14-4.11 (m, 2H), 3.79 (s, 6H), 3.77-3.71 (m, 2H), 3.70-3.66 (m, 2H), 3.57-3.47 (m, 2H), 3.31-3.25 (m, 4H), 3.23-3.15 (m, 2H), 2.55-2.52 (m, 2H), 2.17-2.14 (m, 2H), 1.80-1.60 (m, 2H), 1.53-1.51 (m, 2H), 1.28-1.24 (m, 18H), 1.22-1.16 (m, 6H), 1.14-1.08 (m, 6H), 0.89-0.86 (m, 3H) ppm. ³¹P NMR (CDCl₃): d, 148.56, 148.48 ppm.

A6-R2: (4.2g, 90%); ¹H NMR (CDCl₃): d, 7.42-7.40 (m, 2H, trityl), 7.30-7.23 (m, 6H, trityl), 7.22-7.20 (m, 1H, trityl), 6.87-6.82 (m, 4H, trityl), 4.13-4.11 (m, 2H), 3.79 (s, 6H), 3.78-3.71 (m, 2H), 3.70-3.66 (m, 2H), 3.55-3.50 (m, 2H), 3.31-3.24 (m, 4H), 3.23-3.14 (m, 2H), 2.55-2.53 (m, 2H), 2.17-2.14 (m, 2H), 1.63-1.60 (m, 2H), 1.58-1.51 (m, 2H), 1.28-1.19 (m, 18H), 1.16-1.11 (m, 6H), 1.09-1.08 (m, 6H), 0.89-0.86 (m, 3H) ppm. ³¹P NMR (CDCl₃): d, 148.59, 148.52 ppm.

A6-R4: (3.6g, 88%); ¹H NMR (CDCl₃): d, 7.42-7.40 (m, 2H, trityl), 7.30-7.23 (m, 6H, trityl), 7.23-7.20 (m, 1H, trityl), 6.87-6.82 (m, 4H, trityl), 4.14-4.11 (m, 2H), 3.79 (s, 6H), 3.78-3.72 (m, 2H), 3.71-3.66 (m, 2H), 3.55-3.50 (m, 2H), 3.31-3.24 (m, 4H), 3.23-3.12 (m, 2H), 2.55-2.53 (m, 2H), 2.17-2.14 (m, 2H), 1.67-1.61 (m, 2H), 1.60-1.53 (m, 2H), 1.29-1.19 (m, 24H), 1.16-1.11 (m, 6H), 1.09-1.08 (m, 6H), 0.89-0.86 (m, 3H) ppm. ³¹P NMR (CDCl₃): d, 148.59, 148.52 ppm.

A6-R5: (4.1g, 89%); ¹H NMR (CDCl₃): d, 7.42-7.40 (m, 2H, trityl), 7.30-7.23 (m, 6H, trityl), 7.23-7.19 (m, 1H, trityl), 6.88-6.81 (m, 4H, trityl), 4.13-4.11 (m, 2H), 3.79 (s, 6H), 3.78-3.70 (m, 2H), 3.70-3.67 (m, 2H), 3.55-3.49 (m, 2H), 3.31-3.22 (m, 4H), 3.15-3.12 (m, 2H), 2.56-2.52 (m, 2H), 2.18-2.14 (m, 2H), 1.61-1.52 (m, 4H), 1.28-1.20 (m, 26H), 1.16-1.10 (m, 6H), 1.09-1.08 (m, 6H), 0.89-0.86 (m, 3H) ppm. ³¹P NMR (CDCl₃): d, 148.56, 148.49 ppm.

It can be seen that the structure of the product was confirmed to be correct.

### (6) Synthesis of Lipid Compound Monomers (B7 and B8)

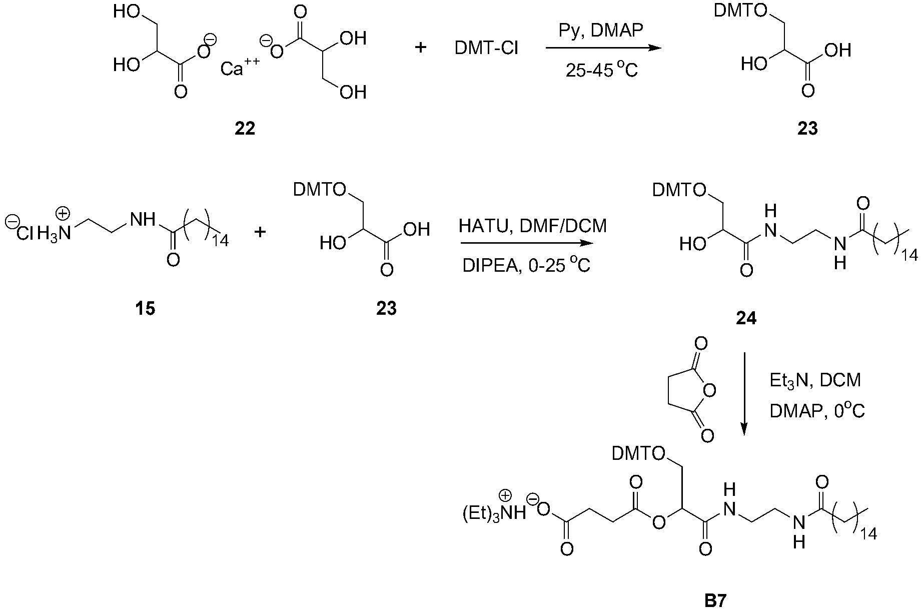

Calcium DL-glycerate hydrate (2.5g) was dissolved in 10 mL of anhydrous pyridine at room temperature. The resulting solution was slowly added to a solution of 4,4'-dimethoxytrityl chloride (6g, 17.7 mmol) in 10 mL of anhydrous pyridine. The mixture was stirred continuously at room temperature for 1 hour, then heated to 45°C and stirred continuously for 12 hours. 40 mL of water was added to the reaction solution, and the resulting mixture was then extracted with ethyl acetate (2×50 mL). The organic phase was concentrated to semi-dryness by rotary evaporation and further purified by silica gel column chromatography: The organic phase was first eluted with n-hexane, and then subjected to one gradient elution (n-hexane/ethyl acetate=3:1/1:1/1:3; v/v, containing 1% triethylamine). The product components were collected and evaporated to dryness under reduced pressure to obtain product 24 as a yellow solid (1.5g, 58%), and the product 24 was used directly in the next step.

Compound 23 (0.9g, 2.2 mmol) was dissolved in 20 mL of a solvent mixture (dried dimethylformamide/dried dichloromethane=1:1, v/v) at room temperature. After complete dissolution, 2-(7-azabenzotriazol-1-yl)-N,N,N, N'-tetramethyluronium hexafluorophosphate (0.76g, 2 mmol) and N,N-diisopropylethylamine (2 mL, 13.2 mmol) were sequentially added under an ice bath, and the reaction solution was stirred thoroughly at 0°C for 20 minutes. After formation of the intermediate product, compound 15 (0.65g, 2.2 mmol) was dissolved in 5 mL of dichloromethane and the resulting mixture was then added dropwise to a reaction flask. The reaction solution was warmed to room temperature (25°C) and further stirred at room temperature for 12 hours. The dichloromethane was removed under reduced pressure by rotary evaporation, the reaction solution was then added into 100 mL of saturated brine, and the resulting mixture was extracted with ethyl acetate (2 × 150 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 24 as a yellow solid (0.84g, 59%).

The compound 24 (0.84g, 1.22 mmol) was dissolved in 50 mL of dry dichloromethane, and 0.85 mL of triethylamine was then added. 4-Dimethylaminopyridine (0.72g, 5.9 mmol) was dissolved in the reaction solution during stirring, succinic anhydride (0.183g, 1.83 mmol) was then dissolved in the reaction solution at room temperature with during, and the resulting mixture was stirred for reaction for 8 hours. Succinic anhydride (50 mg, 0.2 mmol) was further added, and the resulting mixture was further stirred at room temperature for 14 hours. The reaction solution was added into saturated brine, the mixture was extracted with ethyl acetate (2 × 250 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:2:0.01, v/v/v) to obtain the final product B7. The solvent was evaporated under reduced pressure to obtain compound B7 as a pale yellow solid (0.87g, 75%). The confirmed information for the product structure is as follows: ¹H NMR (CDCl₃): d, 7.82 (m, 2H), 7.41-7.39 (m, 3H, trityl), 7.29-7.27 (m, 6H, trityl), 7.25-7.19 (m, 1H, trityl), 6.82-6.80 (m, 4H, trityl), 5.34-5.32 (m, 1H), 3.78 (s, 6H), 3.55-3.52 (m, 2H), 3.46-3.41 (m, 2H), 3.34-3.31 (m, 3H), 3.00-2.96 (m, 2H), 2.72-2.70 (m, 2H), 2.69-2.59 (m, 1H), 2.08-2.05 (m, 2H), 1.52-1.50 (m, 2H), 1.30-1.22 (m, 22H), 0.89-0.86 (m, 3H) ppm. It can be seen that the structure of the product was confirmed to be correct.

The same synthetic procedure was used, except that starting material 15 was replaced with compound 20-R3, to obtain product B8 (0.42g, 77%). The confirmed information for the product structure is as follows: ¹H NMR (CDCl₃): d, 7.47 (m, 2H, trityl), 7.41-7.39 (m, 3H, trityl), 7.30-7.27 (m, 6H, trityl), 7.25-7.19 (m, 1H, trityl), 6.82-6.80 (m, 4H, trityl), 6.18 (m, 1H), 5.40-5.39 (m, 1H), 3.78 (s, 6H), 3.57-3.56 (m, 1H), 3.55-3.54 (m, 1H), 3.45-3.42 (m, 2H), 3.37-3.34 (m, 1H), 3.34-3.29 (m, 1H), 3.08-3.03 (m, 3H), 2.75-2.68 (m, 2H), 2.20-2.17 (m, 2H), 1.74-2.051.71 (m, 2H), 1.62-1.59 (m, 2H), 1.31-1.13 (m, 22H), 0.89-0.86 (m, 3H) ppm. It can be seen that the structure of the product was confirmed to be correct.

### (7) Synthesis of Lipid Compound Monomers (A9 and B9)

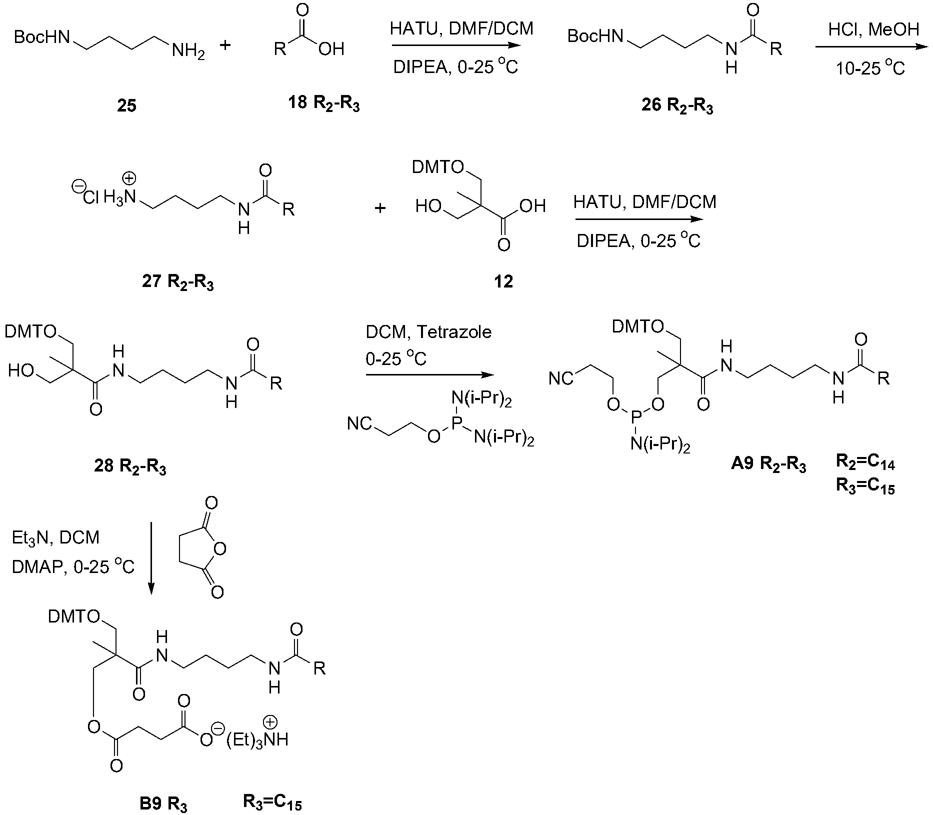

Palmitic acid 18-R3 (12.80g, 50 mmol) was dissolved in 150 mL of a solvent mixture (dried dimethylformamide/dried dichloromethane=1:1, v/v) at room temperature. After complete dissolution, 2-(7-azabenzotriazol-1-yl)-N,N,N,N'-tetramethyluronium hexafluorophosphate (21.2g, 55 mmol) and N,N-diisopropylethylamine (22 mL, 125 mmol) were sequentially added under an ice bath, and the reaction solution was stirred thoroughly at 0°C for 10 minutes. After the intermediate was formed, tert-butyl N-(4-aminobutyl)carbamate 25 (9.5g, 51 mmol) was dissolved in 50 mL of dichloromethane, and the resulting mixture was then added dropwise to a reaction flask. The reaction solution was warmed to room temperature (25°C) and further stirred at room temperature for 6 hours. The dichloromethane was removed under reduced pressure by rotary evaporation, the reaction solution was then added into 500 mL of saturated brine, and precipitation of a solid was observed. After complete precipitation of the solid, the solid was filtered out and washed with water and then with ethyl acetate (2 × 50 mL)to remove the unreacted reagent and the solvent. Finally, product 26-R3 (10.5g, 93%) was dried under vacuum at room temperature and used directly in the next step.

The same synthetic procedure was used, except that palmitic acid was replaced with pentadecanoic acid, to obtain product 26-R2 (7.8g, 91%).

Compound 26-R3 (10.5g, 24.6 mmol) was dissolved in 100 mL of a solvent mixture (methanol/dichloromethane=1:1, v/v) and the reaction solution was mixed with 30 mL of 4M aqueous hydrochloric acid. The mixed reaction solution was stirred continuously at room temperature for 30 minutes, and then was further stirred at room temperature for 20 hours. After concentration by rotary evaporation, methanol and dichloromethane were removed. 100 mL of ethyl acetate was further added, and the reaction solution was mixed with ethyl acetate thoroughly and concentrated by rotary evaporation. The concentrated solution was mixed with 150 mL of a solvent mixture (n-hexane/ethyl acetate=1:1, v/v), and the solid product 27-R3 gradually precipitated. After filtration and vacuum drying, the product 27-R3 (9.5g, 90%) was used directly in the next reaction.

The same synthetic procedure was used, except that compound 26-R2 was subjected to aftertreatment and precipitation, to obtain product 27-R2 (11.2g, 85%).

Compound 12 (8.72g, 20 mmol) was dissolved in 40 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (7.6g, 20 mmol) and N,N-diisopropylethylamine (7 mL, 40 mmol) were sequentially added under an ice bath, and the reaction solution was stirred thoroughly at 0°C for 20 minutes. After formation of the intermediate product, compound 27-R3 (5.8g, 16 mmol) was dissolved in 50 mL of dichloromethane and the resulting mixture was then added dropwise to a reaction flask. The reaction solution was warmed to room temperature (25°C) and further stirred at room temperature for 4 hours. The dichloromethane was removed under reduced pressure by rotary evaporation, the reaction solution was then added into 250 mL of saturated brine, and the resulting mixture was extracted with ethyl acetate (2 × 200 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 28-R3 as a yellow solid (9g, 59%).

The same synthetic procedure was used, except that compound 27-R2 was separated and purified by silica gel column chromatography after reaction, to obtain product 28-R2 (11g, 55%).

The compound 28-R3 (1.33g, 2.0 mmol) was dissolved in 20 mL of dry dichloromethane, and 1.4 mL of triethylamine was then added. 4-Dimethylaminopyridine (0.02g, 5.9 mmol) was dissolved in the reaction solution during stirring, succinic anhydride (0.3g, 3.0 mmol) was then dissolved in the reaction solution at room temperature with during, and the resulting mixture was stirred for reaction for 8 hours. Succinic anhydride (20 mg, 0.2 mmol) was further added, and the resulting mixture was further stirred at room temperature for 14 hours. The reaction solution was added into saturated brine, the mixture was extracted with ethyl acetate (2 × 100 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:2:0.01, v/v/v) to obtain the final product B9. The solvent was evaporated under reduced pressure to obtain compound B9 as a pale yellow solid (1.5g, 78%). The confirmed information for the product structure is as follows: ¹H NMR (CDCl₃): d, 7.40-7.38 (m, 2H, trityl), 7.30-7.27 (m, 6H, trityl), 6.84-6.80 (m, 4H, trityl), 6.70 (m, 1H), 4.33-4.30 (m, 1H), 4.18-4.16 (m, 1H), 3.79 (s, 6H), 3.27-3.21 (m, 7H), 3.04-3.03 (m, 2H), 2.55-2.51 (m, 2H), 1.60-1.58 (m, 3H), 1.46-1.44 (m, 4H), 1.31-1.20 (m, 24H), 0.89-0.86 (m, 3H) ppm. It can be seen that the structure of the product was confirmed to be correct.

Compound 28-R3 (5.3g, 7.1 mmol) was dissolved in 70 mL of dry dichloromethane, and N,N,N',N'-tetraisopropyl-2-cyanoethoxy phosphite (2.8 mL, 9.23 mmol) was then added rapidly to the solution. The reaction solution was stirred at room temperature for 20 minutes under nitrogen atmosphere. 14 mL of a solution of tetrazole (6.39 mmol, 0.45M) in dry dichloromethane was added to the above reaction solution, and the mixture was further stirred for reaction at room temperature under nitrogen atmosphere for 2 hours. The reaction solution was added into saturated brine, the mixture was extracted with dichloromethane (2 × 100 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A9-R3 as a white solid (5.6g, 82%). The confirmed information for the product structure is as follows: ¹H NMR (CDCl₃): d, 7.43-7.41 (m, 2H, trityl), 7.31-7.22 (m, 6H, trityl), 7.23-7.21 (m, 1H, trityl), 6.84-6.82 (m, 4H, trityl), 6.67 (m, 1H), 5.29 (m, 1H), 3.78 (s, 6H), 3.77-3.66 (m, 4H, 3.54-3.51 (m, 2H), 3.28-3.24 (m, 2H), 3.22-3.17 (m, 4H), 2.55-2.53 (m, 2H), 2.09-2.04 (m, 2H), 1.56-1.55 (m, 2H), 1.45-1.42 (m, 4H), 1.30-1.22 (m, 26H), 1.20-1.15 (m, 6H), 1.11-1.08 (m, 6H), 0.89-0.86 (m, 3H) ppm. ³¹P NMR (CDCl₃): d, 148.44, 148.40 ppm. It can be seen that the structure of the product was confirmed to be correct.

The same synthetic procedure was used, except that compound 28-R2 was separated and purified by silica gel column chromatography after reaction, to obtain product A9-R2 (3.3g, 89%). The confirmed information for the product structure is as follows: ¹H NMR (CDCl₃): d, 7.42-7.41 (m, 2H, trityl), 7.31-7.27 (m, 6H, trityl), 7.22-7.21 (m, 1H, trityl), 6.83-6.82(m, 4H, trityl), 4.12-4.11 (m, 1H), 3.79 (s, 6H), 3.77-3.66 (m, 4H), 3.54-3.52 (m, 3H), 3.28-3.22 (m, 2H), 3.20-3.17 (m, 3H), 2.55-2.53 (m, 2H), 2.09-2.06 (m, 2H), 1.58-1.55 (m, 2H), 1.45-1.43 (m, 4H), 1.29-1.20 (m, 29H), 1.11-1.08 (m, 6H), 0.89-0.86 (m, 6H) ppm. ³¹P NMR (CDCl₃): d, 148.50, 148.46 ppm. It can be seen that the structure of the product was confirmed to be correct.

### (8) Synthesis of Lipid Compound Monomers (A10 and B10)

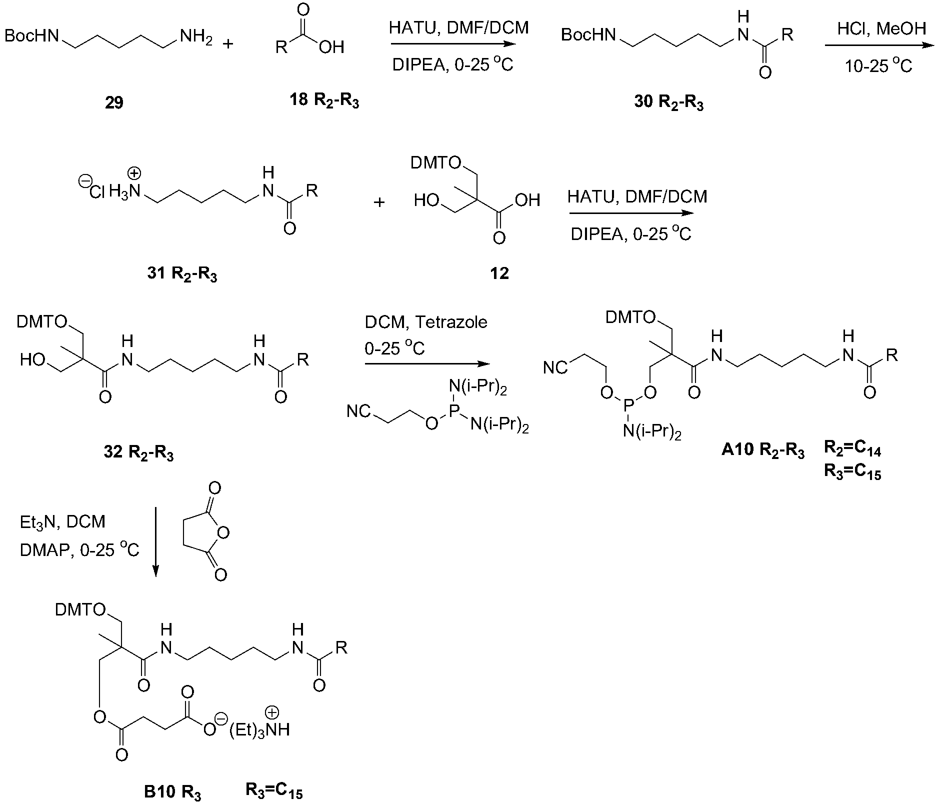

Palmitic acid 18-R3 (6.4g, 25 mmol) was dissolved in 100 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (10.5g, 27.5 mmol) and N,N-diisopropylethylamine (11 mL, 63.2 mmol) were sequentially added under an ice bath, and the reaction solution was stirred thoroughly at 0°C for 10 minutes. After the intermediate was formed, tert-butyl N-(5-aminopentyl)carbamate and tert-butyl N-(4-aminoethyl)carbamate 29 (5.1g, 25.2 mmol) was dissolved in 50 mL of dichloromethane, and the resulting mixture was then added dropwise to a reaction flask. The reaction solution was warmed to room temperature (25°C) and further stirred at room temperature for 6 hours. The dichloromethane was removed under reduced pressure by rotary evaporation, the reaction solution was then added into 200 mL of saturated brine, and precipitation of a solid was observed. After complete precipitation of the solid, the solid was filtered out and washed with water and then with 50 mL of ethyl acetate to remove the unreacted reagent and the solvent. Finally, product 30-R3 (11g, 91%) was dried under vacuum at room temperature and used directly in the next step.

The same synthetic procedure was used, except that palmitic acid was replaced with pentadecanoic acid, to obtain product 30-R2 (9.5g, 94%).

Compound 30-R3 (11g, 24.9 mmol) was dissolved in 100 mL of a solvent mixture (methanol/dichloromethane=1:1, v/v) and the reaction solution was mixed with 30 mL of 4M aqueous hydrochloric acid. The mixed reaction solution was stirred continuously at room temperature for 30 minutes, and then was further stirred at room temperature for 20 hours. After concentration by rotary evaporation, methanol and dichloromethane were removed. 100 mL of ethyl acetate was further added, and the reaction solution was mixed with ethyl acetate thoroughly and concentrated by rotary evaporation. The concentrated solution was mixed with 100 mL of a solvent mixture (n-hexane/ethyl acetate=1:1, v/v), and the solid product 31-R3 gradually precipitated. After filtration and vacuum drying, the product 31-R3 (10g, 90%) was used directly in the next reaction.

The same synthetic procedure was used, except that compound 30-R2 was subjected to aftertreatment and precipitation, to obtain product 31-R2 (8.8g, 87%).

Compound 12 (8.72g, 20 mmol) was dissolved in 40 mL of a solvent mixture (dried dimethylformamide/dried dichloromethane=1:1, v/v) at room temperature. After complete dissolution, 2-(7-azabenzotriazol-1-yl)-N,N,N',N'-tetramethyluronium hexafluorophosphate (7.6g, 20 mmol) and N,N-diisopropylethylamine (7 mL, 40 mmol) were sequentially added under an ice bath, and the reaction solution was stirred thoroughly at 0°C for 20 minutes. After formation of the intermediate product, compound 31-R3 (6g, 16 mmol) was dissolved in 50 mL of dichloromethane and the resulting mixture was then added dropwise to a reaction flask. The reaction solution was warmed to room temperature (25°C) and further stirred at room temperature for 4 hours. The dichloromethane was removed under reduced pressure by rotary evaporation, the reaction solution was then added into 250 mL of saturated brine, and the resulting mixture was extracted with ethyl acetate (2 × 200 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 32-R3 as a yellow solid (9g, 59%).

The same synthetic procedure was used, except that compound 31-R2 was separated and purified by silica gel column chromatography after reaction, to obtain product 32-R2 (7.5g, 54%).

The compound 32-R3 (1.4g, 1.8 mmol) was dissolved in 40 mL of dry dichloromethane, and 1.2 mL of triethylamine was then added. 4-Dimethylaminopyridine (0.02g, 0.17 mmol) was dissolved in the reaction solution during stirring, succinic anhydride (0.57g, 5.6 mmol) was then dissolved in the reaction solution at room temperature with during, and the resulting mixture was stirred for reaction for 8 hours. Succinic anhydride (276 mg, 2.7 mmol) was further added, and the resulting mixture was further stirred at room temperature for 14 hours. The reaction solution was added into saturated brine, the mixture was extracted with ethyl acetate (2 × 150 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine=10:2:0.01, v/v/v) to obtain the final product B10. The solvent was evaporated under reduced pressure to obtain compound B10-R3 as a pale yellow solid (1.16g, 75%). The confirmed information for the product structure is as follows: ¹H NMR (CDCl₃): d, 7.40-7.38 (m, 2H, trityl), 7.30-7.27 (m, 6H, trityl), 7.23-7.19 (m, 1H, trityl), 6.84-6.81 (m, 4H, trityl), 6.68 (m, 1H), 4.34-4.31 (m, 1H), 4.18-4.15 (m, 1H), 3.78 (s, 6H), 3.27-3.18 (m, 6H), 3.15-3.14 (m, 1H), 3.05-3.01 (m, 2H), 2.57-2.50 (m, 4H), 2.15-2.14 (m, 2H), 1.57-1.49 (m, 2H), 1.47-1.43 (m, 5H), 1.31-1.18 (m, 26H), 0.89-0.86 (m, 3H) ppm. It can be seen that the structure of the product was confirmed to be correct.

Compound 32-R3 (6.1g, 8 mmol) was dissolved in 70 mL of dry dichloromethane, and N,N,N',N'-tetraisopropyl-2-cyanoethoxy phosphite (3.14 mL, 10.4 mmol) was then added rapidly to the solution. The reaction solution was stirred at room temperature for 20 minutes under nitrogen atmosphere. 16 mL of a solution of tetrazole (7.2 mmol, 0.45M) in dry dichloromethane was added to the above reaction solution, and the mixture was further stirred for reaction at room temperature under nitrogen atmosphere for 2 hours. The reaction solution was added into saturated brine, the mixture was extracted with dichloromethane (2 × 120 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A10-R3 as a white solid (6.44g, 84%). The confirmed information for the product structure is as follows: ¹H NMR (CDCl₃): d, 7.42-7.40 (m, 2H, trityl), 7.31-7.27 (m, 6H, trityl), 7.26-7.22 (m, 1H, trityl), 6.84-6.82 (m, 4H, trityl), 6.76 (m, 1H), 5.29 (m, 1H), 3.79 (s, 6H), 3.78-3.68 (m, 4H), 5.54-3.51 (m, 3H), 3.27-3.25 (m, 2H), 3.18-3.14 (m, 4H), 2.55-2.52 (m, 2H), 2.12-2.09 (m, 2H), 1.66-1.58 (m, 2H), 1.48-1.40 (m, 4H), 1.31-1.22 (m, 28H), 1.16-1.15 (m, 6H), 1.11-1.09 (m, 6H), 0.89-0.86 (m, 3H) ppm. ³¹P NMR (CDCl₃): d, 148.47, 148.43 ppm. It can be seen that the structure of the product was confirmed to be correct.

The same synthetic procedure was used, except that compound 32-R2 was separated and purified by silica gel column chromatography after reaction, to obtain product A10-R2 (3.25g, 91%). The confirmed information for the product structure is as follows: ¹H NMR (CDCl₃): d, 7.42-7.40 (m, 2H, trityl), 7.31-7.27 (m, 6H, trityl), 7.23-7.21 (m, 1H, trityl), 6.83-6.82 (m, 4H, trityl), 4.12-4.11 (m, 1H), 3.79 (s, 6H), 3.71-3.67 (m, 2H), 3.53-3.52 (m, 2H), 3.27-3.25 (m, 2H), 3.18-3.14 (m, 4H), 2.55-2.52 (m, 2H), 2.12-2.11 (m, 2H), 1.60-1.59 (m, 2H), 1.48-1.40 (m, 4H), 1.31-1.22 (m, 29H), 1.16-1.11 (m, 6H), 1.09-1.08 (m, 6H), 0.89-0.86 (m, 3H) ppm. ³¹P NMR (CDCl₃): d, 148.53, 148.49 ppm. It can be seen that the structure of the product was confirmed to be correct.

### (9) Synthesis of Lipid Compound Monomer (A11)

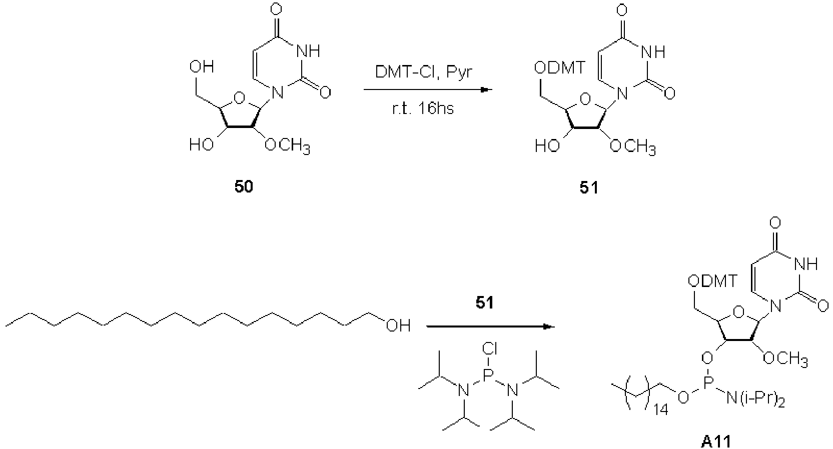

4,4'-Dimethoxytrityl chloride (6.8g, 20 mmol) was dissolved in 10 mL of dichloromethane. At room temperature, the solution was added dropwise slowly to a solution of 2'-O-methyluridine 50 (5.16g, 20 mmol) in anhydrous pyridine (30 mL), and 0.2 mL of 4-(dimethylamino)pyridine was added. The solution was stirred continuously at room temperature for 14 hours. 50 mL of water was added to the reaction mixture, and the resulting mixture was then extracted with ethyl acetate (2 × 80 mL). The organic phase was concentrated to semi-dryness by rotary evaporation and further purified by silica gel column chromatography using one gradient elution: the organic phase was first eluted with solvents (n-hexane/ethyl acetate=3:1, v/v), then with n-hexane and ethyl acetate (n-hexane/ethyl acetate=2:1, v/v), and finally with n-hexane and ethyl acetate (n-hexane/ethyl acetate=1:1, v/v). The product components were collected and evaporated to dryness under reduced pressure to obtain white foam-like solid 51 (9.5g, 58%), and the product 51 was used directly in the next step.

Hexadecanol (2.42g, 10 mmol) was dissolved in 80 mL of dry dichloromethane. Then N,N,N',N'-tetraisopropylphosphoramidous chloride (3.2g, 12 mmol) and diisopropylethylamine (3.6 mL, 24 mmol) were added rapidly to the solution. The reaction solution was stirred at room temperature for 2 hours under nitrogen atmosphere. A solution of compound 51 (5.7g, 10 mmol) in 25 mL of dichloromethane was added to the above reaction solution, and then 1H-tetrazole (5 mL, 0.45 M) was added. The reaction solution was further stirred at room temperature for reaction for two hours. The reaction solution was added into saturated brine, the mixture was extracted with dichloromethane (2 × 80 mL), and the organic phase was separated, dried over anhydrous sodium sulfate, and evaporated to semi-dryness under reduced pressure. The residue was purified by silica gel chromatography using one gradient elution: the residue was first eluted with a solvent mixture (n-hexane/ethyl acetate=10:1, v/v, containing 1% triethylamine), and then with another solvent mixture (n-hexane/ethyl acetate=3:1, v/v, containing 1% triethylamine). The solvent was evaporated under reduced pressure to obtain compound A11 as a nearly white, transparent foam (6.9g, 74%). The confirmed information for the product structure is as follows: ¹H NMR (CD₃CN): d, 8.08-8.03 (m, 2H), 7.42-7.40 (m, 3H, trityl), 7.30-7.27 (m, 6H, trityl), 7.23-7.21 (m, 1H, trityl), 6.85-6.82 (m, 4H, trityl), 5.99-5.98 (m, 2H), 5.20-5.16 (m, 2H), 4.50 (m, 1H), 4.40 (m, 1H), 4.24 (m, 2H), 3.83-3.80 (m, 2H), 3.79 (s, 6H), 3.61-3.57 (m, 4H), 1.70 (m, 2H), 1.50 (m, 2H), 1.31-1.21 (m, 26H), 1.04-1.03 (m, 6H), 0.89-0.86 (m, 6H), 1.09-1.08 (m, 6H) ppm. ³¹P NMR (CDCl₃): d, 149.08, 148.63 ppm. It can be seen that the structure of the product was confirmed to be correct.

### (10) Synthesis of Lipid Compound Monomer (A12)

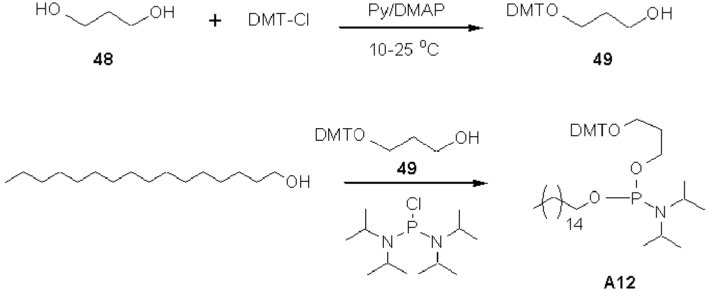

4,4'-Dimethoxytrityl chloride (18g, 0.053 mol) was dissolved in a solvent mixture (40 mL of dichloromethane, 8.7 mL of triethylamine, and 0.1 mL of 4-(dimethylamino)pyridine). 1,3-Propanediol (21g, 0.265 mol) was added dropwise to the above solution during stirring, and the mixture was further stirred at room temperature for 12 hours. The reaction solution was added into 100 mL of saturated brine, the mixture was extracted with 300 mL of dichloromethane, and the organic phase was separated, dried over anhydrous sodium sulfate, and evaporated to semi-dryness under reduced pressure. The residue was purified by silica gel chromatography using one gradient elution: the residue was first eluted with a solvent mixture (n-hexane/ethyl acetate=3:1, v/v), and then with another solvent mixture (n-hexane/ethyl acetate=1:1, v/v). The solvent was evaporated under reduced pressure to obtain an orange compound 1-O-(4,4'-dimethoxytrityl)-1,3-propanediol (49) (16g, 80%). The confirmed information for the product structure is as follows: ¹H NMR (CDCl₃): d, 7.43-7.41 (2H, m), 7.33-7.27 (6H, m), 7.29-7.27 (1H, m), 6.85-6.82 (4H, m), 3.79-3.75 (7H, m), 3.29-3.27 (2H, m), 2.20-2.18 (1H, br), 1.88-1.83 (2H, br), 1.57 (1H, s). It can be seen that the structure of the product was confirmed to be correct.

1-O-(4,4'-dimethoxytrityl)-1,3-propanediol 49 (4.5g, 12 mmol) was dissolved in 80 mL of dry dichloromethane. Then N,N,N',N'-tetraisopropylphosphoramidous chloride (3.85g, 14.4 mmol) and diisopropylethylamine (4.4 mL, 24 mmol) were added rapidly to the solution. The reaction solution was stirred at room temperature for 15 minutes under nitrogen atmosphere. A solution of 1-hexadecanol (3.9g, 15.6 mmol) was added to the above reaction solution, and then 1H-tetrazole (6 mL, 0.45 M, 3 mmol) was added. The reaction solution was further stirred at room temperature for reaction for one hour.

The reaction solution was added into saturated brine, the mixture was extracted with dichloromethane (2 × 60 mL), and the organic phase was separated, dried over anhydrous sodium sulfate, and evaporated to semi-dryness under reduced pressure. The residue was purified by silica gel chromatography using one gradient elution: the residue was first eluted with a solvent mixture (n-hexane/ethyl acetate=10:1, v/v, containing 1% triethylamine), and then with another solvent mixture (n-hexane/ethyl acetate=5:1, v/v, containing 1% triethylamine). The solvent was evaporated under reduced pressure to obtain compound A12 as a nearly white, transparent foam (5g, 56%). The confirmed information for the product structure is as follows: ¹H NMR (DMSO-d₆): d, 7.42-7.40 (m, 3H, trityl), 7.30-7.27 (m, 6H, trityl), 7.23-7.21 (m, 1H, trityl), 6.84-6.82 (m, 4H, trityl), 3.71 (s, 6H), 3.67-3.62 (m, 2H), 3.48-3.42 (m, 4H), 3.06-3.02 (m, 2H), 1.81-1.77 (m, 2H), 1.25-1.17 (m, 25H), 1.11-1.07 (m, 10H), 0.85-0.82 (m, 3H) ppm. ³¹P NMR (DMSO-d₆): d, 145.12, 144.96 ppm. It can be seen that the structure of the product was confirmed to be correct.

### (11) Synthesis of Lipid Compound Monomer (A19)

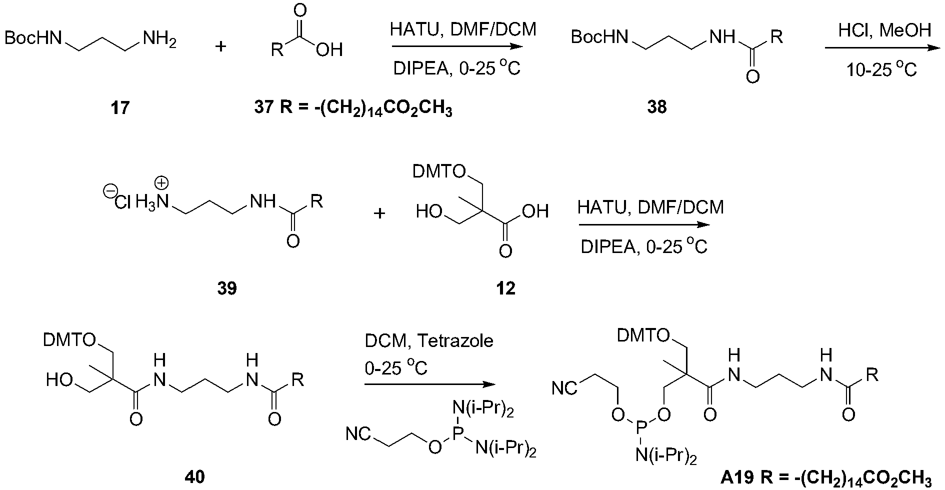

Palmitic acid (5g, 16.64 mmol) was dissolved in 25 mL of a solvent mixture (dried dimethylformamide/dried dichloromethane=1.5:1, v/v) at room temperature. After complete dissolution, 2-(7-azabenzotriazol-1-yl)-N,N,N, N'-tetramethyluronium hexafluorophosphate (6.6g, 17.47 mmol) and N,N-diisopropylethylamine (8.7 mL, 49.92 mmol) were sequentially added under an ice bath, and the reaction solution was stirred thoroughly at 0°C for 20 minutes. After the intermediate was formed, tert-butyl N-(3-aminopropyl)carbamate 37 (2.9g, 16.64 mmol) was dissolved in 10 mL of dichloromethane, and the resulting mixture was then added dropwise to a reaction flask. The reaction solution was warmed to room temperature (25°C) and further stirred at room temperature for 5 hours. The dichloromethane was removed under reduced pressure by rotary evaporation, the reaction solution was then added into 500 mL of saturated brine, and precipitation of a solid was observed. After complete precipitation of the solid, the solid was filtered out and washed with water and then with 50 mL of ethyl acetate to remove the unreacted reagent and the solvent. Finally, product 38 (4.7g, 92%) was dried under vacuum at room temperature and used directly in the next step.

Compound 38 (4.6g, 10.1 mmol) was dissolved in 30 mL of methanol. The reaction solution was mixed with 10 mL of 4 M aqueous hydrochloric acid, and 30 mL of dioxane was added. The mixed reaction solution was stirred continuously under ice bath for 30 minutes, and then was further warmed to and stirred at room temperature for 18 hours. After concentration by rotary evaporation, methanol and dioxane were removed. 50 mL of ethyl acetate was further added, and the reaction solution was mixed with ethyl acetate thoroughly and concentrated by rotary evaporation. The concentrated solution was mixed with 100 mL of a solvent mixture (n-hexane/ethyl acetate=1:1, v/v), and the solid product 39 gradually precipitated. After filtration and vacuum drying, the product 39 (4g, 90%) was used directly in the next reaction.

Compound 12 (4.7g, 7.94 mmol) was dissolved in 50 mL of a solvent mixture (dried dimethylformamide/dried dichloromethane=1:1, v/v) at room temperature. After complete dissolution, 2-(7-azabenzotriazol-1-yl)-N,N,N, N'-tetramethyluronium hexafluorophosphate (3g, 7.9 mmol) and N,N-diisopropylethylamine (5.5 mL, 26.48 mmol) were sequentially added under an ice bath, and the reaction solution was stirred thoroughly at 0°C for 20 minutes. After formation of the intermediate product, compound 39 (2.6g, 6.62 mmol) was dissolved in 20 mL of dichloromethane and the resulting mixture was then added dropwise to a reaction flask. The reaction solution was warmed to room temperature (25°C) and further stirred at room temperature for 5 hours. The dichloromethane was removed under reduced pressure by rotary evaporation, the reaction solution was then added into 500 mL of saturated brine, and the resulting mixture was extracted with ethyl acetate (2 × 250 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=2:1, v/v, containing 1% triethylamine), further with n-hexane and ethyl acetate (n-hexane/ethyl acetate=1:1, v/v, containing 1% triethylamine), and finally with ethyl acetate and 1% triethylamine. The product components were collected and evaporated to dryness under reduced pressure to obtain compound 40 as a yellow solid (3.4g, 67%).

Compound 40 (3g, 3.88 mmol) was dissolved in 40 mL of dry dichloromethane, and N,N,N',N'-tetraisopropyl-2-cyanoethoxy phosphite (1.5 mL, 5.04 mmol) was then added rapidly to the solution. The reaction solution was stirred at room temperature for 20 minutes under nitrogen atmosphere. 20 mL of a solution of tetrazole (1.37 mL, 0.45M, 6.16 mmol) in dry dichloromethane was added to the above reaction solution, N,N,N',N'-tetraisopropyl-2-cyanoethoxy phosphite (0.22 mL, 0.11 mmol) was further added to the reaction solution, and the mixture was further stirred for reaction at room temperature under nitrogen atmosphere for 5 hours. The reaction solution was added into saturated brine, the mixture was extracted with dichloromethane (2 × 100 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A19 as a white solid (2.9g, 84%). The confirmed information for the product structure is as follows: ¹H NMR (CDCl₃): d, 7.42-7.39 (m, 2H, trityl), 7.30-7.26 (m, 6H, trityl), 7.23-7.21 (m, 1H, trityl), 6.84-6.81 (m, 4H, trityl), 3.79 (s, 6H), 3.77-3.70 (m, 2H), 3.69 (s, 3H), 3.69-3.65 (m, 2H), 3.53-3.51 (m, 4H), 3.29-3.23 (m, 4H), 3.23-3.13 (m, 2H), 2.31-2.28 (m, 2H), 2.17-2.14 (m, 2H), 2.04 (s, 1H), 1.63-1.59 (m, 2H), 1.31-1.20 (m, 22H), 1.16-1.14 (m, 6H), 1.10-1.08 (m, 6H) ppm. ³¹P NMR (CDCl₃): d, 148.53, 148.46 ppm. It can be seen that the structure of the product was confirmed to be correct.

### (12) Synthesis of Lipid Compound Monomer (A20)

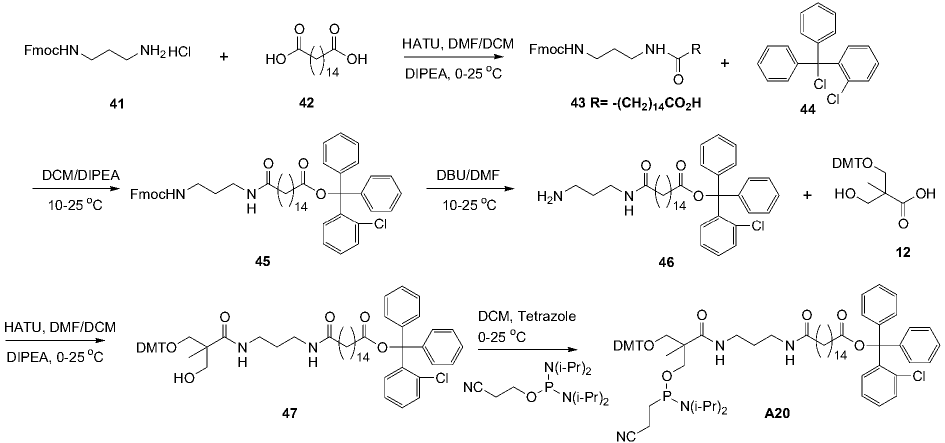

Hexadecanedioic acid 42 (0.286g, 1 mmol) was dissolved in 5 mL of a solvent mixture (dried dimethylformamide/dried dichloromethane=1.5:1, v/v) at room temperature. After complete dissolution, 2-(7-azabenzotriazol-1-yl)-N,N,N',N'-tetramethyluronium hexafluorophosphate (0.338g, 1 mmol) and N,N-diisopropylethylamine (0.4 mL, 4 mmol) were sequentially added under an ice bath, and the reaction solution was stirred thoroughly at 0°C for 20 minutes. After the intermediate was formed, N-(9-fluorenylmethoxycarbonyl)-1,3-propanediamine hydrochloride 41 (0.332g, 1 mmol) was dissolved in 2 mL of dichloromethane, and the resulting mixture was then added dropwise to a reaction flask. The reaction solution was warmed to room temperature (25°C) and further stirred at room temperature for 5 hours. The resulting mixture was extracted with ethyl acetate (2×50 mL). The organic phase was concentrated to semi-dryness by rotary evaporation and further purified and separated by silica gel column chromatography: The organic phase was eluted with a solvent mixture (ethyl acetate/dichloromethane/methanol=75:20:5, v/v). The product components were collected and evaporated to dryness under reduced pressure to obtain product 43 as a yellow solid (2.58g, 87%).

Chlorotrityl chloride 44 (1.6g, 5.1 mmol) was dissolved in 5 mL of dichloromethane. The solution was slowly added dropwise to a solution of compound 43 (2.58g, 4.57 mmol) in 10 mL of a solvent mixture (dichloromethane/N,N-diisopropylethylamine=8.4:1.6, v/v) at room temperature. The solution was stirred continuously at room temperature for 4 hours. The reaction solution was added into 50 mL of saturated brine, and the resulting mixture was extracted with ethyl acetate (2 × 50 mL). The solvent was evaporated under reduced pressure to obtain yellow solid 45 (5g, 58%). The product 45 was used directly in the next reaction.

Compound 45 (5g, 2.9 mmol) was dissolved in 10 mL of dry dimethylformamide (DMF). The solution was slowly added dropwise to 1,8-diazabicyclo[5.4.0]undec-7-ene (DBU) (1 mL) at room temperature. The solution was stirred continuously at room temperature for 1 hours. The reaction solution was added into 50 mL of saturated brine, and the resulting mixture was extracted with ethyl acetate (2 × 50 mL). The solvent was evaporated under reduced pressure to obtain yellow solid 46 (4.7g, 95%), and the crude product 46 was used directly in the next reaction.

The crude product, compound 46, (4.7g, 7.6 mmol) was dissolved in 20 mL of dimethylformamide At 0°C, the reaction solution was mixed with 3-O-(4,4'-dimethoxytrityl)-2-hydroxy-2-methylpropanoic acid 12 (2.3g, 5.3 mmol), and 2-(7-azabenzotriazol-1-yl)-N,N,N',N'-tetramethyluronium hexafluorophosphate (1.7g, 5.8 mmol) and N,N-diisopropylethylamine (3 mL) were then added. The mixture was stirred for 20 minutes. The temperature of the reaction solution was slowly raised to room temperature, and the mixture was further stirred for 2 hours. The reaction solution was mixed with 20 mL of saturated brine and extracted with ethyl acetate (2 × 50 mL). The organic phase was dried over anhydrous sodium sulfate and concentrated under reduced pressure by rotary evaporation to obtain crude product 47. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain compound 47 (1.4g, 56%).

Compound 47 (1.4g, 1.35 mmol) was dissolved in 20mL of dry dichloromethane, and N,N,N',N'-tetraisopropyl-2-cyanoethoxy phosphite (0.61 mL, 2.03 mmol) was then added rapidly to the solution. The reaction solution was stirred at room temperature for 20 minutes under nitrogen atmosphere. 20 mL of a solution of tetrazole (2.7 mL, 0.45M, 1.22 mmol) in dry dichloromethane was added to the above reaction solution, N,N,N',N'-tetraisopropyl-2-cyanoethoxy phosphite (0.1 mL, 0.33 mmol) was further added to the reaction solution, and the mixture was further stirred for reaction at room temperature under nitrogen atmosphere for 5 hours. The reaction solution was added into saturated brine, the mixture was extracted with dichloromethane (2 × 100 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A20 as a white solid (1.48g, 90%). The confirmed information for the product structure is as follows: ¹H NMR (CDCl₃): d, 7.42-7.40 (m, 2H, trityl), 7.30-7.18 (m, 22H, trityl), 6.84-6.81 (m, 4H, trityl), 3.79 (s, 6H), 3.76-3.69 (m, 3H), 3.68-3.51 (m, 2H), 3.29-3.22 (m, 4H), 3.14 (m, 2H), 2.55-2.51 (m, 2H), 2.17-2.14 (m, 2H), 2.05 (s, 1H), 1.67-1.61 (m, 2H), 1.29-1.16 (m, 30H), 1.16-1.14 (m, 6H), 1.11-1.08 (m, 6H) ppm. ³¹P NMR (CDCl₃): d, 148.53, 148.46 ppm. It can be seen that the structure of the product was confirmed to be correct.

### (13) Synthesis of Lipid Compound Monomers (A21 and B21)

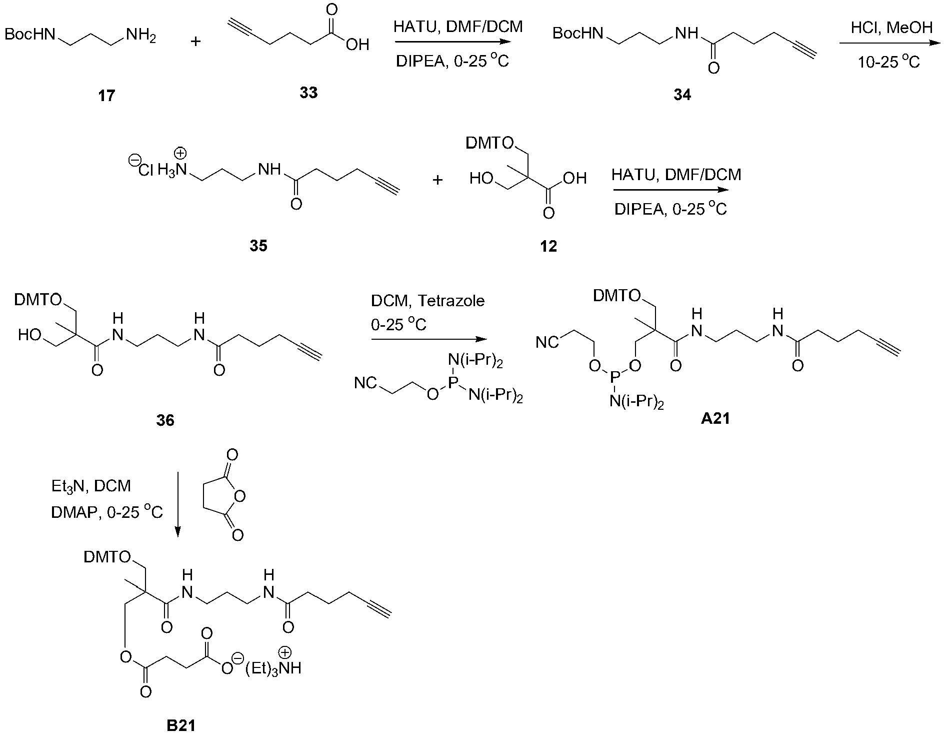

5-Hexynoic acid 33 (4.84g, 43.2 mmol) was dissolved in 50 mL of a solvent mixture (dried dimethylformamide/dried dichloromethane=1:1, v/v) at room temperature. After complete dissolution, 2-(7-azabenzotriazol-1-yl)-N,N,N,N'-tetramethyluronium hexafluorophosphate (16.5g, 43.2 mmol) and N,N-diisopropylethylamine (15 mL, 68.2 mmol) were sequentially added under an ice bath, and the reaction solution was stirred thoroughly at 0°C for 60 minutes. After the intermediate was formed, tert-butyl N-(2-aminoethyl)carbamate 17 (7.53g, 43.2 mmol) was dissolved in 50 mL of dichloromethane, and the resulting mixture was then added dropwise to a reaction flask. The reaction solution was warmed to room temperature (25°C) and further stirred at room temperature for 6 hours. The dichloromethane was removed under reduced pressure by rotary evaporation, the reaction solution was then added into 500 mL of saturated brine, and the resulting mixture was extracted with ethyl acetate (2 × 250 mL). Ethyl acetate was removed by rotary evaporation under reduced pressure to finally afford crude product 34 (5.5g, 93%). The crude product was dried under vacuum at room temperature and used directly in the next reaction.

Compound 34 (5.5g, 40.1 mmol) was dissolved in 50 mL of a solvent mixture (methanol/dichloromethane=1:1, v/v) and the reaction solution was mixed with 20 mL of 4M aqueous hydrochloric acid. The mixed reaction solution was stirred continuously at room temperature for 30 minutes, and then was further stirred at room temperature for 24 hours. After concentration by rotary evaporation, methanol and dichloromethane were removed. 100 mL of ethyl acetate was further added, and the reaction solution was mixed with ethyl acetate thoroughly and concentrated by rotary evaporation. The concentrated solution was mixed with 150 mL of a solvent mixture (n-hexane/ethyl acetate=1:1, v/v), and the solid product 35 gradually precipitated. After filtration and vacuum drying, the product 35 (5.1g, 90%) was used directly in the next reaction.

Compound 12 (11.8g, 20.0 mmol) was dissolved in 200 mL of a solvent mixture (dried dimethylformamide/dried dichloromethane=1:1, v/v) at room temperature. After complete dissolution, 2-(7-azabenzotriazol-1-yl)-N,N,N, N'-tetramethyluronium hexafluorophosphate (7.6g, 20.0 mmol) and N,N-diisopropylethylamine (7 mL, 23.6 mmol) were sequentially added under an ice bath, and the reaction solution was stirred thoroughly at 0°C for 15 minutes. After formation of the intermediate product, compound 35 (2.8g, 20.1 mmol) was dissolved in 50 mL of dichloromethane and the resulting mixture was then added dropwise to a reaction flask. The reaction solution was warmed to room temperature (25°C) and further stirred at room temperature for 12 hours. The dichloromethane was removed under reduced pressure by rotary evaporation, the reaction solution was then added into 500 mL of saturated brine, and the resulting mixture was extracted with ethyl acetate (2 × 250 mL). The organic phase was concentrated to semi-dryness by rotary evaporation and further purified and separated by silica gel column chromatography using one gradient elution: the organic phase was first eluted with n-hexane, then with n-hexane and ethyl acetate (n-hexane/ethyl acetate=1:2, v/v, containing 1% triethylamine), further with n-hexane and ethyl acetate (n-hexane/ethyl acetate=1:4, v/v, containing 1% triethylamine), and finally with ethyl acetate and 1% triethylamine. The product components were collected and evaporated to dryness under reduced pressure to obtain compound 36 as a yellow solid (8.8g, 59%).

Compound 36 (1.17g, 2.0 mmol) was dissolved in 25 mL of dry dichloromethane, and 1.4 mL of triethylamine was then added. 4-Dimethylaminopyridine (0.2g, 1.6 mmol) was dissolved in the reaction solution during stirring, succinic anhydride (0.3g, 3 mmol) was then dissolved in the reaction solution at room temperature with during, and the resulting mixture was stirred for reaction for 8 hours. Succinic anhydride (50 mg, 0.5 mmol) was further added, and the resulting mixture was further stirred at room temperature for 14 hours. The reaction solution was added into saturated brine, the mixture was extracted with ethyl acetate (2 × 250 mL), and the organic phase was separated, dried over anhydrous sodium sulfate, and evaporated to semi-dryness under reduced pressure. The residue was purified by silica gel column chromatography using one gradient elution: the residue was first eluted with a solvent mixture (ethyl acetate/triethylamine=100:1.5, v/v), and then with a solvent mixture (ethyl acetate/methanol/triethylamine= 100: 10: 1, v/v/v) to obtain the final product B21. The solvent was evaporated under reduced pressure to obtain compound B21 as a pale yellow solid (0.97g, 81%). The confirmed information for the product structure is as follows: ¹H NMR (CDCl₃): d, 7.39-7.37 (m, 2H, trityl), 7.30-7.27 (m, 6H, trityl), 7.23-7.21 (m, 1H, trityl), 6.84-6.81 (m, 4H, trityl), 3.79 (s, 6H), 3.28-3.20 (m, 4H), 3.03-3.00 (m, 4H), 2.53-2.51 (m, 4H), 2.33-2.30 (m, 2H), 2.25-2.22 (m, 2H), 1.97 (m, 1H), 1.87-1.83 (m, 2H), 1.58-1.54 (m, 2H), 1.28-1.23 (m, 5H) ppm. It can be seen that the structure of the product was confirmed to be correct.

Compound 36 (6.5g, 11.1 mmol) was dissolved in 80 mL of dry dichloromethane, and N,N,N',N'-tetraisopropyl-2-cyanoethoxy phosphite (4.0 mL, 13.3 mmol) was then added rapidly to the solution. The reaction solution was stirred under an ice bath for 20 minutes under nitrogen atmosphere. 23.4 mL of a solution of tetrazole (10.5 mmol) in dry dichloromethane was added to the above reaction solution, and the mixture was further stirred for reaction in an ice bath under nitrogen atmosphere for 3 hours. The reaction solution was added into saturated brine, the mixture was extracted with ethyl acetate (2 × 150 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane, then with n-hexane and ethyl acetate (n-hexane/ethyl acetate=3:1, v/v, containing 1% triethylamine), further with n-hexane and ethyl acetate (n-hexane/ethyl acetate=1:1, v/v, containing 1% triethylamine), and finally with n-hexane and ethyl acetate (n-hexane/ethyl acetate=1:2, v/v, containing 1% triethylamine). The product components were collected and evaporated to dryness under reduced pressure to obtain product A21 as a white solid (7.4g, 80%). The confirmed information for the product structure is as follows: ¹H NMR (CDCl₃): d, 7.42-7.39 (m, 2H, trityl), 7.30-7.27 (m, 6H, trityl), 7.23-7.21 (m, 1H, trityl), 6.84-6.81 (m, 4H, trityl), 3.79 (s, 6H), 3.78-3.76 (m, 2H), 3.71-3.68 (m, 2H), 3.29-3.23 (m, 4H), 3.17-3.15 (m, 2H), 2.56-2.52(m, 2H), 2.32-3.22 (m, 4H), 1.96-1.95 (m, 1H), 1.87-1.84 (m, 2H), 1.71 (s, 1H), 1.53 (m, 2H), 1.28-1.26 (m, 5H), 1.16-1.12 (m, 6H), 1.10-1.08 (m, 6H) ppm. ³¹P NMR (CDCl₃): d, 148.62, 148.54 ppm. It can be seen that the structure of the product was confirmed to be correct.

### (14) Chemical Synthesis of Lipid Compound Monomer (A22)

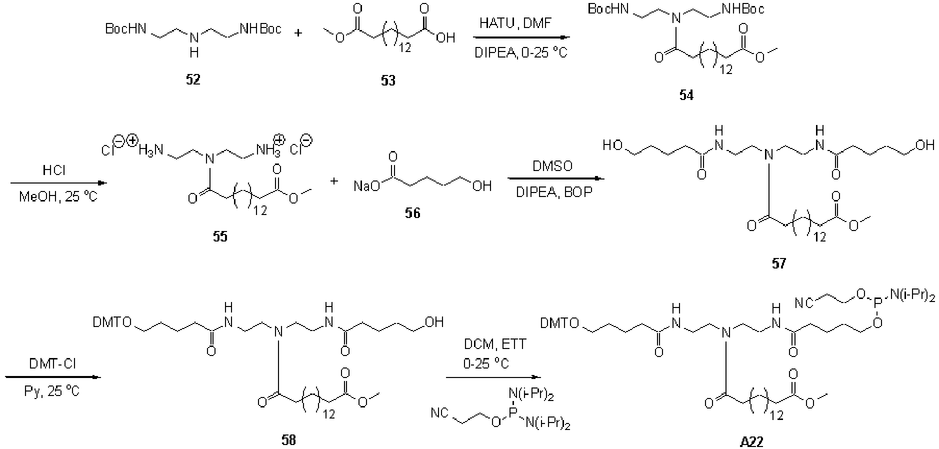

Monomethyl hexadecanedioate 53 (6g, 20 mmol) was dissolved in 20 mL of a solvent mixture (dried dimethylformamide) at room temperature. After complete dissolution, 2-(7-azabenzotriazol-1-yl)-N,N,N',N'-tetramethyluronium hexafluorophosphate (8g, 40 mmol) and N,N-diisopropylethylamine (7 mL, 40 mmol) were sequentially added under an ice bath, and the reaction solution was stirred thoroughly at 0°C for 20 minutes. After formation of the intermediate product, 1,7-Bis-Boc-1,4,7-triazaheptane 52 (6.2g, 20 mmol) was dissolved in 10 mL of dimethylformamide and the resulting mixture was then added dropwise to a reaction flask. The reaction solution was warmed to room temperature (25°C) and further stirred at room temperature for 10 hours. The reaction solution was added into 100 mL of saturated brine, and the resulting mixture was then extracted with ethyl acetate (2 × 150 mL). The solvent was evaporated under reduced pressure to obtain yellow solid 54 (12g, 85%). The crude product may be directly used for the next reaction.

Compound 54 (12g, 20 mmol) was dissolved in 50 mL of methanol. The reaction solution was mixed with 30 mL of 4 M aqueous hydrochloric acid. The mixed reaction solution was stirred continuously at room temperature for 60 minutes. After concentration by rotary evaporation, 50 mL of ethyl acetate was added, and the mixture was further concentrated by rotary evaporation to remove methanol and excess water, to obtain viscous crude product 55 (9g, 90%). The crude product was used directly in the next reaction.

The crude product compound 55 (3.73g, 8.14 mmol) and 5-hydroxypentanoic acid (2.85g, 20.35 mmol) were dissolved in 50 mL of dimethyl sulfoxide (DMSO), and the mixture was stirred at room temperature for 20 minutes. Benzotriazol-1-yloxytris(dimethylamino)phosphonium hexafluorophosphate (BOP, 9g, 20.3 mmol) and N,N-diisopropylethylamine (3 mL, 40.6 mmol) were added to the reaction solution, and the mixture was further stirred at room temperature for 4 hours. The reaction solution was mixed with 50 mL of water to obtain a white precipitate. After washing with water and filtration, product 57 (3.6g, 76%) was obtained. The crude product was directly used for the next reaction.

Compound 57 was eluted with pyridine, then evaporated twice and dried under vacuum for later use. 2-Chlorotrityl chloride (2g, 6 mmol) was dissolved in 5 mL of pyridine. The solution was slowly added dropwise to a solution of compound 57 (3.6g, 6.1 mmol) in 20 mL of pyridine at room temperature. The solution was stirred continuously at room temperature for 4 hours. The reaction solution was added into 50 mL of saturated brine, and the resulting mixture was extracted with ethyl acetate (2 × 100 mL). The solvent was evaporated under reduced pressure to obtain crude product 58 as a yellow solid. The crude product was further purified and separated by silica gel column chromatography using one gradient elution: the crude product was first eluted with a solvent mixture (ethyl acetate/methanol=3:1, v/v), further with a solvent mixture (ethyl acetate/methanol=5:1, v/v), and finally with a solvent mixture (ethyl acetate/methanol=10:1, v/v). The product components were collected and evaporated to dryness under reduced pressure to obtain compound 58 (2.2g, 41%).

Compound 58 (2.1g, 2.36 mmol) was dissolved in 30 mL of dry dichloromethane, and N,N,N',N'-tetraisopropyl-2-cyanoethoxy phosphite (926 mg, 3.07 mmol) was then added rapidly to the solution. The reaction solution was stirred at room temperature for 20 minutes under nitrogen atmosphere. 5 mL of a solution of thioethyltetrazole (ETT, 307 mg, 2.36 mmol) in dry dichloromethane was added to the above reaction solution, and the mixture was further stirred for reaction at room temperature under nitrogen atmosphere for 2 hours. The reaction solution was added into saturated brine (25 mL) and an aqueous solution of saturated sodium bicarbonate (25 mL), the mixture was extracted with ethyl acetate (2 × 150 mL), and the organic phase was separated, dried over anhydrous sodium sulfate, and evaporated to semi-dryness under reduced pressure. The residue was further purified and separated by silica gel column chromatography using one gradient elution: the residue was first eluted with n-hexane and ethyl acetate (n-hexane/ethyl acetate=1:1, v/v, containing 1% triethylamine), then with n-hexane and ethyl acetate (n-hexane/ethyl acetate=0:1, v/v, containing 1% triethylamine), and finally with n-hexane and ethyl acetate (ethyl acetate/methanol=20:1, v/v, containing 1% triethylamine). The product components were collected and evaporated to dryness under reduced pressure to obtain product A22 as a nearly white solid (2g, 78%). The confirmed information for the product structure is as follows: ¹H NMR (CDCl₃): d, 7.42-7.40 (m, 3H, trityl), 7.30-7.27 (m, 6H, trityl), 7.23-7.21 (m, 1H, trityl), 6.84-6.82 (m, 4H, trityl), 4.12-4.11 (m, 1H), 3.81-3.80 (m, 1H), 3.78 (s, 6H), 3.59-3.55 (m, 4H), 3.49-3.47 (m, 3H), 3.40-3.42 (m, 2H), 3.41-3.40 (m, 7H), 3.06-3.04 (m, 3H), 2.64-2.59 (m, 2H), 2.31-2.29 (m, 4H), 2.28-2.16 (m, 4H), 1.70-1.58 (m, 8H), 1.29-1.23 (m, 28H), 1.17-1.15 (m, 8H) ppm. ³¹P NMR (CDCl₃): d, 147.32, 147.23 ppm. It can be seen that the structure of the product was confirmed to be correct.

### (15) Synthesis of Compounds C1-C14

The synthetic methods for compounds C1-C14 are similar, except that appropriate starting materials are selected for substitution based on structures of the compounds.

In this example, the preparation method is described by using compound C1 as an example. In this step, the solid support for compound C1 is prepared by linking conjugate molecules of compound B1 to the solid support.

The lipid compound monomer hemisuccinate (B1, 50 mg, 0.072 mmol) and 2-(7-azabenzotriazol-1-yl)-N,N,N',N'-tetramethyluronium hexafluorophosphate (10 mg, 0.026 mmol) were dissolved in 1.25 mL of anhydrous acetonitrile at room temperature. N,N-diisopropylethylamine (10 µL) was added to the reaction solution. After all reagents were dissolved, 125 mg of long-chain alkylamine controlled-pore glass (500 Å, native LCAA-CPG, Chemgenes, USA) was added to the reaction solution. Both the solid and liquid phases were rotated and stirred at 300 rpm at room temperature. After 2 hours of reaction, the residual solution was filtered out, and the solid support, long-chain alkylamine controlled-pore glass, was washed with acetonitrile three times (3 × 1 mL). 0.5 mL of a solution of capping reagent A (10% v/v, acetic anhydride) in tetrahydrofuran and 0.5 mL of a solution of capping reagent B (N-methylimidazole) in pyridine and acetonitrile (N-methylimidazole/pyridine/acetonitrile=15:10:75, v/v/v) were stirred rotationally with the long-chain alkylamine controlled-pore glass at room temperature for 1 hour. The reaction solution was filtered out, and the solid support, long-chain alkylamine controlled-pore glass, was rinsed with acetonitrile three times, and then dried under reduced pressure by using a vacuum oil pump for 2 hours. The solid support (603C, 130 mg), controlled-pore glass, was obtained. 8.3 mg of the solid support, long-chain alkylamine controlled-pore glass C1, was weighed, 100 mL of dichloromethane containing 3% trichloroacetic acid was added, and the mixture was stirred rotationally for 30 seconds and left standing for 1 minute. The supernatant was taken to measure the visible light absorption at 498 nm, and the absorbance was 0.309. The loading capacity of the lipid compound monomer C1 was calculated to be 53.25 µmol/g.

### Example 7

In this example, the siRNA conjugate structure was designed and synthesized based on the structure of the lipid compound shown in the foregoing examples.

This example relates to two designs: a double-stranded siRNA is conjugated to one lipid compound; or a double-stranded siRNA is linked to a single-stranded phosphorothioate oligonucleotide via conjugation with one lipid compound. The single-stranded phosphorothioate oligonucleotide comprises a single-chain phosphate oligonucleotide which is formed by thiolation of the double-bonded oxygen atom and/or single-bonded hydroxyl oxygen atom in the phosphate structural moiety.

The structures of the conjugates comprise the specific structures shown in Tables 3 to 5 above.

In the siRNA structures of this example, uppercase letters C, G, U and A represent the base composition of ribonucleotides; lowercase letters g, t, and a represent the base composition of 2'-deoxynucleotides; lowercase letter m indicates that the nucleotide adjacent to the right side of letter m is a 2'-methoxy modified nucleotide (i.e., the 2'-hydroxyl group on the pentose of the nucleotide is substituted with a methoxy group); the lowercase letter f indicates that the nucleotide adjacent to the left side of letter f is a 2'-fluoro modified nucleotide (i.e., the 2'-hydroxyl group on the pentose of the nucleotide is substituted with a fluorine atom); bold uppercase letters T, G and A represent 2'-O-methoxyethyl modified nucleotides; bold uppercase letter C represents a 5-methyl-2'-O-methoxyethyl modified cytidine nucleotide; italic uppercase letter C represents a 5-methyl-2'-deoxy modified cytidine nucleotide; the lowercase letter s indicates that two nucleotides adjacent to letter s are linked by a phosphorothioate diester bond (i.e., the non-bridging oxygen atom in the phosphodiester bond is replaced by a sulfur atom); where no additional letter is present between two adjacent nucleotides, the two nucleotides are linked via a phosphodiester bond; ss represents the sense strand, and as represents the antisense strand.

As reported in the literature, feasible structures at present may adopt a loop complementary structure, and the synthesis is generally performed via conventional solid-phase synthesis.

This example relates to a method for preparing the nucleic acid-lipid chain conjugate described above. The preparation process of the nucleic acid conjugate refers to the cyclic reaction shown below (this reaction is only intended for illustrating the principle of the method in the present disclosure, and the preparation method in the present disclosure is not limited thereto. A person skilled in the art can prepare the conjugate according to the present disclosure by appropriately adjusting the preparation method according to the disclosure of the present disclosure).

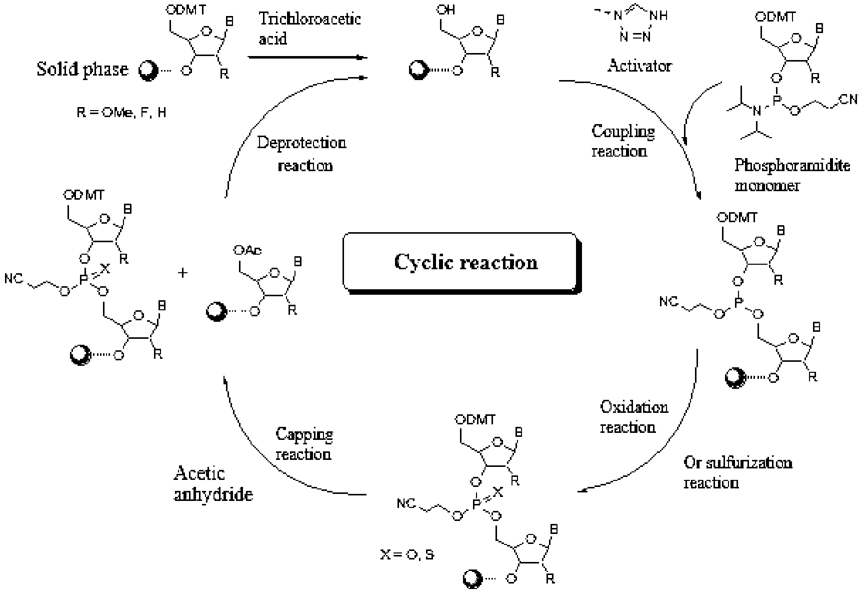

In the above figure, B is selected from formula (B1), (B2), or (B3), and DMT represents a dye. Therefore, in some embodiments of the present disclosure, the intermediate of the lipid compound monomer of series A has any one of the structures represented by the formulae below, thereby forming a monomer that may be used to synthesize the nucleic acid-containing conjugate: 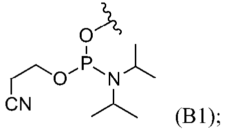 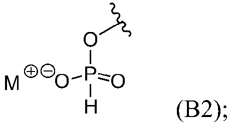 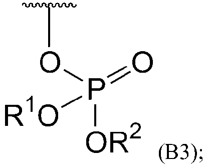 where M in formula (B2) is selected from one of TEA (triethylamine), trimethylamine, triisopropylamine, and tripropylamine. In formula (B3), R¹ and R² may each independently be selected from 2,2,2-trichloroethyl, a phenyl group, an o-chlorophenyl group, and a cyanoethyl group. In formulae (B1), (B2), and (B3), 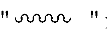 represents a site that may be covalently linked to a lipid compound (for example, L1 to L36).

For the method for preparing the nucleic acid conjugate according to the present disclosure, the solid-phase phosphoramidite synthesis method was used based on the nucleic acid to be prepared, for example an RNA sequence. Modified or unmodified RNA phosphoramidites are commercially available unless otherwise specified.

As an embodiment, the solid support required for solid-phase synthesis of nucleotides is selected from commercially available universal solid supports, for example NittoPhase^{®} HL UnyLinker^{™} 300 Oligonucleotide Synthesis Support (Kinovate Life Sciences), or long-chain alkylamine controlled-pore glass (CPG), (500Å, 1000Å, Chemgenes). 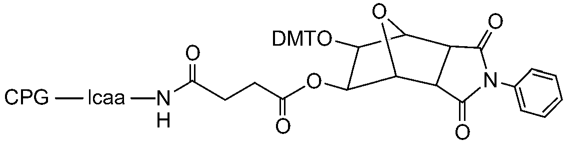 Universal Linker Conjugated to Controlled Pore Glass 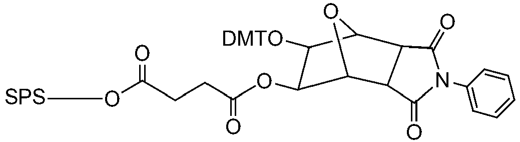 NittoPhase^{®} HL UnyLinker^{™} 300

CPG is the abbreviation for controlled pore glass; LCAA is the abbreviation for long-chain alkylamine; and SPS is the abbreviation for sodium 3,3'-dithiodipropane sulfonate.

The loading capacity of the solid support is generally expressed as the micromoles of compound that may be coupled and loaded per gram of solid support (30-200 µmol/g).

In the preparation method of the nucleic acid conjugate of the present disclosure, based on the phosphoramidite solid-phase synthesis strategy, the cyclic reactions as described above are adopted. Based on the nucleic acid sequence, nucleoside monomers or lipid compound monomers are sequentially coupled in the 3' to 5' direction. The coupling of each nucleoside monomer or lipid compound monomer includes four reaction steps: deprotection, coupling, capping, and oxidation (see the cyclic reaction described above).

In this example, the preparation of a conjugate (No. SN-16983) containing the sense and antisense sequences of rat SOD1 siRNA was used as an example to illustrate the preparation method of the conjugate.

In this example, the sequence of the mouse SOD1-targeted siRNA (No. SN-16983) is shown below, where "ss" represents the sense strand and "as" represents the antisense strand:
Sense strand (ss): 
5'-mCsmAsmUmUmUmUAfmAUfCfCfmUmCmAmCmUmCmUmAsmAsmA-3'-
L3; and
Antisense strand (as): 
5'-
mUsUfsmUmAmGAfmGUfGfmAmGmGmAUfmUAfmAmAmAmUmGsmAsmG-3';
where uppercase letters C, G, U and A represent the bases of ribonucleotides; the lowercase letter m indicates that the nucleotide immediately adjacent to the right of letter m is a 2'-methoxy-modified nucleotide; the lowercase letter f indicates that the nucleotide adjacent to the left of letter f is a 2'-fluoro-modified nucleotide; the lowercase letter s indicates that two nucleotides adjacent to letter s are linked via a phosphorothioate diester bond; the linkage between two adjacent nucleotides without an additional intervening letter is a phosphodiester bond. L3 represents the structural moiety of the lipid chain conjugate, and the specific structure is detailed in the foregoing examples.

For the method for preparing the nucleic acid conjugate in this example, the nucleic acid conjugate was prepared via the phosphoramidite solid-phase synthesis method in which nucleoside monomers or lipid compound monomers were sequentially coupled in the 3' to 5' direction according to the above sequence. The coupling of each nucleoside monomer or lipid compound monomer comprised four steps: deprotection, coupling, capping, and oxidation.

Specifically, the preparation method of the solid-phase synthesis reagents was as follows:
The deprotection reagent was a solution of trichloroacetic acid or dichloroacetic acid in dichloromethane (3%, v/v). Nucleoside monomers were dissolved in anhydrous acetonitrile at a concentration of 0.05M to 0.1M, and an appropriate amount of 3Å molecular sieves were added for drying. The coupling activator was a solution of 5-ethylthio-1H-tetrazole in anhydrous acetonitrile at a concentration of 0.25M or 0.45M. 1H-tetrazole, 5-benzylthio-1H-tetrazole, or 4,5-dicyanoimidazole may also be selected as the activator. Specifically, capping reagent A was a solution of acetic anhydride in tetrahydrofuran (10% v/v), and capping reagent B was a mixed solution of N-methylimidazole in pyridine and acetonitrile (15:10:75, v/v/v). Specifically, the oxidizing reagent was a solution of iodine in water and pyridine (0.05M, containing 95% aqueous pyridine). The sulfurizing reagent was a solution of (t-butylformyl)amino-3H-1,2,4-dithiazoline-3-thione in pyridine and acetonitrile at a concentration of 0.05M. The cleavage and deprotection reagent was 28% concentrated ammonia water.

Specifically, the reaction conditions and procedure for solid-phase synthesis were as follows:
On the synthesizer, the molar ratio of the 4,4'-dimethoxytrityl protecting group on the solid support or the nucleoside monomer bound to the support to a solution of trichloroacetic acid in dichloromethane (3%, v/v) was 1:30. The solid-phase reaction was carried out at room temperature for 1.5 minutes, and the operation was repeated three times. The addition of the deprotection solution was stopped when the eluate of the solid support changed from red to colorless. After repeated washing with anhydrous acetonitrile, the nucleoside monomer (or A6-R3, which is the lipid compound monomer used for the synthesis of L3) and the coupling activator were added at a ratio of 1:1. The molar ratio of the solid support to the nucleoside monomer was 1:5 to 1:6. Each reaction cycle of the reagent and the solid state was performed at room temperature for 3 to 4 minutes, and the reaction was terminated after two cycles. After washing with anhydrous acetonitrile, the oxidizing reagent solution was added, and the molar ratio of the solid support to the oxidizing agent was 1:6. The oxidation reagent reacted with the solid support at room temperature for approximately 2 minutes, and the reaction was carried out twice. After the coupling reaction, if a sulfurization step was required, the sulfurizing reagent solution was added, and the molar ratio of the solid support to the sulfurizing agent was 1:6. The oxidation reagent reacted with the solid support at room temperature for approximately 4 to 5 minutes, and the reaction was carried out twice. For the capping reaction, the capping reagents were added, and the molar ratio of the solid support to the capping reagent was 1:80. The capping reagent reacted with the solid support at room temperature for approximately 1 to 2 minutes, and the reaction was carried out twice. The foregoing steps of deprotection, coupling, oxidation, and capping were repeated cyclically until the coupling of the last nucleotide was completed. The solid support carrying the sense or antisense strand of the nucleic acid sequence was transferred to a vial, followed by the addition of 28% aqueous ammonia solution. The vial was tightly sealed and heated to 55°C to hydrolyze and remove the base protecting groups on the sense or antisense strand and also cleave the sense or antisense strand from the solid support via hydrolysis. The reaction was maintained for 16 hours. The resulting small nucleic acid sequence solution was filtered and separated from the solid support. After concentration, the crude product of the small nucleic acid sequence strand was obtained.

Specifically, the purification, isolation, and desalination procedures for preparative high-performance liquid chromatography were as follows:
The small nucleic acids were purified by gradient elution with NaBr by using a preparative anion-exchange chromatography column (Source 15Q). Mobile phase A was 20 mM sodium phosphate (pH 8.0), mobile phase B was 20 mM sodium phosphate (pH 8.0), and 1M sodium bromide was added to an aqueous solution containing 10% acetonitrile. Column temperature was 65°C and the flow rate was 10 mL/min. The gradient elution started with the mobile phase A, followed by an increase in the percentage of mobile phase B from 0% to 20% over 12 minutes. Subsequently, the mobile phase B was increased from 20% to 50% over 15 minutes. The product eluate was collected, and fraction analysis and fraction pooling were performed. Desalination was carried out by using a reversed-phase chromatography purification column or by dialysis. After concentration and lyophilization, purified small nucleic acids were obtained. For the synthesized sense and antisense strands, the purity was determined by anion-exchange high-performance liquid chromatography (AX-HPLC), and the full-sequence molecular weight was measured and analyzed by reversed-phase liquid chromatography-mass spectrometry (LC-MS) to confirm the synthesized nucleic acid sequences.

Specifically, the annealing procedure was as follows:
The synthesized sense strand (ss strand) and antisense strand (as strand) were mixed at an equimolar ratio in physiological saline for injection in the foregoing method. The mixture was heated at 90°C for 5 minutes, then cooled slowly to room temperature, and stored in a refrigerator at 4°C for 12 hours to allow the formation of the double-stranded structure via hydrogen bonding, thereby obtaining the siRNA conjugate.

In the present disclosure, based on the principle and idea of the foregoing preparation method, all conjugates with desired structures in the present disclosure may be prepared in a similar manner, which is entirely feasible for a person skilled in the art.

### Example 8

In this example, the conjugate synthesized in Example 7 is further illustrated. In this example, conjugates with serial numbers of SN-16983 and SN-16996 were synthesized. Sequences of the conjugates are shown below, where "ss" represents the sense strand and "as" represents the antisense strand:
SN-16983:
   Sense strand (ss): 5'-mCsmAsmUmUmUmUAfmAUfCfCfmUmCmAmCmU mCmUmAsmAsmA-3'-L3';
   Antisense strand (as): 5'-mUsUfsmUmAmGAfmGUfGfmAmGmGmAUfmUAf mAmAmAmUmGsmAsmG-3';
SN-16996:
   Sense strand (ss): 5'-mCsmAsmUmUmUmUAfmAUfCfCfmUmCmAmCmU mCmUmAsmAsmA-L17'- TsAGsGAstsastsastststsCstsasCAsGCsT 3'; and
   Antisense strand (as): 5'-mUsUfsmUmAmGAfmGUfGfmAmGmGmAUfmUAfmA mAmAmUmGsmAsmG-3'.

The monomers (phosphoramidites) required for synthesis are:
mC represents 2'-O-methylcytidine phosphoramidite, for example, CAS: 199593-09-4,
mG represents 2'-O-methylguanosine phosphoramidite, for example, CAS: 150780-67-9,
mA represents 2'-O-methyladenosine phosphoramidite, for example, CAS: 11-782-31-5,
mU represents 2'-O-methyluridine phosphoramidite, for example, CAS: 110764-79-9,
fC represents 2'-fluorocytidine phosphoramidite, for example, CAS: 159414-99-0,
fG represents 2'-fluoroguanosine phosphoramidite,
fA represents 2'-fluoroadenosine phosphoramidite, for example, CAS: 136834-22-5,
fU represents 2'-fluorouridine phosphoramidite,
t represents thymidine phosphoramidite, for example, CAS: 98796-51-1,
C represents 2'-methoxyethoxy 5-methylcytidine phosphoramidite, for example, CAS: 163759-94-2,
G represents 2'-methoxyethoxy guanosine phosphoramidite, for example, CAS: 251647-55-9,
A represents 2'-methoxyethoxy adenosine phosphoramidite, for example, CAS: 251647-53-7,
T represents 2'-methoxyethoxy thymidine phosphoramidite,
C represents 5-methylcytidine deoxyribonucleoside phosphoramidite, for example, 105931-57-5;
Lowercase letter a represents the base composition of 2'-deoxynucleotide, for example, dA amidite, CAS: 98796-53-3,
Lowercase letter g represents the base composition of 2'-deoxynucleotide, for example, dG amidite, CAS: 93183-15-4,
All the above monomers were purchased from Hongene Biotech.

The lipid compound monomer A6 (namely, L3 or L17) phosphoramidite synthesized in this example was prepared with reference to the corresponding method in Example 6.

The above monomers were synthesized by using an RNA/DNA automatic synthesizer (MerMode-12).

### Example 9

The structures of the conjugates synthesized in the foregoing examples were confirmed by the following instruments, equipment, and software:
Equipment: CTC autosampler, Agilent 1100 Series HPLC, and FINNIGAN LTQ MASS 004 mass spectrometer.

Software: LTQ Tune, Xcalibur, and ProMass for Xcalibur.

The relevant workflow is as follows.
2.1. Preparation of Mobile Phases
2.1.1. Reagent Specifications
   1) HPLC-grade H₂O (reagent water) and ACN (acetonitrile);
   2) Purity ≥ 99%: HFIP (Hexafluoroisopropanol), DIEA (N,N-Diisopropylethylamine), EDTA (Ethylenediaminetetraacetic acid), and ammonia water.
2.1.2. Mobile Phases A and B
2.1.2.1. EDTA Stock Solution 1mM: 1 mM EDTA + 1 mL of ammonia water in 1 L of 15% ACN/water (v:v);
2.1.2.2. Phase A: 750 µL of HFIP + 375 µL of DIEA + 10 mL of EDTA stock solution in 990 mL of water; and
2.1.2.3. Phase B: 750 µL of HFIP + 375 µL of DIEA + 10 mL of EDTA stock solution in 990 mL of 65% ACN/water (v:v).
2.2. Preparation of Sample Solution for Instrument Analysis

According to the LC-MS injection requirements, the sample was prepared at 0.5 OD/200 µL (approximately 0.014 nmol/µL).
2.3. Instrument Method Setup
2.3.1. Column Specification

XBridge Oligonucleotide BEH C18 Column 130Å, 2.5µm, 4.6 × 50mm.

### 2.3.2. Method Setup

Flow rate: 0.8 mL/min; injection volume: 10 µL;

| Gradient elution: | Time | A% | B% |
|---|---|---|---|
| | 0.0 | 10% | 90%; |
| | 2.0 | 10% | 90%. |

MS parameters: Polarity: Negative; Data type: Centroid; Ion source: ESI;
Scan range (m/z): 550-1600; Run time: 2 min;
Flow rate (arb) of sheath gas: 45;
Flow rate (arb) of Aux gas: 10;
Flow rate (arb) of sweep gas: 10;
I spray voltage {kV} I: 3.00;
Capillary temperature {°C}: 350.0;
Capillary voltage {V}: -35;
Tube lens {V}: -135.

In the present disclosure, the mass spectrometry data of the conjugates containing the sense strand and antisense strand of siRNA are shown below.

**Table 9**

| | |
|---|---|
| SN-16981 | ss: 5'-mCsmAsmUmUmUmUAfmAUfCfCfmUmCmAmCmUmCmUmAsmAsmA-3'-L1'; |
| | Calculated mass: 7296.16; found: 7293.9; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-16982 | ss: 5'-mCsmAsmUmUmUmUAfmAUfCfCfmUmCmAmCmUmCmUmAsmAsmA-3'-L2'; |
| | Calculated mass: 7310.19; found: 7307.9; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-16983 | ss: 5'-mCsmAsmUmUmUmUAfmAUfCfCfmUmCmAmCmUmCmUmAsmAsmA-3'-L3'; |
| | Calculated mass: 7324.21; found: 7326.1; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-16984 | ss: 5'-mCsmAsmUmUmUmUAfmAUfCfCfmUmCmAmCmUmCmUmAsmAsmA-3'-L4'; Calculated mass: 7338.24; found: 7340.6 |
| | as: 5'-mUsUfsmUmAmGAfmGUfGfmAmGmGmAUfmUAfmAmAmAmUmGsmAsmG-3'; Calculated mass: 7775.2; found: 7773.0; |
| SN-16985 | ss: 5'-mCsmAsmUmUmUmUAfmAUfCfCfmUmCmAmCmUmCmUmAsmAsmA-3'-L6'; Calculated mass: 7225.08; found: 7223.7; |
| | as: 5'-mUsUfsmUmAmGAfmGUfGfmAmGmGmAUfmUAfmAmAmAmUmGsmAsmG-3'; Calculated mass: 7775.2; found: 7773.0; |
| SN-16986 | ss: 5'-mCsmAsmUmUmUmUAfmAUfCfCfmUmCmAmCmUmCmUmAsmAsmA-3'-L7'; Calculated mass: 7310.19; found: 7312.4 |
| | as: 5'-mUsUfsmUmAmGAfmGUfGfmAmGmGmAUfmUAfmAmAmAmUmGsmAsmG-3'; Calculated mass: 7775.2; found: 7773.0; |
| SN-16987 | ss: 5'-mCsmAsmUmUmUmUAfmAUfCfCfmUmCmAmCmUmCmUmAsmAsmA-3'-L8'; Calculated mass: 7338.23; found: 7337.9; |
| | as: 5'-mUsUfsmUmAmGAfmGUfGfmAmGmGmAUfmUAfmAmAmAmUmGsmAsmG-3'; Calculated mass: 7775.2; found: 7773.0; |
| SN-16988 | ss: 5'-mCsmAsmUmUmUmUAfmAUfCfCfmUmCmAmCmUmCmUmAsmAsmA-3'-L9'; Calculated mass: 7352.26; found: 7351.7; |
| | as: 5'-mUsUfsmUmAmGAfmGUfGfmAmGmGmAUfmUAfmAmAmAmUmGsmAsmG-3'; Calculated mass: 7775.2; found: 7773.0; |
| SN-16995 | ss:5'-TsAGsGAstsastsastststsCstsasCAsGCsT-L17'- mCsmAsmUmUmUmUAfmAUfCfCfmUmCmAmCmUmCmUmAsmAsmA-3'; |
| | Calculated mass: 14501.9; found: 14502.1; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-16996 | ss:5'-mCsmAsmUmUmUmUAfmAUfCfCfmUmCmAmCmUmCmUmAsmAsmA-L17'- TsAGsGAstsastsaststststsCstsasCAsGCsT 3'; |
| | Calculated mass: 14501.9; found: 14502.5; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-16998 | ss:5'-mCsmAsmUmUmUmUAfmAUfCfCfmUmCmAmCmUmCmUmAsmAsmA-L17'- CsCsGsTsCsgsCsCsCststsCsasgsCsAsCsGsCsA-3'; |
| | Calculated mass: 14571.2; found: 14571.3; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-17002 | ss: 5'-mCsmAsmUmUmUmUAfmAUfCfCfmUmCmAmCmUmCmUmAsmAsmA-3'-L16'; |
| | Calculated mass: 7356.2; found: 7353.8; |
| | as: 5'-mUsUfsmUmAmGAfmGUfGfmAmGmGmAUfmUAfmAmAmAmUmGsmAsmG-3'; |
| | Calculated mass: 7775.2; found: 7776.4; |
| SN-17003 | ss:5'-mCsmAsmUmUmUmUAfmAUfCfCfmUmCmAmCmUmCmUmAsmAsmA-L17'- CsGsTsCsgsCsCsCststsCsasgsCsAsCsGsC-3'; |
| | Calculated mass: 13922.7; found: 13922.0; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-17004 | ss:5'-mCsmAsmUmUmUmUAfmAUfCfCfmUmCmAmCmUmCmUmAsmAsmA-L17'- GsTsCsgsCsCsCststsCsasgsCsAsCsG-3'; |
| | Calculated mass: 13136.0; found: 13136.0; |
| | as: 5'-mUsUfsmUmAmGAfmGUfGfmAmGmGmAUfmUAfmAmAmAmUmGsmAsmG-3'; |
| | Calculated mass: 7775.2; found: 7776.4; |
| SN-17005 | ss:5'-mCsmAsmUmUmUmUAfmAUfCfCfmUmCmAmCmUmCmUmAsmAsmA-L17'- TsCsgsCsCsCststsCsasgsCsAsC-3'; |
| | Calculated mass: 12297.3; found: 12297.3; |
| | as: 5'-mUsUfsmUmAmGAfmGUfGfmAmGmGmAUfmUAfmAmAmAmUmGsmAsmG-3'; |
| | Calculated mass: 7775.2; found: 7773.0; |
| SN-17006 | ss:5'-mCsmAsmUmUmUmUAfmAUfCfCfmUmCmAmCmUmCmUmAsmAsmA-L19'- GsTsCsgsCsCsCststsCsasgsCsAsCsG-3'; |
| | Calculated mass: 13170.0; found: 13164.9; |
| | as: 5'-mUsUfsmUmAmGAfmGUfGfmAmGmGmAUfmUAfmAmAmAmUmGsmAsmG-3'; |
| | Calculated mass: 7775.2; found: 7776.4; |
| SN-17011 | ss:	5'-mGsmCsmUmCmAmGmCfmAmUfmUfmGfmCmCmUmGmAmAmUmAmAmA-3'- L3'; |
| | Calculated mass: 7449.2; found: 7451.5; |
| | as:                                                                                                                    5'- mUsmUfsmUmAfmUmUfmCmAmGmGmCmAmAmUfmGmCfmUmGmAmGmCsmUsmU- 3'; |
| | Calculated mass: 7613.0; found: 7615.6; |
| SN-683081 | ss: 5'-mUsmGsmCmAmAmAmUfmAmGfmUfmCfmUmAmCmAmAmAmCmCsmAsmA-3'- L16'; |
| | Calculated mass: 7504.4; found: 7501.5; |
| | as:                                                                                                                                                    5'- |
| | mUsmUfsmGmGmUmUmUmGmUmAmGmAmCmUfmAmUfmUmUmGmCmAsmCsmA-3'; |
| | Calculated mass: 7638.1; found: 7640.1; |

### Example 10

In this example, biological assays were performed on siRNA and/or single-stranded phosphorothioate oligonucleotides, as well as the siRNA conjugates synthesized in Example 7, including *in vitro* assays, *in vivo* assays, and scanning imaging. In this example, the siRNA conjugates, siRNA, and/or single-stranded phosphorothioate oligonucleotides were dissolved in a solvent (PBS buffer; manufacturer: Absin; cat. no.: abs962) to prepare PBS solutions. The solutions may be used as injections. The solutions were used for cell experiments and rat experiments.

### (1) Residual SOD1 mRNA Levels in Rat B35 Cells

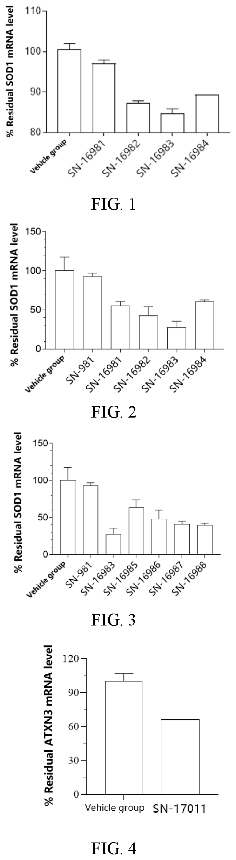

Different siRNA conjugates from Example 7 and the vehicle (PBS) were taken to test the residual SOD1 mRNA levels in rat B35 cells, respectively. The results are shown in FIG. 1.

12000 rat B35 cells were incubated overnight, followed by the addition of the siRNA conjugate to a final concentration of 2 µM, and the mixture was further incubated for 24 hours. After incubation, the cells were washed with PBS, and RNA was extracted as recommended by the RNeasy Mini Kit (Qiagen). Subsequently, RT-PCR was performed to generate cDNA based on the following reaction components and protocol.

**Table 10**

| Components | Volume (µL) |
|---|---|
| 100 mM dNTP | 0.8 |
| 50 U/µL Multiscribe reverse transcriptase | 1 |
| 10 × RT Buffer | 2 |
| 10 × RT random primers | 2 |
| Nuclease-free water | 4.2 |
| RNA sample | 10 |

The RT-PCR protocol was as follows: 25°C for 10 min → 37°C for 2h → 85°C for 5 min → hold at 4°C.

The resulting cDNA was subjected to TaqMan^{™} Fast qPCR, with 2 technical replicates per sample, according to the following reaction components.

**Table 11**

| Components | Volume (µL) |
|---|---|
| 2 × Taqman Fast Advanced Master Mix | 10 |
| SOD1 (20×) FAM (Rn00566938_m1) | 0.5 |
| HPRT1 (60×) VIC (Rn01527840_m1) | 0.17 |
| Nuclease-free water | 3.33 |
| cDNA | 1 |

qPCR was performed on QuantStudio^{™} 6 Pro (Thermo Fisher) with the following protocol:

40 ×
50°C 2 min → 95°C 20 s → 95°C 1 s → 60°C 20 s → hold at 4°C.

SOD1 gene expression levels were detected by qPCR. It can be seen that when the lipid compound has the structure of formula (I) and R'₁ is an alkyl chain, within the C₁₃ to C₁₆ alkyl chain range, the conjugate with R'₁ being a C₁₅ alkyl chain achieves the optimal intracellular delivery and the highest inhibition of mRNA.

### (2) Residual SOD1 mRNA Levels in the Brains of SD Rats

siRNA (SN-981), different siRNA conjugates from Example 7, and the vehicle (PBS) were taken to test the residual SOD1 mRNA levels in the brains of SD rats, respectively.

On day 0, SD rats received unilateral intracranial injection of vehicle (without siRNA or siRNA conjugates), or a PBS solution containing 0.9 mg of siRNA or siRNA conjugates. On day 7, RNA was extracted from brain tissues using TRIzol^{®} RNA Extraction Reagent (Thermo Fisher). cDNA was synthesized by the foregoing RT-PCR method, and rat SOD1 gene expression was determined by the foregoing qPCR method. Each group included 3 rats. The results are shown in FIG. 2.

SN-981, which has the sense strand and antisense strand shown below, is a double-stranded nucleic acid not conjugated with the lipid compound. SN-981 was used to indicate a case where the lipid compound according to the present disclosure was not used to deliver the nucleic acid.

**Table 12**

| | |
|---|---|
| SN-981 | ss: 5'-mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |

The experimental results demonstrate that compared with the case where nucleic acids are delivered without using the lipid compounds according to the present disclosure, delivery of the nucleic acids using the lipid compounds according to the present disclosure can significantly improve the intracellular delivery of nucleic acids and the inhibition of mRNA. When the lipid compound has the structure of formula (I), Q₁ and Q₄ are amide groups (-NHCO- or -CONH-), and R'₁ is an alkyl chain, within the C₁₃ to C₁₆ alkyl chain range, the conjugate with R'₁ being a C₁₅ alkyl chain achieves the optimal intracellular delivery and the highest inhibition of mRNA.

### (3) Residual SOD1 mRNA Levels in the Brains of SD Rats

siRNA (SN-981), different siRNA conjugates from Example 7, and the vehicle (PBS) were taken to test the residual SOD1 mRNA levels in the brains of SD rats, respectively.

On day 0, SD rats received unilateral intracranial injection of vehicle (without siRNA or siRNA conjugates) or a PBS solution containing 0.9 mg of siRNA or siRNA conjugates. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 3.

The experimental results demonstrate that when the lipid compound has the structure of formula (I), Q₁ and Q₄ are amide groups (-NHCO- or -CONH-), and R'₁ is an alkyl chain, within the C₂ to C₅ alkyl chain range, the C₃ alkyl chain exhibits the optimal effect, indicating that among the lipid compounds, the conjugate with three carbon atoms spacing the two amide groups in the left-side structure of the lipid compound achieves the optimal intracellular delivery and the highest inhibition of mRNA.

### (4) Residual ATXN3 mRNA Levels in the Brains of Mice

The siRNA conjugate from the above Example 7 and the vehicle (PBS) were taken for testing residual ATXN3 mRNA levels in the brains of mice, respectively. The specific structure of the siRNA conjugate is shown in Table 13 below.

**Table 13 Structure of siRNA Conjugate**

| | Sense strand | Antisense strand | Target gene |
|---|---|---|---|
| SN-17011 | 5'- mGsmCsmUmCmAmGmCfmAmU fmUfmGfmCmCmUmGmAmAmU mAmAmA-3'-L3' | 5'- mUsmUfsmUmAfmUmUfmCmAmGm GmCmAmAmUfmGmCfmUmGmAmG mCsmUsmU-3' | Mouse ATXN3 |

On day 0, mice received an intracranial injection of the vehicle (without SN-17011) or a PBS solution containing 0.5 mg of SN-17011. On day 30, mRNA was extracted from brain tissues by the foregoing TRIzol^{®} method. The expression level of the mouse ATXN3 gene was detected by the foregoing RT-PCR and qPCR methods. The probe for mouse ATXN3 was Mm00804702_m1, and the probe for mouse HPRT was Mm03024075_m1. Each group included 2 or 3 mice. The results are shown in FIG. 4. It can be seen that the conjugate formed by L3' achieves significant intracellular delivery and enhanced inhibition of mRNA at different targets.

### (5) Residual SOD1 mRNA Levels in the Brains of SD Rats

The vehicle (PBS) and the siRNA conjugate from Example 7 were taken to test the residual SOD1 mRNA levels in the brains of SD rats, respectively.

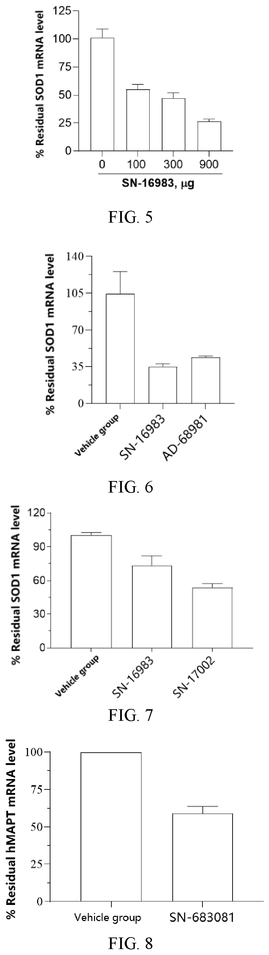

On day 0, SD rats received unilateral intracranial injection of vehicle (without SN-16983) or a PBS solution containing 0.1, 0.3, or 0.9 mg of SN-16983. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 5. It can be seen that the conjugate formed by L3' at various concentrations achieved significant intracellular delivery and enhanced inhibition.

### (6) Residual SOD1 mRNA Levels in the Brains of Rats

A known drug, the siRNA conjugate from the above Example 7, and vehicle (PBS) were taken for testing residual SOD1 mRNA levels in the brains of rats, respectively. The specific structures of the known drug and the siRNA conjugate are shown in Table 14 below.

**Table 14 Structures of Known Drug and Conjugate**

| | Sense strand | Antisense strand | Target gene |
|---|---|---|---|
| SN-16983 | 5'- mCsmAsmUmUmUmUAfmA UfCfCfmUmCmAmCmUmCm UmAsmAsmA-3'-L3'; | 5'- mUsUfsmUmAmGAfmGUfGf mAmGmGmAUfmUAfmAmA mAmUmGsmAsmG-3'; | Rat SOD1 |
| AD-68981 | 5'- CsAsUUU(Uhd)AfAUfCfCfU CACUCUAsAsA-3' | 5'- UsUfsUAGAfGUfGfAGGAUf UAfAAAUGsAsG-3' | Rat SOD1 |

On day 0, SD rats received an intracranial injection of the vehicle (without SN-16983 or AD-68981) or a PBS solution containing 0.9 mg of SN-16983 or AD-68981. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 6. It can be seen that the conjugate formed by L3' according to the present disclosure exhibits a better effect than the known drug (AD-68981 disclosed in US20220125823A1).

### (7) Residual SOD1 mRNA Levels in the Brains of Rats

Different siRNA conjugates from the above Example 7 and the vehicle (PBS) were taken to test the residual SOD1 mRNA levels in the brains of rats, respectively.

On day 0, SD rats were injected via the cisterna magna with the vehicle (without SN-16983 or SN-17002) or a PBS solution containing 0.6 mg of SN-16983 or SN-17002. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 7. It can be seen that when the lipid compound has the structure of formula (I), Q₁ and Q₄ are amide groups (-NHCO- or -CONH-), and R'₁ is an alkyl chain or a carboxy-substituted alkyl chain, the conjugate formed by the lipid compound further modified with -COOH on its alkyl chain achieves better intracellular delivery and better inhibition of mRNA.

### (8) Residual Human MAPT mRNA Levels in the Brains of Mice

The siRNA conjugate from the above Example 7 and the vehicle (PBS) were taken to test residual human MAPT mRNA levels in the brains of mice, respectively. The specific structure of the siRNA conjugate is shown in Table 15 below.

**Table 15 Structure of siRNA Conjugate**

| | Sense strand | Antisense strand | Target gene |
|---|---|---|---|
| SN-683081 | 5'- mUsmGsmCmAmAmAmUfmAm GfmUfmCfmUmAmCmAmAmAm CmCsmAsmA-3'-L16' | 5'- mUsmUfsmGmGmUmUmUmGmUmAm GmAmCmUfmAmUfmUmUmGmCmAs mCsmA-3' | Human MAPT |

On day 0, human MAPT transgenic mice received an intracranial injection of the vehicle (without SN-68081) or a PBS solution containing 500 mg of SN-68081. On day 30, mRNA was extracted from the brain tissue by the foregoing TRIzol^{®} method, and the expression level of the human MAPT gene was detected by the foregoing RT-PCR and qPCR methods, the probe for human MAPT was Hs00902194_m1 and the internal reference was the foregoing mouse HPRT. Each group included 2 or 3 mice. The results are shown in FIG. 8. It can be seen that the conjugate formed by L16' according to the present disclosure significantly improves intracellular delivery and mRNA inhibition for different nucleic acids at different targets.

### (9) Residual SOD1 mRNA Levels in the Brains of Rats

Different siRNA conjugates from the above Example 7 and the vehicle (PBS) were taken to test the residual SOD1 mRNA levels in the brains of rats, respectively.

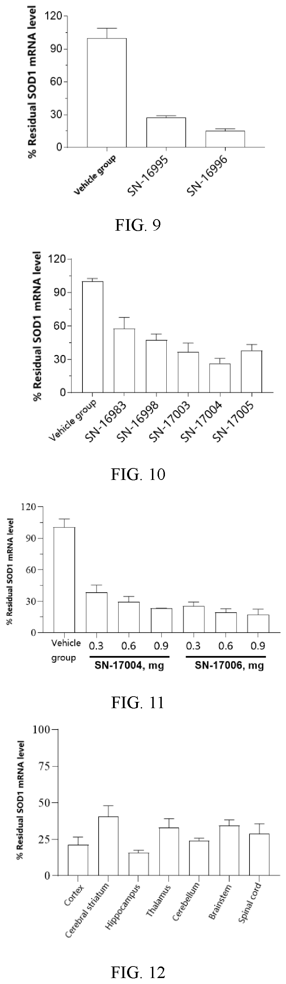

On day 0, SD rats received an intracranial injection of the vehicle (without SN-16995 or SN-16996) or a PBS solution containing 0.6 mg of SN-16995 or SN-16996. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 9. It can be seen that in the conjugates according to the present disclosure, the conjugation mode between the conjugate moiety and double-stranded nucleic acids, especially double-stranded siRNA and single-stranded phosphorothioate oligonucleotides, affects the functional efficacy. When one end of the conjugate moiety is conjugated to the sense strand of the double-stranded nucleic acid and the other end is conjugated to a single-stranded nucleic acid to form the sense strand of the nucleic acid conjugate, the conjugate with the single-stranded nucleic acid at the 3'-end of the sense strand exhibits superior intracellular delivery and higher mRNA inhibition efficiency, compared with the conjugate in which the single-stranded nucleic acid is at the 5'-end of the sense strand.

### (10) Residual SOD1 mRNA Levels in the Brains of Rats

Different siRNA conjugates from the above Example 7 and the vehicle (PBS) were taken to test the residual SOD1 mRNA levels in the brains of rats, respectively.

On day 0, SD rats were injected via the cisterna magna with the vehicle (without siRNA conjugates) or a PBS solution containing 0.6 mg of siRNA conjugates. On day 7, RNA was extracted from brain tissues by the foregoing TRIzol^{®} method, and rat SOD1 gene expression was determined by the foregoing RT-PCR and qPCR methods. Each group included 5 or 6 rats. The results are shown in FIG. 10. It can be seen that, in the conjugates according to the present disclosure, compared with conjugates only containing double-stranded nucleic acids such as siRNA double-stranded ribonucleic acid, the conjugates formed by conjugating both a double-stranded nucleic acid and a single-stranded phosphorothioate oligonucleotide via the lipid compound achieve better intracellular delivery and mRNA inhibition. Among the conjugates formed by conjugating both the double-stranded nucleic acid and the single-stranded phosphorothioate oligonucleotide via the lipid compound, within the range of 14-20 nucleotides, the single-stranded phosphorothioate oligonucleotide with 16 nucleotides achieves the optimal intracellular delivery and mRNA inhibition.

### (11) Residual SOD1 mRNA Levels in the Brains of Rats

Different siRNA conjugates from the above Example 7 and the vehicle (PBS) were taken to test the residual SOD1 mRNA levels in the brains of rats, respectively.

On day 0, SD rats received an intracranial injection of the vehicle (without siRNA conjugate) or the PBS solution containing 0.3, 0.6 or 0.9 mg of SN-17004 or SN-17006. On day 7, RNA was extracted from brain tissues by the foregoing TRIzol^{®} method, and rat SOD1 gene expression was determined by the foregoing RT-PCR and qPCR methods. Each group included 3 rats. The results are shown in FIG. 11. It can be seen that for conjugates formed by conjugating both the double-stranded nucleic acid and the single-stranded phosphorothioate oligonucleotide via the lipid compound, under the condition that the lengths and conjugation positions of the single-stranded phosphorothioate oligonucleotides are identical, when the lipid compound has the structure of formula (I), Q₁ and Q₄ are amide groups (-NHCO- or -CONH-), and R'₁ is an alkyl chain or a carboxy-substituted alkyl chain, the conjugate formed by the lipid compound further modified with -COOH on its alkyl chain achieves better intracellular delivery and better inhibition of mRNA.

### (12) Residual SOD1 mRNA Levels in Each Region of the Bains of Rts

The siRNA conjugate from the above Example 7 was taken to test the residual SOD1 mRNA levels in each region of the brains of rats.

On day 0, SD rats were injected via the cisterna magna with the PBS solution containing 0.9 mg of SN-17006. On day 14, RNA was extracted from each brain tissue by the foregoing TRIzol^{®} method, and rat SOD1 gene expression was determined by the foregoing RT-PCR and qPCR methods, with the vehicle group set as 100% for comparison. Each group included 3 rats. The results are shown in FIG. 12. It can be seen that the conjugates according to the present disclosure improve intracellular delivery and mRNA inhibition in different regions of the brain. Therefore, it is demonstrated that the conjugates according to the present disclosure can improve the intracellular delivery of nucleic acids and the inhibition of mRNA in various cells.

### (13) Residual SOD1 mRNA Levels in the Brains of Rats

SiRNA (SN-981), the single-stranded oligonucleotide (SN-17035), the siRNA conjugate from the above Example 7, and the vehicle (PBS) were taken to test the residual SOD1 mRNA levels in the brains of rats, respectively.

On day 0, SD rats received an intracranial injection of the vehicle (without SN-17006, SN-17035, or SN-981) or a PBS solution containing 0.6 mg of SN-17006, 0.6 mg of SN-17035, or 0.6 mg of SN-981. On day 7, RNA was extracted from brain tissues by the foregoing TRIzol^{®} method, and rat SOD1 gene expression was determined by the foregoing RT-PCR and qPCR methods. Each group included 3 rats.

SN-17035 is a single-stranded oligonucleotide having the following sequence:

**Table 16**

| | |
|---|---|
| SN-17035 | 5'-GsTsCsgsCsCsCststsCsasgsCsAsCsG-3' |

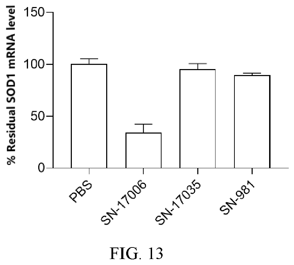

The results are shown in FIG. 13. Compared with intracellular delivery of a single double-stranded nucleic acid, the simultaneous delivery of both double-stranded and single-stranded nucleic acids is generally much more difficult to achieve. However, this example demonstrates that the lipid compound with the specific structure of the present disclosure can form a conjugate conjugated with both double-stranded and single-stranded nucleic acids. The conjugate significantly improves intracellular delivery and mRNA inhibition. Using the mRNA level (100.0 ± 5.2) corresponding to PBS as a reference, SN-17006 (mRNA level: 33.9 ± 8.6) reduced the mRNA level by approximately 66.1%, while SN-17035 (mRNA level: 95.5 ± 5.3) and SN-981 (mRNA level: 89.7 ± 2.0) only reduced the mRNA level by approximately 4.5% and 10.3%, respectively. Therefore, this example further demonstrates that the conjugate according to the present disclosure achieves a synergistic effect, which is superior to an effect of using the double-stranded nucleic acid or the single-stranded nucleic acid alone, that is, 1+1>2.

Obviously, the foregoing examples are merely for a purpose of clear explanation and are not intended to limit the embodiments. For a person of ordinary skill in the art, other modifications or changes in various forms can also be made on the basis of the above description. It is unnecessary and impossible to exhaustively enumerate all the embodiments herein. Obvious changes or modifications derived from the above Examples shall still fall within the protection scope of the present disclosure

## Claims

1. A lipid compound having a structure represented by formula (I), (II) or (V) below: 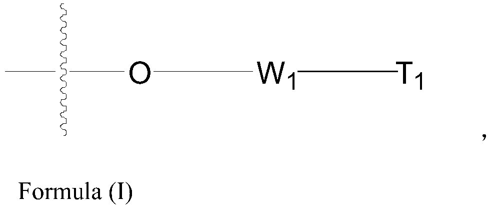
wherein W₁ is selected from a direct bond or 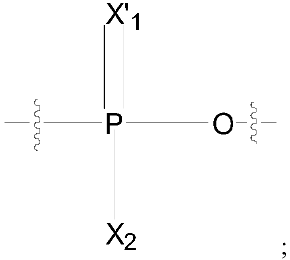
X'₁ is selected from an O or S atom, or is absent,
when X'₁ is selected from an O or S atom, X₂ is selected from -O-, -S-, -SH, -OH (hydroxyl group), -NH₂ (amino group), a C₁-C₆ alkyl group, a C₁-C₆ alkoxy group, or -O-(CH₂)_{n'}-OR'₅,
wherein R'₅ is selected from H, a direct bond or 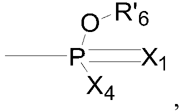 R'₆ is H or a direct bond, X₁ is selected from an O or S atom, X₄ is -OH or -SH, and n' is an integer from 1 to 10; or when X'₁ is absent, X₂ is a direct bond;
T₁ is selected from -(CH₂)ₘCH₃ or 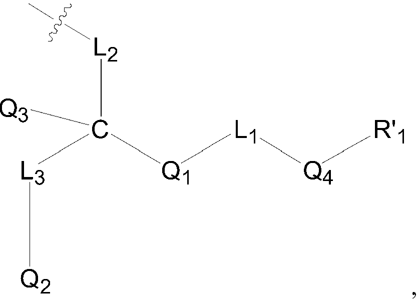 wherein m is an integer from 10 to 30,
wherein Q₁ and Q₄ are each independently selected from a direct bond, -NH₂ (amino group), - COOH (carboxyl group), an amide group (-NHCO- or -CONH-), -O-, -S-, -S-S-, a phosphate group, or a phosphorothioate group;
Q₂ is selected from -SH, -OH (hydroxyl group), -NH₂ (amino group), -H, or a C₁-C₆ alkyl group, preferably, -CH₃ (methyl group), -COOH (carboxyl group), an amide group (-NHCO- or - CONH-), -O-, -S-, -S-S-, a phosphate group, a phosphorothioate group, or 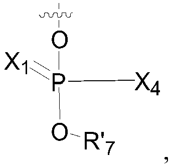
wherein R'₇ is H or a direct bond; and definitions of X₁ and X₄ are the same as defined above;
Q₃ is selected from -H or a C₁-C₁₀ alkyl group;
L₁ is -(CH₂)ₗ-(NR'₄)ₜ-(CH₂)_{q}-, wherein l and q are each an integer from 0 to 10, and l+q=1 to 10; t is 0 or 1; R'₄ is -CO(CH₂)ᵣCOOH; and r is an integer from 10 to 30;
L₂ and L₃ are each independently selected from a C₁-C₁₀ saturated alkyl chain or a direct bond;
R'₁ is selected from a C₁₀-C₃₀ saturated fatty acid chain, a C₁₀-C₃₀ saturated alkyl chain, a C₁₀-C₃₀ unsaturated hydrocarbon group, or -(CH₂)ₘ-X₃-R'₃, wherein m is an integer from 10 to 30;
X₃ is selected from a direct bond, an oxygen atom or a sulfur atom; R'₃ is selected from a saturated six-membered heterocyclic group containing nitrogen and oxygen, H, a direct bond, or 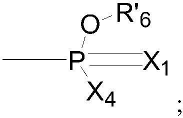 and definitions of R'₆, X₁ and X₄ are the same as defined above; or when
X₃ is a direct bond, R'₃ is not H or a direct bond;
when W₁ is a direct bond, T₁ is not -(CH₂)ₘCH₃; 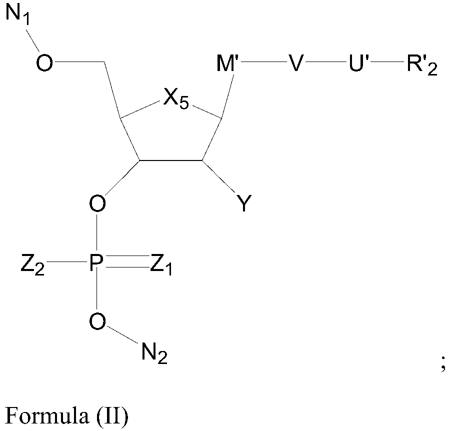 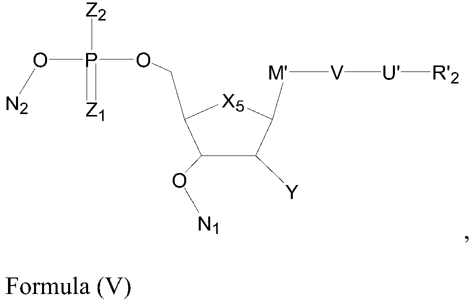
in formula (II) and formula (V), the five-membered ring is a five-membered sugar ring structure of ribose or deoxyribose, wherein X₅ is selected from -CH₂-, -CH(CH₃)-, -C(CH₃)₂-, -O-, -NH-, -N(CH₃)-, or -S-;
M' is selected from H, -O-, -C-, or a modified or unmodified nucleobase;
N₁ is selected from a direct bond, H, a C₁-C₃ alkyl group, or 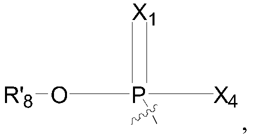 wherein R'₈ is H or a direct bond; and definitions of X₁ and X₄ are the same as defined above;
N₂ is selected from a direct bond, H, or a C₁-C₃ alkyl group;
Y is selected from H, -NH₂, -OH, halogen, a C₁-C₆ alkyl group, a C₁-C₆ haloalkyl group, -OR'₉, or -O-(CH₂)ₙ-O-R'₁₀, wherein R'₉ is a C₁-C₆ alkyl group, preferably -O-CH₃; n is an integer from 1 to 6; R'₁₀ a C₁-C₆ alkyl group, preferably, n is 2, and R'₁₀ is a C₁ alkyl group, namely 2'-methoxyethoxy group;
V is selected from a C₁-C₄ saturated alkyl chain or is absent;
U' is selected from -NH₂ (amino group), -COOH (carboxyl group), or an amide group (-NHCO- or -CONH-), or is absent;
Z₁ is selected from an O or S atom;
Z₂ is selected from a C₁₀-C₃₀ alkoxy group or a fatty acid chain, preferably, a fatty acid with a terminal carboxyl group, an amide lipid chain, an alkenyl chain, or an alkyl chain; and
R'₂ is selected from a C₁₀-C₃₀ alkoxy group or a fatty acid chain, preferably, a fatty acid with a terminal carboxyl group, an amide lipid chain, an alkenyl chain, or an alkyl chain; or is absent.

2. The lipid compound of claim 1, wherein the lipid compound has a structure represented by formula (I),
when W₁ is 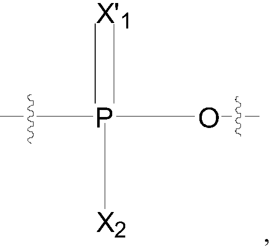 X'₁ is selected from an O or S atom;
T₁ is 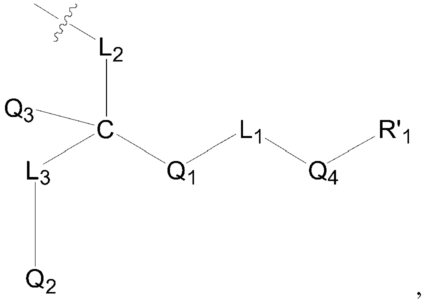
wherein:
Q₁ and Q₄ are selected from a direct bond, -NH₂ (amino group), -COOH (carboxyl group), an amide group (-NHCO- or -CONH-), -O-, -S-, -S-S-, a phosphate group, or a phosphorothioate group;
Q₂ is selected from -SH, -OH (hydroxyl group), -NH₂ (amino group), -H, or a C₁-C₆ alkyl group, preferably, -CH₃ (methyl group), -COOH (carboxyl group), an amide group (-NHCO- or -
CONH-), -O-, -S-, -S-S-, a phosphate group, a phosphorothioate group, or 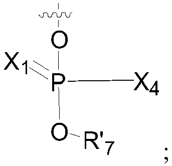
Q₃ is selected from -H or a C₁-C₁₀ alkyl group;
L₂ and L₃ are selected from a C₁-C₁₀ saturated alkyl chain or a direct bond;
X₁ is selected from an O or S atom;
X₂ is selected from -O-, -S-, -SH, -OH (hydroxyl group), -NH₂ (amino group), or a C₁-C₆ alkyl group, preferably, -CH₃ (methyl group), -CH₂CH₃ (ethyl group), or a C₁-C₆ alkoxy group, more preferably, -OCH₃ (methoxy group), -OCH₂CH₃ (ethoxy group), or -O-(CH₂)ₙ-OR'₅, wherein
R'₅ is selected from H, a direct bond, or 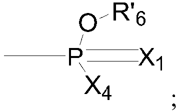 and
R'₁ is selected from a C₁₀-C₃₀ saturated fatty acid chain, a C₁₀-C₃₀ saturated alkyl chain, a C₁₀-C₃₀ unsaturated hydrocarbon group, or -(CH₂)ₘ-X₃-R'₃, wherein m is an integer from 10 to 30;
X₃ is selected from a direct bond, an oxygen atom or a sulfur atom; and R'₃ is a saturated six-membered heterocyclic group containing nitrogen and oxygen, H, a direct bond, or

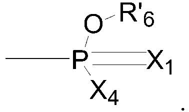

3. The lipid compound of claim 2, wherein both Q₁ and Q₄ are amide groups (-NHCO- or -CONH-); Q₂ is selected from -SH, -OH (hydroxyl group), -S-, -O-, or 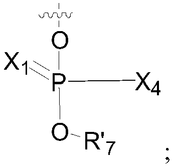 and R'₁ is selected from a C₁₀-C₃₀ saturated fatty acid chain or a C₁₀-C₃₀ saturated alkyl chain, preferably, a C₁₃-C₁₆ saturated alkyl chain, more preferably, a C₁₅ saturated alkyl chain, a C₁₀-C₃₀ unsaturated hydrocarbon group, or -(CH₂)ₘ-X₃-R'₃, wherein m is an integer from 10 to 30, preferably, an integer from 2 to 5, more preferably, m is 3; X₃ is selected from a direct bond, an oxygen atom or a sulfur atom; and R'₃ is a saturated six-membered heterocyclic group containing nitrogen and oxygen, H, a direct bond, or

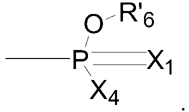

4. The lipid compound of claim 3, wherein L₁ is -(CH₂)ₗ-(NR'₄)ₜ-(CH₂)_{q}-, wherein t is 0.

5. The lipid compound of claim 3, wherein L₁ is -(CH₂)ₗ-(NR'₄)ₜ-(CH₂)_{q}-, wherein l+q=1 to 10, and t is 1;
L₂ is selected from a C₁-C₁₀ saturated alkyl chain or a direct bond;
L₃ is a direct bond;
Q₂ is H;
Q₃ is selected from -H or a C₁-C₁₀ alkyl group; and
R'₁ is -(CH₂)ₘ-X₃-R'₃, wherein m is an integer from 10 to 30; X₃ is selected from an oxygen atom or a sulfur atom; and R'₃ is H, a direct bond, or

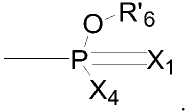

6. The lipid compound of claim 2, wherein Q₁ is a direct bond, and Q₄ is an amide group (-NHCO- or -CONH-);
L₁ is -(CH₂)ₗ-(NR'₄)ₜ-(CH₂)_{q}-, wherein l+q=1 to 10, and t is 0;
L₂ is a C₁-C₁₀ saturated alkyl chain;
L₃ is a direct bond; and
Q₂ is selected from -SH, -OH (hydroxyl group), -S-, -O-, or 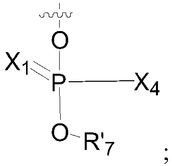 and definitions of R'₇ and X₄ are the same as defined above.

7. The lipid compound of any one of claims 1 to 6, wherein:
W₁ is a direct bond; Q₂ is selected from -SH, -OH (hydroxyl group), or 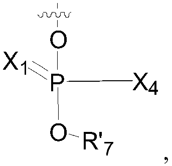
wherein R'₇ is H; and R'₆ in R'₁ is not a direct bond;
W₁ is a direct bond; Q₂ is selected from -S-, -O-, or 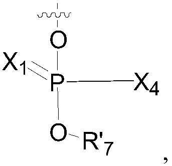 wherein R'₇ is a direct bond; and R'₆ in R'₁ is not a direct bond;
W₁ is a direct bond; Q₂ is not -S-, -O-, or -S-S-, and when Q₂ is 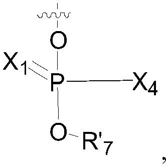 R'₇ is H; and
R'₁ is -(CH₂)ₘ-X₃-R'₃, wherein R'₃ is a direct bond or 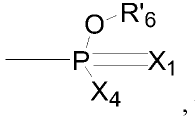 wherein R'₆ is a direct bond;
W₁ is 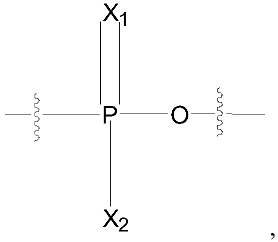 wherein X₂ is selected from -OH or -SH; Q₂ is selected from -SH or -OH (hydroxyl group); and R'₃ and R'₆ in R'₁ are not direct bonds;
W₁ is 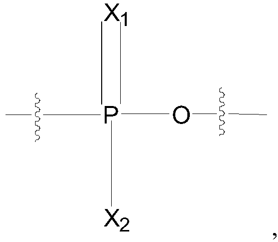 wherein X₂ is selected from -OH or -SH; Q₂ is selected from -S-, -O-, or 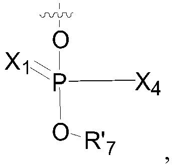 wherein R'₇ is a direct bond; and R'₃ and R'₆ in R'₁ are not direct bonds; or
W₁ is 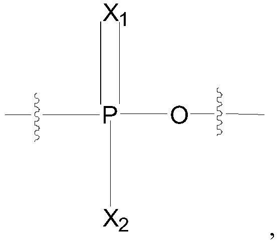 wherein X₂ is selected from -OH or -SH; Q₂ is not -S-, -O-, or -S-S-, and when Q₂ is 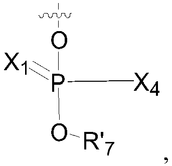 R'₇ is H; and R'₃ in R'₁ is a direct bond; or when R'₃ is 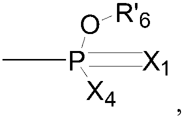 R'₆ is a direct bond;
preferably, W₁ is a direct bond; the wavy line in formula (I) is linked to X₆, and X₆ has the following structure 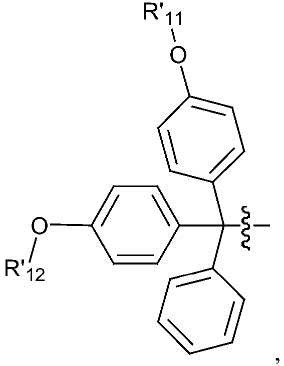 wherein R'₁₁ and R'₁₂ are each independently selected from a C₁-C₆ alkyl group; Q₁ is a direct bond or an amide group (-NHCO- or -CONH-), and Q₄ is an amide group (-NHCO- or -CONH-); Q₂ is selected from -S-, -O- or H; L₁ is - (CH₂)ₗ-(NR'₄)ₜ-(CH₂)_{q}-, wherein l and q each an integer from 0 to 10, l+q=1 to 10, and t is 0 or 1, when Q₂ is H, L₃ is a direct bond, and t is 1; or when Q₂ is -S- or -O-, Q₂ is linked to X₇, and X₇ is selected from (B'1) or (B'2), 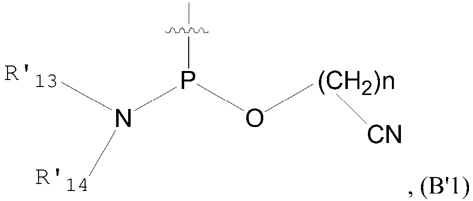
wherein R'₁₃ and R'₁₄ are each a C₁-C₆ alkyl group, preferably, a C₃ alkyl group, more preferably, an isopropyl group; and n is an integer from 1 to 6; 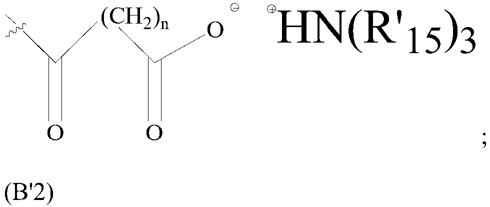
wherein R'₁₅ is a C₁-C₆ alkyl group, and n is an integer from 1 to 6; R'₁ is selected from a C₁₀-C₃₀ saturated alkyl chain, a C₁₀-C₃₀ unsaturated hydrocarbon group, or -(CH₂)ₘ-X₃-R'₃, wherein m is an integer from 10 to 30, X₃ is selected from a direct bond, an oxygen atom, or a sulfur atom, and R'₃ is selected from a saturated six-membered heterocyclic group containing nitrogen and oxygen, or a direct bond, and when Q₂ is H, R'₃ is a direct bond, and R'₃ is linked to 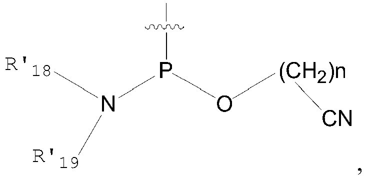 wherein R'₁₈ and R'₁₉ are each independently a C₁-C₆ alkyl group, and n is an integer from 1 to 6; or
preferably, W₁ is 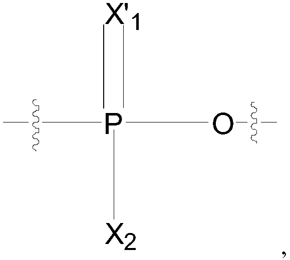 wherein X'₁ is absent, and X₂ is a direct bond; the wavy line in formula (I) is linked to -(CH₂)ₙ-O-X₆, wherein n is an integer from 1 to 6, and the definition of X₆ is the same as defined above; T₁ is -(CH₂)ₘCH₃; and X₂ is linked to - N(R'₂₀)₂, wherein R'₂₀ is a C₁-C₆ alkyl group, preferably, a C₃ alkyl group, more preferably, an isopropyl group.

8. The lipid compound of any one of claims 1 to 7, wherein in formula (I):
R'₁ is a C₁₀-C₃₀ saturated fatty acid chain, and the C₁₀-C₃₀ saturated fatty acid chain is -(CH₂)ₘ-COOH or -(CH₂)ₘ-COOR'₁₆, wherein m is an integer from 10 to 30; R'₁₆ is a C₁-C₆ alkyl group or 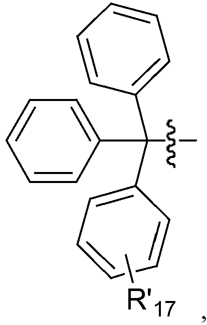 preferably, 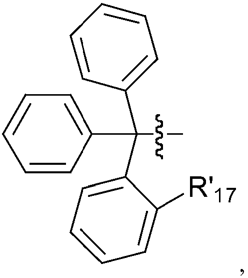 wherein the C₁-C₆ alkyl group is preferably a methyl group, an ethyl group, an isopropyl group or a tert-butyl group; and R'₁₇ is a halogen, preferably Cl;
R'₁ is a C₁₀-C₃₀ unsaturated hydrocarbon group, and the C₁₀-C₃₀ unsaturated hydrocarbon group is -(CH₂)ₘ-R'₄, wherein m is an integer from 10 to 30; and R'₄ is an unsaturated bond, preferably a triple bond; or
R'₁ is -(CH₂)ₙ-X₃-R'₃, wherein in -(CH₂)ₙ-X₃-R'₃, R'₃ is a saturated six-membered heterocyclic group containing nitrogen and oxygen, and X₃ is bonded to the nitrogen atom of R'₃, preferably, the six-membered heterocyclic ring in R'₃ contains one nitrogen atom and one oxygen atom, and the nitrogen atom and the oxygen atom are located at para positions of the six-membered heterocyclic ring; and/or
X₂ is selected from -OH, -SH, -CH₃ (methyl group), -CH₂CH₃ (ethyl group), -OCH₃ (methoxy group), or -OCHH₂CH₃ (ethoxy group), preferably, -OH or -SH.

9. The lipid compound of claim 1, wherein W₁ is 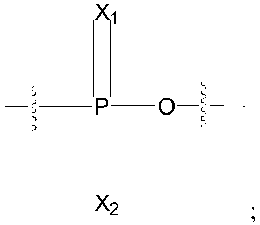 X'₁ is O or S; X₂ is -(CH₂)_{n'}-OR'₅, wherein R'₅ is selected from H, a direct bond, or 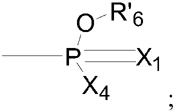 the definitions of X₁, R'₆ and X₄ are the same as defined above; n' is an integer from 1 to 10; and T₁ is -(CH₂)ₘCH₃, wherein m is an integer from 10 to 30.

10. The lipid compound of claim 9, wherein R'₅ is selected from a direct bond or 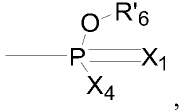 wherein R'₆ is a direct bond.

11. The lipid compound of any one of claims 1 to 10, wherein the wavy line in formula (I) is linked to H or X₆.

12. The lipid compound of claim 1, wherein the lipid compound has a structure represented by formula (II) or formula (V), wherein N₁ is a direct bond, H, or 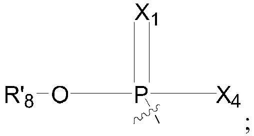
N₂ is selected from a direct bond or H;
Y is a C₁-C₆ alkoxy group;
M' is selected from -O-, -C-, or a modified or unmodified nucleobase,
when M' is a modified or unmodified nucleobase, U', V, and R'₂ are all absent, preferably, M' is independently selected from adenine, uracil, thymine, guanine, or cytosine, more preferably, M' is 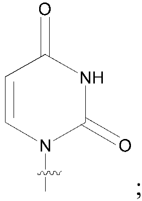 or
when M' is -O- or -C-, V is a C₁-C₄ saturated alkyl chain, U' is an amide group (-NHCO- or - CONH-), and R'₂ is a C₁₀-C₃₀ alkyl chain.

13. The lipid compound of claim 12, wherein the lipid compound has a structure represented by formula (II) or formula (V), and X₅ is O or S.

14. The lipid compound of claim 12 or 13, wherein:
N₁ is not a direct bond; R'₈ is not a direct bond; and N₂ is a direct bond, preferably, N₂ is linked to X₇;
N₁ is a direct bond or 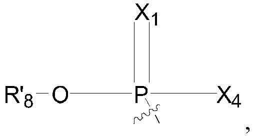 wherein R'₈ is a direct bond; and N₂ is not a direct bond, preferably, N₁ is linked to X₆; or
N₁ is a direct bond or 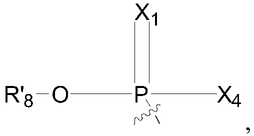 wherein R'₈ is a direct bond; and N₂ is a direct bond, preferably, N₁ is linked to X₆, and N₂ is linked to X₇.

15. The lipid compound of any one of claims 12 to 14, wherein when N₁, N₂ or R'₈ is a direct bond, N₁, N₂ or R'₈ is connected to H.

16. The lipid compound of claim 1, wherein the lipid compound is selected from at least one of the following structures (L1) to (L36), or (L'10):
| | | | |
|---|---|---|---|
| L1 | 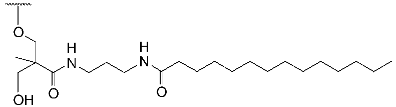 | L21 | 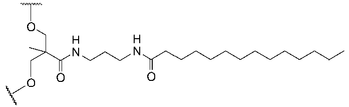 |
| L2 | 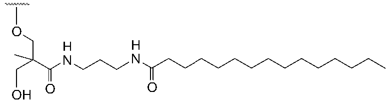 | L22 | 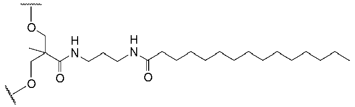 |
| L3 | 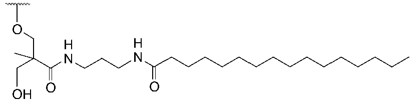 | L17 | 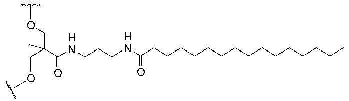 |
| L4 | 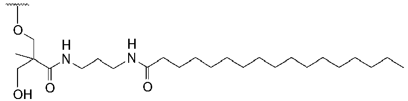 | L23 | 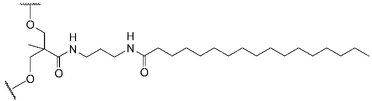 |
| L5 | 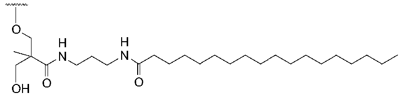 | L24 | 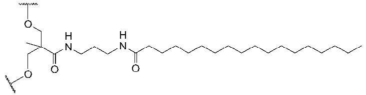 |
| L6 | 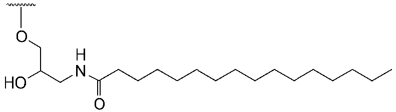 | L25 | 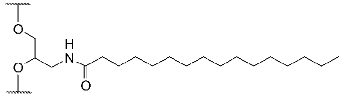 |
| L7 | 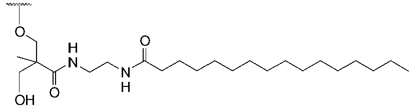 | L26 | 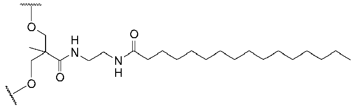 |
| L8 | 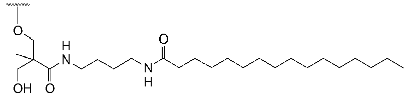 | L27 | 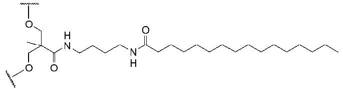 |
| L9 | 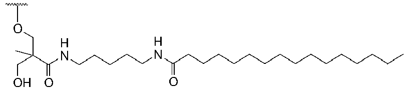 | L28 | 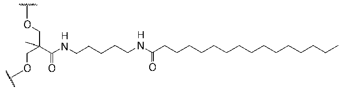 |
| L10 | 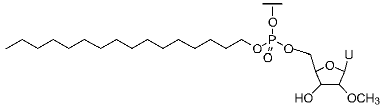 | L'10 | 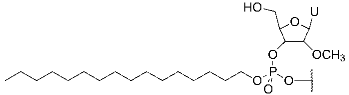 |
| L11 | 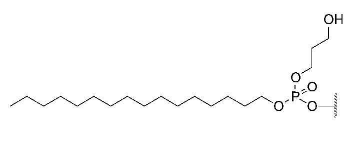 | L29 | 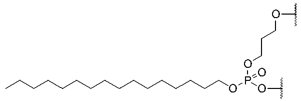 |
| L12 | 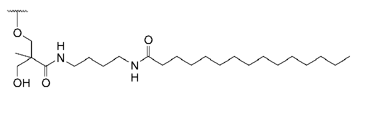 | L30 | 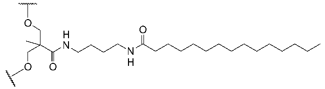 |
| L13 | 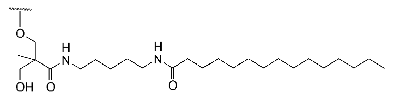 | L31 | 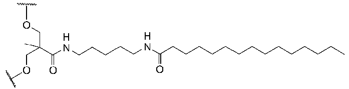 |
| L14 | 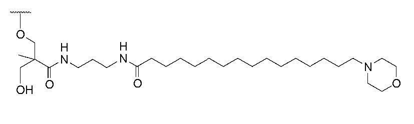 | L32 | 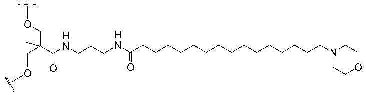 |
| L15 | 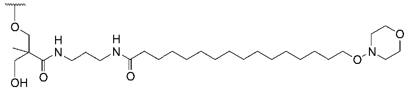 | L33 | 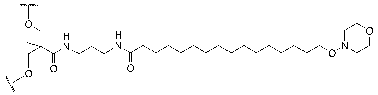 |
| L16 | 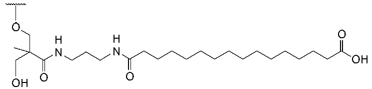 | L19 | 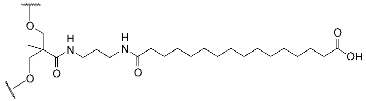 |
| L34 | 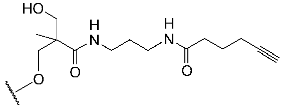 | L18 | 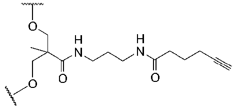 |
| L35 | 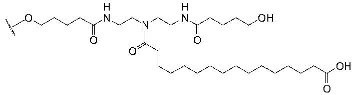 | L20 | 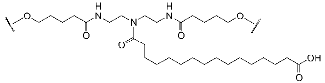 |
| L36 | 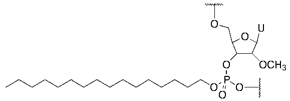 | | |
wherein U is

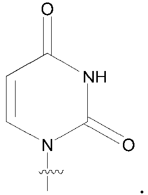

17. The lipid compound of claim 1, wherein the lipid compound is selected from at least one of the following structures (L1') to (L36'), or (L'10'):
| | | | |
|---|---|---|---|
| L1' | 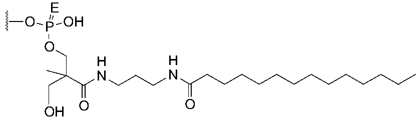 | L21' | 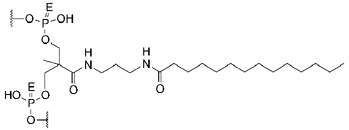 |
| L2' | 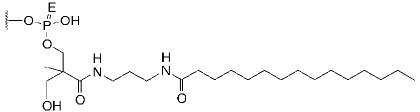 | L22' | 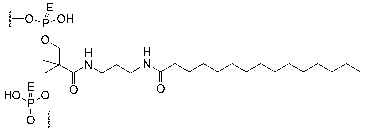 |
| L3' | 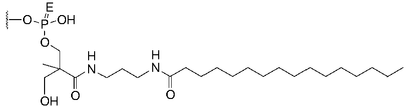 | L17' | 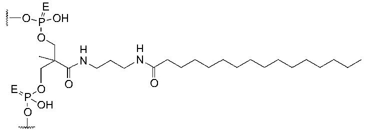 |
| L4' | 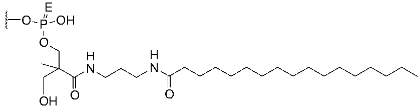 | L23' | 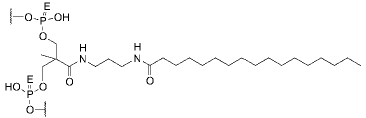 |
| L5' | 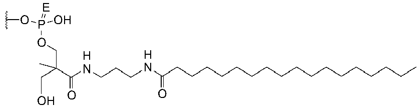 | L24' | 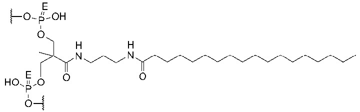 |
| L6' | 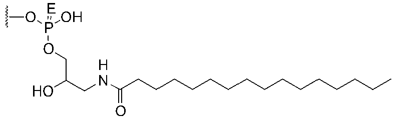 | L25' | 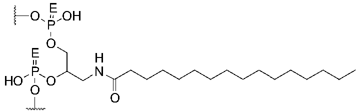 |
| L7' | 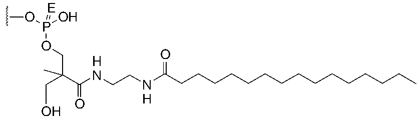 | L26' | 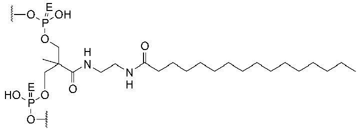 |
| L8' | 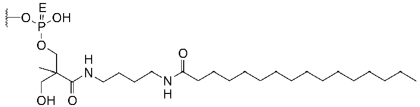 | L27' | 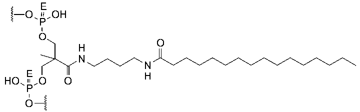 |
| L9' | 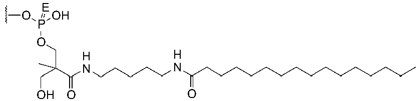 | L28' | 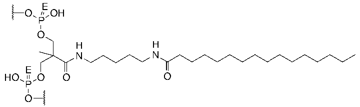 |
| L10' | 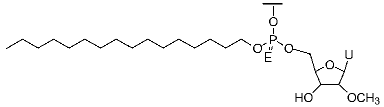 | L'10' | 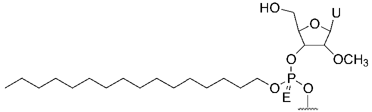 |
| L11' | 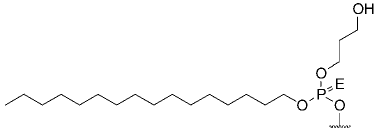 | L29' | 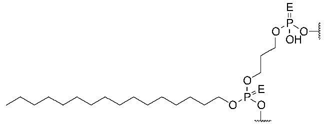 |
| L12' | 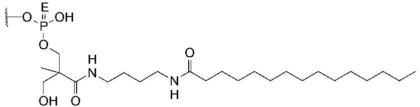 | L30' | 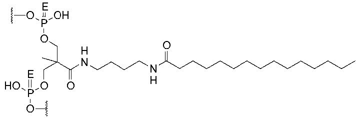 |
| L13' | 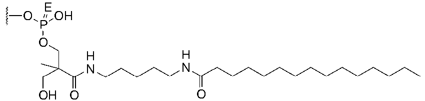 | L31' | 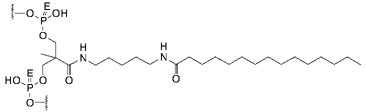 |
| L14' | 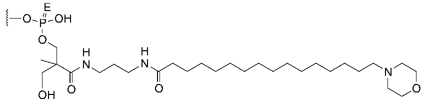 | L32' | 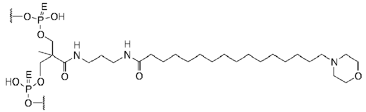 |
| L15' | 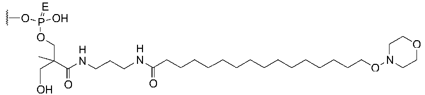 | L33' | 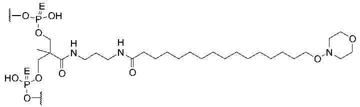 |
| L16' | 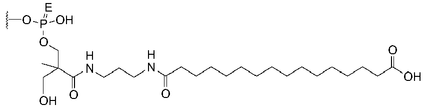 | L19' | 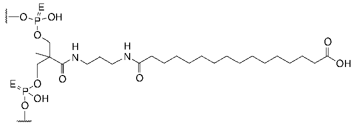 |
| L34' | 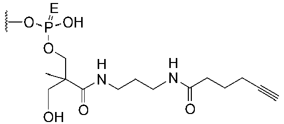 | L18' | 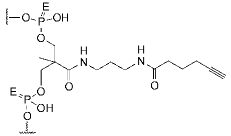 |
| L35' | 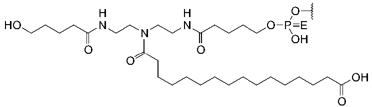 | L20' | 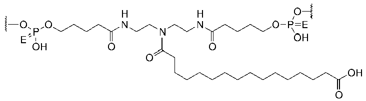 |
| L36' | 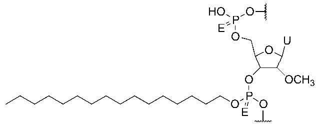 | | |
wherein E is selected from O and S, and U is

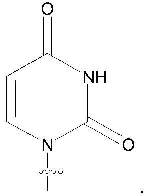

18. A nucleic acid conjugate comprising a nucleic acid and a conjugate moiety conjugated to the nucleic acid,
wherein the conjugate moiety is selected from the lipid compound of any one of claims 1 to 17,
preferably, the conjugate moiety is conjugated to a phosphate group of the nucleic acid or a hydroxyl group of the ribose,
more preferably, the nucleic acid conjugate has the following structure:
Nu-O-W₁-T₁; Formula (III) 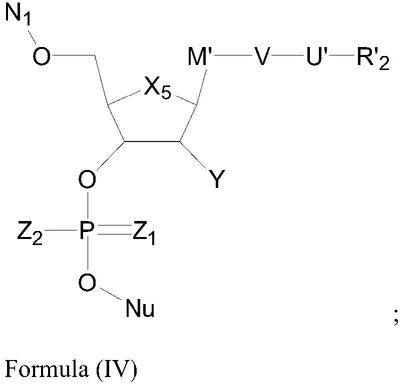
or 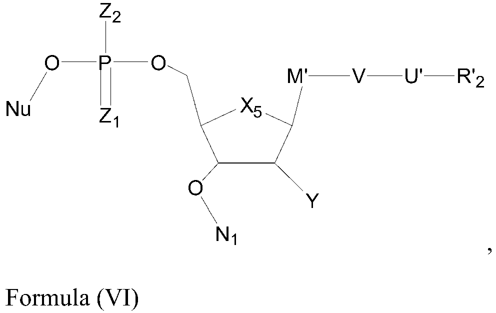
wherein Nu is a nucleic acid or a nucleic acid fragment, and definitions of other variables are the same as defined in any one of claims 1 to 17.

19. The nucleic acid conjugate of claim 18, wherein the nucleic acid conjugate has a structure of formula (III), wherein X₂ is -O-(CH₂)_{n'}-OR'₅, wherein R'₅ is selected from a direct bond or 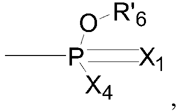 wherein R'₆ is a direct bond, and n' is an integer from 1 to 10;
Q₂ is selected from -O-, -S-, or 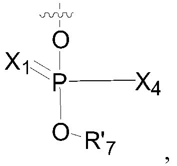 wherein R'₇ is a direct bond;
R'₁ is -(CH₂)ₘ-X₃-R'₃, wherein m is an integer from 10 to 30; X₃ is selected from an oxygen atom, a sulfur atom, a direct bond or 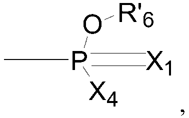 wherein R'₆ is a direct bond; or
the nucleic acid conjugate has a structure of formula (IV) or (VI), wherein N₁ is a direct bond or 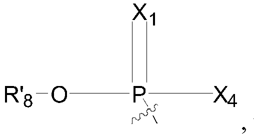 wherein R'₈ is a direct bond.

20. The nucleic acid conjugate of claim 18, wherein Nu is a nucleic acid or a nucleic acid fragment, and definitions of other variables are the same as defined in any one of claims 7 to 10, claims 12 to 14, and claims 16 to 17.

21. The nucleic acid conjugate of any one of claims 18 to 20, wherein the direct bond is conjugated to the nucleic acid or nucleic acid fragment.

22. The nucleic acid conjugate of any one of claims 18 to 21, wherein the nucleic acid is selected from a single-stranded nucleic acid or a fragment thereof, or a double-stranded nucleic acid or a fragment thereof; a length of the double-stranded nucleic acid or the fragment thereof is preferably 12-30 mer, and the double-stranded nucleic acid or the fragment thereof is preferably siRNA or a fragment thereof; preferably, a molecular weight of the double-stranded nucleic acid or the fragment thereof ranges from 6000 to 20000 Daltons; a length of the single-stranded nucleic acid or the fragment thereof is preferably 12-30 mer, and the single-stranded nucleic acid or the fragment thereof is preferably a single-stranded phosphorothioate oligonucleotide or a fragment thereof; preferably, a molecular weight of the single-stranded nucleic acid or the fragment thereof ranges from 3000 to 10000 Daltons.

23. The nucleic acid conjugate of claim 22, wherein each nucleotide in the nucleic acid is independently a modified or unmodified nucleotide; or two adjacent nucleotides in the nucleic acid are linked via a phosphodiester bond, wherein one or more of the phosphodiester bonds are phosphorothioate diester bonds,
preferably, each nucleotide in the nucleic acid is independently a nucleotide with fluorine-substitution modification or a nucleotide with non-fluorine-substitution modification,
preferably, the fluorine-substitution modification is that the hydroxy group at the 2'-position of the pentose of the nucleotide is substituted with F,
preferably, the non-fluorine-substitution modification is that the hydroxy group at the 2'-position of the pentose of the nucleotide is substituted with an alkoxy group, and the hydroxy group at the 2'-position is preferably substituted with a methoxy group or a 2'-methoxyethoxy group.

24. The nucleic acid conjugate of claim 22 or 23, wherein the conjugate moiety is conjugated to the double-stranded nucleic acid, and the double-stranded nucleic acid comprises a sense strand and an antisense strand, preferably, the conjugate moiety is conjugated to the 3' or 5' end of the sense strand or the antisense strand, more preferably, the conjugate moiety is conjugated to the 3' end of the sense strand.

25. The nucleic acid conjugate of any one of claims 22 to 25, wherein the conjugate moiety is conjugated to the double-stranded nucleic acid on one end, and conjugated to the single-stranded nucleic acid on the other end; preferably, the conjugate moiety is conjugated to the sense strand of the double-stranded nucleic acid on one end, and conjugated to the single-stranded nucleic acid on the other end to form a sense strand of the nucleic acid conjugate; preferably, the single-stranded nucleic acid is located at the 3' end or 5' end of the sense strand of the nucleic acid conjugate; more preferably, the single-stranded nucleic acid is located at the 3' end of the sense strand of the nucleic acid conjugate.

26. The nucleic acid conjugate of claim 24 or 25, wherein a sequence of the sense strand is selected from the following sequences:
(1) CAUUUUAAUCCUCACUCUAAA;
(2) GCUCAGCAUUGCCUGAAUAAA; or
(3) UGCAAAUAGUCUACAAACCAA,
a sequence of the antisense strand is selected from the following sequences:
(4) UUUAGAGUGAGGAUUAAAAUGAG;
(5) UUUAUUCAGGCAAUGCUGAGCUU; or
(6) UUGGUUUGUAGACUAUUUGCACA.

27. The nucleic acid conjugate according to any one of claims 22 to 25, wherein the single-stranded nucleic acid comprises 14-20 nucleotides, preferably 16 nucleotides, and preferably, the single-stranded nucleic acid comprises a sequence selected from the following:
CCGTCGCCCTTCAGCACGCA;
CGTCGCCCTTCAGCACGC;
GTCGCCCTTCAGCACG; or
TCGCCCTTCAGCAC,
preferably, the single-stranded nucleic acid comprises TCGCCCTTCAGCAC.

28. The conjugate of claim 18, wherein the conjugate is selected from at least one of the following structures (L1") to (L36"), or (L'10"):
| | | | |
|---|---|---|---|
| L1" | 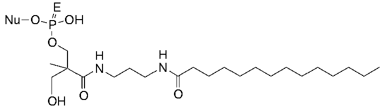 | L21" | 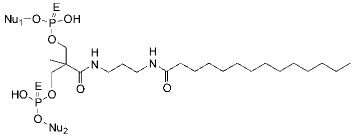 |
| L2" | 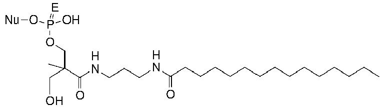 | L22" | 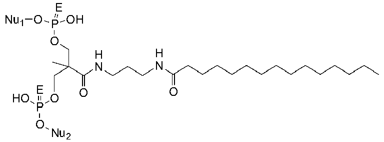 |
| L3" | 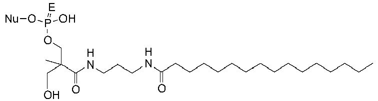 | L17" | 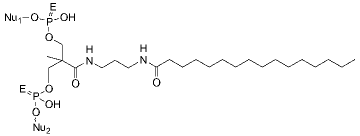 |
| L4" | 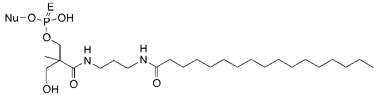 | L23" | 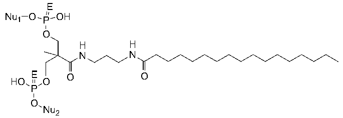 |
| L5" | 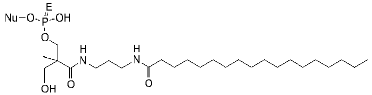 | L24" | 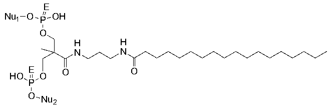 |
| L6" | 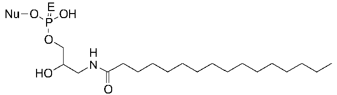 | L25" | 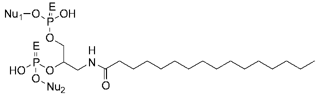 |
| L7" | 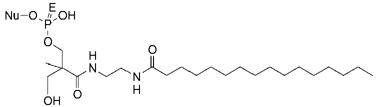 | L26" | 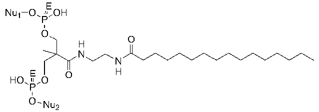 |
| L8" | 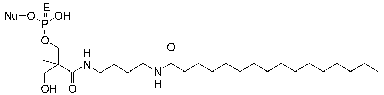 | L27" | 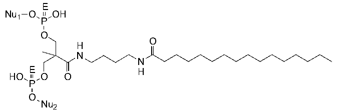 |
| L9" | 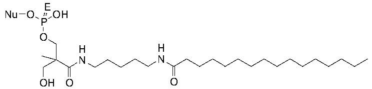 | L28" | 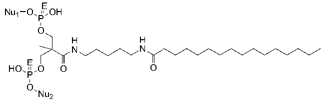 |
| L10" | 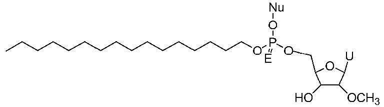 | L'10" | 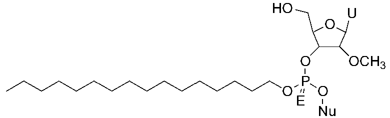 |
| L11" | 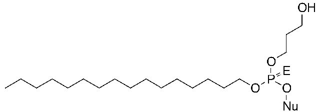 | L29" | 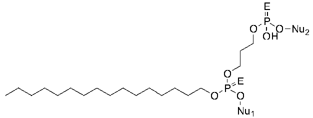 |
| L12" | 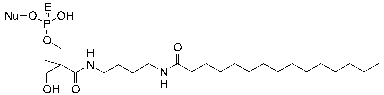 | L30" | 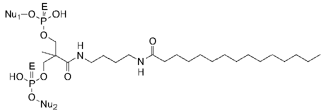 |
| L13" | 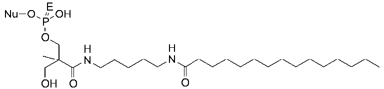 | L31" | 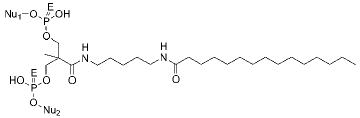 |
| L14" | 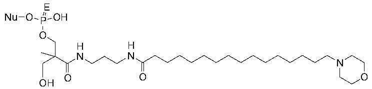 | L32" | 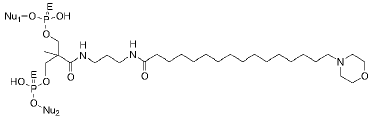 |
| L15" | 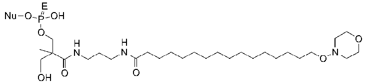 | L33" | 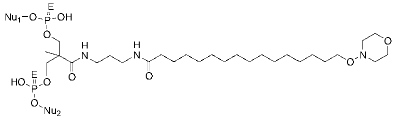 |
| L16" | 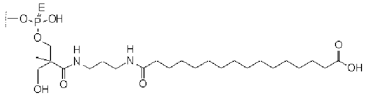 | L19" | 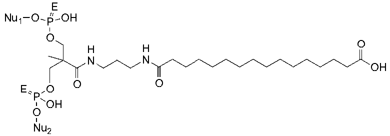 |
| L34" | 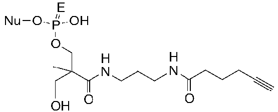 | L18" | 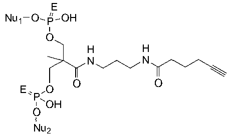 |
| L35" | 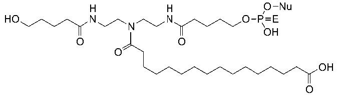 | L20" | 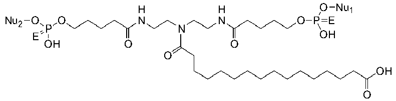 |
| L36" | 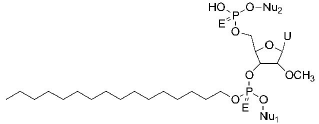 | | |
in the above table, Nu, Nu₁ and Nu₂ each independently represents a nucleic acid or a fragment of a nucleic acid; and Nu, Nu₁ and Nu₂ may be the same or different; and
wherein E is selected from O or S.

29. The nucleic acid conjugate of claim 18, wherein the nucleic acid conjugate has a structure selected from a group consisting of:
| | |
|---|---|
| SN-16981 | ss: 5'-mCsmAsmUmUmUmUAfmAUfCfCfmUmCmAmCmUmCmUmAsmAsmA-3'-L1'; |
| | as: 5'-mUsUfsmUmAmGAfmGUfGfmAmGmGmAUfmUAfmAmAmAmUmGsmAsmG-3'; |
| SN-16982 | ss: 5'-mCsmAsmUmUmUmUAfmAUfCfCfmUmCmAmCmUmCmUmAsmAsmA-3'-L2'; |
| | as: 5'-mUsUfsmUmAmGAfmGUfGfmAmGmGmAUfmUAfmAmAmAmUmGsmAsmG-3'; |
| SN-16983 | ss: 5'-mCsmAsmUmUmUmUAfmAUfCfCfmUmCmAmCmUmCmUmAsmAsmA-3'-L3'; |
| | as: 5'-mUsUfsmUmAmGAfmGUfGfmAmGmGmAUfmUAfmAmAmAmUmGsmAsmG-3'; |
| SN-17011 | ss: 5'-mGsmCsmUmCmAmGmCfmAmUfmUfmGfmCmCmUmGmAmAmUmAmAmA-3'- L3'; |
| | as: 5'- mUsmUfsmUmAfmUmUfmCmAmGmGmCmAmAmUfmGmCfmUmGmAmGmCsmUsmU- 3'; |
| SN-16984 | ss: 5'-mCsmAsmUmUmUmUAfmAUfCfCfmUmCmAmCmUmCmUmAsmAsmA-3'-L4'; |
| | as: 5'-mUsUfsmUmAmGAfmGUfGfmAmGmGmAUfmUAfmAmAmAmUmGsmAsmG-3'; |
| SN-17012 | ss: 5'-mCsmAsmUmUmUmUAfmAUfCfCfmUmCmAmCmUmCmUmAsmAsmA-3'-L5'; |
| | as: 5'-mUsUfsmUmAmGAfmGUfGfmAmGmGmAUfmUAfmAmAmAmUmGsmAsmG-3'; |
| SN-16985 | ss: 5'-mCsmAsmUmUmUmUAfmAUfCfCfmUmCmAmCmUmCmUmAsmAsmA-3'-L6'; |
| | as: 5'-mUsUfsmUmAmGAfmGUfGfmAmGmGmAUfmUAfmAmAmAmUmGsmAsmG-3'; |
| SN-16986 | ss: 5'-mCsmAsmUmUmUmUAfmAUfCfCfmUmCmAmCmUmCmUmAsmAsmA-3'-L7'; |
| | as: 5'-mUsUfsmUmAmGAfmGUfGfmAmGmGmAUfmUAfmAmAmAmUmGsmAsmG-3'; |
| SN-16987 | ss: 5'-mCsmAsmUmUmUmUAfmAUfCfCfmUmCmAmCmUmCmUmAsmAsmA-3'-L8'; |
| | as: 5'-mUsUfsmUmAmGAfmGUfGfmAmGmGmAUfmUAfmAmAmAmUmGsmAsmG-3'; |
| SN-16988 | ss: 5'-mCsmAsmUmUmUmUAfmAUfCfCfmUmCmAmCmUmCmUmAsmAsmA-3'-L9'; |
| | as: 5'-mUsUfsmUmAmGAfmGUfGfmAmGmGmAUfmUAfmAmAmAmUmGsmAsmG-3'; |
| SN-17028 | ss: 5'-mCsmAsmUmUmUmUAfmAUfCfCfmUmCmAmCmUmCmUmAsmA-(L10')-3'; |
| | as: 5'-mUsUfsmUmAmGAfmGUfGfmAmGmGmAUfmUAfmAmAmAmUmGsmAsmG-3'; |
| SN-16991 | ss: 5'-mCsmAsmUmU-(L36')-mUAfmAUfCfCfmUmCmAmCmUmCmUmAsmAsmA-3 '; |
| | as: 5'-mUsUfsmUmAmGAfmGUfGfmAmGmGmAUfmUAfmAmAmAmUmGsmAsmG-3'; |
| SN-16992 | ss: 5'-mCsmAsmUmUmUmUAfmAUfCfCfmUmCmAmCmUmCmUmAsmAsmA-3'-L11'; |
| | as: 5'-mUsUfsmUmAmGAfmGUfGfmAmGmGmAUfmUAfmAmAmAmUmGsmAsmG-3'; |
| SN-16989 | ss: 5'-mCsmAsmUmUmUmUAfmAUfCfCfmUmCmAmCmUmCmUmAsmAsmA-3'-L12'; |
| | as: 5'-mUsUfsmUmAmGAfmGUfGfmAmGmGmAUfmUAfmAmAmAmUmGsmAsmG-3'; |
| SN-16990 | ss: 5'-mCsmAsmUmUmUmUAfmAUfCfCfmUmCmAmCmUmCmUmAsmAsmA-3'-L13'; |
| | as: 5'-mUsUfsmUmAmGAfmGUfGfmAmGmGmAUfmUAfmAmAmAmUmGsmAsmG-3'; |
| SN-17013 | ss: 5'-mCsmAsmUmUmUmUAfmAUfCfCfmUmCmAmCmUmCmUmAsmAsmA-3'-L14'; |
| | as: 5'-mUsUfsmUmAmGAfmGUfGfmAmGmGmAUfmUAfmAmAmAmUmGsmAsmG-3'; |
| SN-17014 | ss: 5'-mCsmAsmUmUmUmUAfmAUfCfCfmUmCmAmCmUmCmUmAsmAsmA-3'-L15'; |
| | as: 5'-mUsUfsmUmAmGAfmGUfGfmAmGmGmAUfmUAfmAmAmAmUmGsmAsmG-3'; |
| SN-17002 | ss: 5'-mCsmAsmUmUmUmUAfmAUfCfCfmUmCmAmCmUmCmUmAsmAsmA-3'-L16'; |
| | as: 5'-mUsUfsmUmAmGAfmGUfGfmAmGmGmAUfmUAfmAmAmAmUmGsmAsmG-3'; |
| SN-683081 | ss: 5'-mUsmGsmCmAmAmAmUfmAmGfmUfmCfmUmAmCmAmAmAmCmCsmAsmA-3'- L16'; |
| | as: 5'- mUsmUfsmGmGmUmUmUmGmUmAmGmAmCmUfmAmUfmUmUmGmCmAsmCsmA- 3'; |
| SN-17034 | ss: 5'-mCsmAsmUmUmUmUAfmAUfCfCfmUmCmAmCmUmCmUmAsmAsmA-3'-L34'; |
| | as: 5'-mUsUfsmUmAmGAfmGUfGfmAmGmGmAUfmUAfmAmAmAmUmGsmAsmG-3'; |
| SN-17009 | ss:5'-mCsmAsmUmUmUmUAfmAUfCfCfmUmCmAmCmUmCmUmAsmAsmA-3'-L35'; |
| | as: 5'-mUsUfsmUmAmGAfmGUfGfmAmGmGmAUfmUAfmAmAmAmUmGsmAsmG-3'; |
| | |
|---|---|
| SN-17015 | ss: 5'-mCsmAsmUmUmUmUAfmAUfCfCfmUmCmAmCmUmCmUmAsmAsmA-L21'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17016 | ss: 5'-mCsmAsmUmUmUmUAfmAUfCfCfmUmCmAmCmUmCmUmAsmAsmA-L22'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17029 | ss: 5'-mCsmAsmUmUmUmUAfmAUfCfCfmUmCmAmCmUmCmUmAsmAsmA-L17'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17017 | ss: 5'-mCsmAsmUmUmUmUAfmAUfCfCfmUmCmAmCmUmCmUmAsmAsmA-L23'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17025 | ss: 5'-mCsmAsmUmUmUmUAfmAUfCfCfmUmCmAmCmUmCmUmAsmAsmA-L24'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17018 | ss: 5'-mCsmAsmUmUmUmUAfmAUfCfCfmUmCmAmCmUmCmUmAsmAsmA-L25'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17019 | ss: 5'-mCsmAsmUmUmUmUAfmAUfCfCfmUmCmAmCmUmCmUmAsmAsmA-L26'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17020 | ss: 5'-mCsmAsmUmUmUmUAfmAUfCfCfmUmCmAmCmUmCmUmAsmAsmA-L27'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17021 | ss: 5'-mCsmAsmUmUmUmUAfmAUfCfCfmUmCmAmCmUmCmUmAsmAsmA-L28'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17030 | ss: 5'-mCsmAsmUmUmUmUAfmAUfCfCfmUmCmAmCmUmCmUmAsmAsmA-L29'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17022 | ss: 5'-mCsmAsmUmUmUmUAfmAUfCfCfmUmCmAmCmUmCmUmAsmAsmA-L30'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17023 | ss: 5'-mCsmAsmUmUmUmUAfmAUfCfCfmUmCmAmCmUmCmUmAsmAsmA-L31'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17026 | ss: 5'-mCsmAsmUmUmUmUAfmAUfCfCfmUmCmAmCmUmCmUmAsmAsmA-L32'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17027 | ss: 5'-mCsmAsmUmUmUmUAfmAUfCfCfmUmCmAmCmUmCmUmAsmAsmA-L33'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17031 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-16995 | ss: 5'-TsAGsGAstsastsastststsCstsasCAsGCsT-L17'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-16996 | ss: 5'-mCsmAsmUmUmUmUAfmAUfCfCfmUmCmAmCmUmCmUmAsmAsmA-L17'- TsAGsGAstsastsastststsCstsasCAsGCsT 3'; |
| | as: 5'-mUsUfsmUmAmGAfmGUfGfmAmGmGmAUfmUAfmAmAmAmUmGsmAsmG-3'; |
| SN-16997 | ss: 5'-CsCsGsTsCsgsCsCsCststsCsasgsCsAsCsGsCsA-L17'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-16998 | ss: 5'-mCsmAsmUmUmUmUAfmAUfCfCfmUmCmAmCmUmCmUmAsmAsmA-L17'- CsCsGsTsCsgsCsCsCststsCsasgsCsAsCsGsCsA-3'; |
| | as: 5'-mUsUfsmUmAmGAfmGUfGfmAmGmGmAUfmUAfmAmAmAmUmGsmAsmG-3'; |
| SN-17003 | ss: 5'-mCsmAsmUmUmUmUAfmAUfCfCfmUmCmAmCmUmCmUmAsmAsmA-L17'- CsGsTsCsgsCsCsCststsCsasgsCsAsCsGsC-3'; |
| | as: 5'-mUsUfsmUmAmGAfmGUfGfmAmGmGmAUfmUAfmAmAmAmUmGsmAsmG-3'; |
| SN-17004 | ss: 5'-mCsmAsmUmUmUmUAfmAUfCfCfmUmCmAmCmUmCmUmAsmAsmA-L17'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17005 | ss: 5'-mCsmAsmUmUmUmUAfmAUfCfCfmUmCmAmCmUmCmUmAsmAsmA-L17'- TsCsgsCsCsCststsCsasgsCsAsC-3'; |
| | as: 5'-mUsUfsmUmAmGAfmGUfGfmAmGmGmAUfmUAfmAmAmAmUmGsmAsmG-3'; |
| SN-17001 | ss: 5'-CsCsGsTsCsgsCsCsCststsCsasgsCsAsCsGsCsA-L18'- mCsmAsmUmUmUmUAfmAUfCfCfmUmCmAmCmUmCmUmAsmAsmA-3'; |
| | as: 5'-mUsUfsmUmAmGAfmGUfGfmAmGmGmAUfmUAfmAmAmAmUmGsmAsmG-3'; |
| SN-17024 | ss: 5'-mCsmAsmUmUmUmUAfmAUfCfCfmUmCmAmCmUmCmUmAsmAsmA-L18'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17032 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17006 | ss: 5'-mCsmAsmUmUmUmUAfmAUfCfCfmUmCmAmCmUmCmUmAsmAsmA-L19'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
| SN-17010 | ss: 5'-mCsmAsmUmUmUmUAfmAUfCfCfmUmCmAmCmUmCmUmAsmAsmA-L20'- GsTsCsgsCsCsCststsCsasgsCsAsCsG-3'; |
| | as: 5'-mUsUfsmUmAmGAfmGUfGfmAmGmGmAUfmUAfmAmAmAmUmGsmAsmG-3'; |
preferably, E is O.

30. Use of the lipid compound of any one of claims 1 to 17 in preparation of the nucleic acid conjugate of any one of claims 18 to 29.

31. Use of the nucleic acid conjugate of any one of claims 18 to 29 in preparation of a medicament for treatment of a gene-related disease,
preferably, the gene is selected from APP, SOD1, HTT, MeCP2, DUX4, HDAC2, GPR75, Ataxin 1, Ataxin 2, Ataxin 3, Ataxin 6, Ataxin 7, C9ORF72, UBE3A, Prion, PMP22, Tau, LRRK2, LINGO2, GYS1, KCNT1, IRF5, Progranulin, GFAP, TARDBP, SNCA, FUS, SCA1, SCA7, SCA8, MeCP2, PRNP, SOD1, DMPK, MAPT, or TTR,
preferably, the disease is a central nervous system disease; preferably, the central nervous system disease is Alzheimer's disease, preferably, the central nervous system disease is presenile dementia, amyotrophic lateral sclerosis, Huntington's disease, Parkinson's disease, Dravet syndrome, Charcot triad, Alexander disease, spinocerebellar ataxia, or Angelman syndrome, more preferably, the disease is selected from presenile dementia, amyotrophic lateral sclerosis, or spinocerebellar ataxia; and
preferably, the medicament is an injection or an oral preparation, more preferably, an injection administered intracranially, intrathecally, subcutaneously, intravenously or intramuscularly.
